(19) Europäisches Patentamt
European Patent Office
Office européen des brevets

(11) EP 1 424 557 A1

(12) EUROPEAN PATENT APPLICATION
published in accordance with Art. 158(3) EPC

(43) Date of publication:
**02.06.2004 Bulletin 2004/23**

(21) Application number: **02762957.5**

(22) Date of filing: **02.09.2002**

(51) Int Cl.[7]: **G01N 33/50**, G01N 33/15, G01N 33/566, A61K 38/17, A61K 45/00, A61P 35/00, A61P 43/00

(86) International application number:
**PCT/JP2002/008873**

(87) International publication number:
**WO 2003/021263 (13.03.2003 Gazette 2003/11)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **03.09.2001 JP 2001266308**

(71) Applicant: **Takeda Chemical Industries, Ltd.**
**Osaka-shi, Osaka 541-0045 (JP)**

(72) Inventors:
- **HINUMA, Shuji**
**Tsukuba-shi, Ibaraki 305-0821 (JP)**
- **HOSOYA, Masaki**
**Tsuchiura-shi, Ibaraki 300-0007 (JP)**

(74) Representative: **Rickard, Timothy Mark Adrian**
**Takeda Euro IP Department,**
**11-12 Charles II Street**
**London SW1Y 4QU (GB)**

# (54) USE OF G PROTEIN-COUPLED RECEPTOR PROTEIN

(57) Using a G protein-coupled receptor protein (TGR4) having the same or substantially the same amino acid sequence as the amino acid sequence represented by SEQ ID NO: 1, or its salt and a phospholipid compound, a compound or its salt that alters the binding properties of the receptor protein or its salt to the phospholipid compound can be screened efficiently.

EP 1 424 557 A1

## Description

FIELD OF THE INVENTION

The present invention relates to a method of screening an agonist/antagonist using a human-derived G protein-coupled receptor protein (H7TMB56) or its salts and an isoprenoid-related substance.

BACKGROUND ART

Physiologically active substances such as various hormones and neurotransmitters regulate the biological functions via specific receptor proteins present on cell membranes. Many of these receptor proteins are coupled with guanine nucleotide-binding protein (hereinafter sometimes simply referred to as G protein) and mediate the intracellular signal transduction via activation of G protein. These receptor proteins possess the common structure containing seven transmembrane domains and are thus collectively referred to as G protein-coupled receptors or seven-transmembrane receptors (7TMR).
G protein-coupled receptor proteins present on the cell surface of each functional cells and organs in the body, and play important physiological roles as the target of the molecules that regulate the functions of these cells and organs, e.g., hormones, neurotransmitters, physiologically active substances and the like. Receptors transmit signals to cells via binding with physiologically active substances, and the signals induce various reactions such as activation and inhibition of the cells.
To elucidate the relationship between substances that regulate complex biological functions in various cells and organs, and their specific receptor proteins, in particular, G protein-coupled receptor proteins would elucidate the functional mechanisms in various cells and organs in the body to provide a very important means for development of pharmaceutical drugs closely associated with these functions.
For example, in various organs, their physiological functions are controlled in vivo through regulation by many hormones, hormone-like substances, neurotransmitters or physiologically active substances. In particular, physiologically active substances are found in numerous sites of the body and regulate the physiological functions through their corresponding receptor proteins. However, it is supposed that many unknown hormones, neurotransmitters or many other physiologically active substances still exist in the body and, as to their receptor proteins, many of these proteins have not yet been reported. In addition, it is yet unknown if there are subtypes of known receptor proteins.
It is very important for development of pharmaceutical drugs to clarify the relationship between substances that regulate elaborated functions in vivo and their specific receptor proteins. Furthermore, for efficient screening of agonists and antagonists to receptor proteins in development of drugs, it was required to clarify functional mechanisms of receptor protein genes expressed in vivo and express the genes in an appropriate expression system.
In recent years, random analysis of cDNA sequences has been actively studied as a means for analyzing genes expressed in vivo. The sequences of cDNA fragments thus obtained have been registered on and published to databases as Expressed Sequence Tag (EST). However, since many ESTs contain sequence information only, it is difficult to predict their functions from the information.
In WO 00/20456, the G protein-coupled receptor protein (H7TMB56) used in the present invention and its DNA are described.
The present invention investigates a ligand to known G protein-coupled receptor protein (H7TMB56), and provides a method of screening a compound (antagonist, agonist) that alters the binding properties of the ligand to the receptor protein; or the like.

DISCLOSURE OF THE INVENTION

The present inventors made extensive investigations to solve the problems described above and as a result, found that a ligand to G protein-coupled receptor protein (H7TMB56) derived from human hippocampus is an isoprenoid-related substance. It is known that isoprenoid-related substances are metabolite intermediates of important substances in vivo, such as cholesterol, hem α, dolichol, ubiquinone, etc. and at the same time, play important roles in regulating functional modification, cell proliferation, cell differentiation, cell maturation or apoptosis due to isoprenylation of various proteins (The Journal of Neuroscience, 20(8):2852-2859, 2000, Journal of Cardiovascular Pharmacology, 39:310-317, 2002), functional maintenance of cells, etc. With regard to G protein-coupled receptor proteins which will recognize the isoprenoid-related substances, however, nothing was known at all to date. Based on these findings, the present inventors have made further investigations and as a result, have come to accomplish the present invention.
Thus, the present invention provides the following features.

(1) A method of screening a compound or its salt that alters the binding properties of a G protein-coupled receptor protein containing the same or substantially the same amino acid sequence as the amino acid sequence represented by SEQ ID NO: 1 or its salt to an isoprenoid-related substance, which comprises using (1) said receptor protein, its partial peptide, or a salt thereof and (2) the isoprenoid-related substance;
(2) A method of screening according to (1), wherein the isoprenoid-related substance is a compound, in which a pyrophosphate group is bound to the tail of the recurring structure of at least 2 isoprenic units;
(3) A method of screening according to (1), wherein the isoprenoid-related substance is farnesyl pyrophosphate (FPP), geranyl pyrophosphate (GPP) or geranylgeranyl pyrophosphate (GGPP);
(4) A method of screening according to (1), wherein the G protein-coupled receptor protein is a protein consisting the amino acid sequence represented by SEQ ID NO: 1 or SEQ ID NO: 6;
(5) A kit for screening a compound or its salt that alters the binding properties of a G protein-coupled receptor protein containing the same or substantially the same amino acid sequence as the amino acid sequence represented by SEQ ID NO: 1 to an isoprenoid-related substance, comprising (1) said receptor protein, its partial peptide, or a salt thereof and (2) the isoprenoid-related substance;
(6) A compound or its salt that alters the binding properties of an isoprenoid-related substance to a G protein-coupled receptor protein containing the same or substantially the same amino acid sequence represented by SEQ ID NO: 1, or its salt, which is obtainable using the screening method according to (1) or the screening kit according to (5);
(7) A pharmaceutical comprising a compound or its salt that alters the binding properties of an isoprenoid-related substance to a G protein-coupled receptor protein containing the same or substantially the same amino acid sequence as the amino acid sequence represented by SEQ ID NO: 1, or its salt;
(8) A pharmaceutical according to (7), which is a cell growth regulator, a cell differentiation regulator or an apoptosis regulator;
(9) A method of determining a ligand to a G protein-coupled receptor protein containing the same or substantially the same amino acid sequence as the amino acid sequence represented by SEQ ID NO: 1, or its salt, which comprises measuring an intracellular $Ca^{2+}$ level increasing activity or an intracellular cAMP producing activity, in the case where a test compound is brought in contact with a cell containing said G protein-coupled receptor protein;
(10) A ligand obtained by the method according to (9);
(11) A method of screening a compound or its salt that alters the binding properties of a G protein-coupled receptor protein containing the same or substantially the same amino acid sequence as the amino acid sequence represented by SEQ ID NO: 1 or its salt to an isoprenoid-related substance, which comprises comparing (i) the case where a G protein-coupled receptor protein containing the same or substantially the same amino acid sequence as the amino acid sequence represented by SEQ ID NO: 1, its partial peptide, or a salt thereof, is brought in contact with ① an isoprenoid-related substance or ② a compound or its salt that alters the binding properties of said receptor protein or its salt to the isoprenoid-related substance, and (ii) the case where a G protein-coupled receptor protein containing the same or substantially the same amino acid sequence as the amino acid sequence represented by SEQ ID NO: 1, its partial peptide, or a salt thereof is brought in contact with ① an isoprenoid-related substance or ② a compound or its salt that alters the binding properties of said receptor protein or its salt to the isoprenoid-related substance, and a test compound;
(12) A method of screening a compound or its salt that alters the binding properties of a G protein-coupled receptor protein containing the same or substantially the same amino acid sequence as the amino acid sequence represented by SEQ ID NO: 1 or its salt to an isoprenoid-related substance, which comprises measuring a binding amount of ① a labeled form of the isoprenoid-related substance or ② a labeled form of the compound or its salt that alters the binding properties of said receptor protein or its salt and the isoprenoid-related substance, to the receptor protein, its partial peptide, or a salt thereof, (i) in the case where ① a labeled form of the isoprenoid-related substance or ② a labeled form of the compound or its salt that alters the binding properties of a G protein-coupled receptor protein containing the same or substantially the same amino acid sequence as the amino acid sequence represented by SEQ ID NO: I or its salt to the isoprenoid-related substance, is brought in contact with the receptor protein, its partial peptide, or a salt thereof, and (ii) in the case where ① a labeled form of the isoprenoid-related substance or ② a labeled form of the compound or its salt that alters the binding properties of a G protein-coupled receptor protein containing the same or substantially the same amino acid sequence as the amino acid sequence represented by SEQ ID NO: 1 or its salt to the isoprenoid-related substance, and a test compound are brought in contact with the receptor protein, its partial peptide, or a salt thereof; and comparing the binding amount between (i) and (ii);
(13) A method of screening a compound or its salt that alters the binding properties of a G protein-coupled receptor protein containing the same or substantially the same amino acid sequence as the amino acid sequence represented by SEQ ID NO: 1 or its salt to an isoprenoid-related substance, which comprises measuring a binding amount of ① a labeled form of the isoprenoid-related substance or ② a labeled form of the compound or its salt that alters the binding properties of said receptor protein or its salt to the isoprenoid-related substance, to a cell or cell membrane fraction containing the receptor protein, (i) in the case where ① a labeled form of the isoprenoid-related substance or ② a labeled form of the compound or its salt that alters the binding properties of a G protein-coupled receptor protein containing the same or substantially the same amino acid sequence as the amino acid sequence represented by SEQ ID NO: 1 or its salt to the isoprenoid-related substance, is brought in contact with the cell or cell membrane fraction containing said receptor protein, and (ii) in the case where ① a labeled form of the isoprenoid-related substance or ② a labeled form of the compound or its salt that alters the binding properties of a G protein-coupled receptor protein containing the same or substantially the same amino acid sequence as the amino acid sequence represented by SEQ ID NO: 1 or its salt to the isoprenoid-related substance, and a test compound are brought in contact with the cell or cell membrane fraction containing said receptor protein; and comparing the binding amount between (i) and (ii);

(14) A method of screening a compound or its salt that alters the binding properties of a G protein-coupled receptor protein containing the same or substantially the same amino acid sequence as the amino acid sequence represented by SEQ ID NO: 1 or its salt to an isoprenoid-related substance, which comprises measuring a cell stimulating activity mediated by said receptor protein, (i) in the case where ① an isoprenoid-related substance or ② a compound or its salt that alters the binding properties of a G protein-coupled receptor protein containing the same or substantially the same amino acid sequence as the amino acid sequence represented by SEQ ID NO: 1 or its salt to the isoprenoid-related substance, is brought in contact with a cell containing said receptor protein, and (ii) in the case where ① an isoprenoid-related substance or ② a labeled form of the compound or its salt that alters the binding properties of a G protein-coupled receptor protein containing the same or substantially the same amino acid sequence as the amino acid sequence represented by SEQ ID NO: 1 or its salt to the isoprenoid-related substance, and a test compound are brought in contact with the cell containing the receptor protein; and comparing the cell stimulating activity between (i) and (ii);

(15) A screening method according to (14), wherein the cell stimulating activity is an intracellular $Ca^{2+}$ level increasing activity or an intracellular cAMP producing activity;

(16) A screening method according to (11) through (15), wherein the isoprenoid-related substance is a compound, in which a pyrophosphate group is bound to the tail of the recurring structure of at least 2 isoprenic units;

(17) A screening method according to (11) through (16), wherein the isoprenoid-related substance is farnesyl pyrophosphate (FPP), geranyl pyrophosphate (GPP) or geranylgeranyl pyrophosphate (GGPP);

(18) A screening method according to (11) through (17), wherein the test compound is a compound designed to bind, based on the atomic coordinate and the position of the ligand-binding pocket in the active site of the receptor protein of the present invention containing the same or substantially the same amino acid sequence as the amino acid sequence represented by SEQ ID NO: 1, to the ligand-binding pocket;

(19) A compound or its salt that alters the binding properties of a G protein-coupled receptor protein containing the same or substantially the same amino acid sequence as the amino acid sequence represented by SEQ ID NO: 1 or its salt to an isoprenoid-related substance, which is obtainable by using the screening method according to any one of (11) through (18);

(20) A cell growth regulator, a cell differentiation regulator or an apoptosis regulator, comprising a G protein-coupled receptor protein containing the same or substantially the same amino acid sequence as the amino acid sequence represented by SEQ ID NO: 1, its partial peptide, or a salt thereof;

(21) A cell growth regulator, a cell differentiation regulator or an apoptosis regulator, comprising a polynucleotide containing a polynucleotide encoding a G protein-coupled receptor protein containing the same or substantially the same amino acid sequence as the amino acid sequence represented by SEQ ID NO: 1 or its partial peptide;

(22) A diagnostic for central dysfunctions, inflammatory diseases, cardiovascular diseases, cancer, mellitus diabetes, immune system diseases, liver/gall bladder diseases, alimentary disorders, anxiety, pains or obesity, comprising a polynucleotide containing a polynucleotide encoding a G protein-coupled receptor protein containing the same or substantially the same amino acid sequence as the amino acid sequence represented by SEQ ID NO: 1 or its partial peptide;

(23) A cell growth regulator, a cell differentiation regulator or an apoptosis regulator, comprising an antibody to a G protein-coupled receptor protein containing the same or substantially the same amino acid sequence as the amino acid sequence represented by SEQ ID NO: 1, its partial peptide, or a salt thereof;

(24) A diagnostic for central dysfunctions, inflammatory diseases, cardiovascular diseases, cancer, mellitus diabetes, immune system diseases, liver/gall bladder diseases, alimentary disorders, anxiety, pains or obesity, comprising an antibody to a G protein-coupled receptor protein containing the same or substantially the same amino acid sequence as the amino acid sequence represented by SEQ ID NO: 1, its partial peptide, or a salt thereof;

(25) A cell growth regulator, a cell differentiation regulator or an apoptosis regulator, comprising a whole or part of the base sequence complementary to a polynucleotide containing a polynucleotide encoding a G protein-coupled receptor protein containing the same or substantially the same amino acid sequence as the amino acid sequence represented by SEQ ID NO: 1 or its partial peptide;

(26) A cell growth regulator, a cell differentiation regulator or an apoptosis regulator, comprising a compound or its salt that alters the expression level of a G protein-coupled receptor protein containing the same or substantially the same amino acid sequence as the amino acid sequence represented by SEQ ID NO: 1;

(27) A method of regulating cell differentiation or apoptosis, which comprises administering to a mammal an effective dose of ① a compound or its salt that alters the binding properties of an isoprenoid-related substance to a G protein-coupled receptor protein containing the same or substantially the same amino acid sequence as the amino acid sequence represented by SEQ ID NO: 1 or its salt, ② a G protein-coupled receptor protein containing the same or substantially the same amino acid sequence as the amino acid sequence represented by SEQ ID NO: 1, its partial peptide, or a salt thereof, ③ a polynucleotide containing a polynucleotide encoding a G protein-coupled receptor protein containing the same or substantially the same amino acid sequence as the amino acid sequence represented by SEQ ID NO: 1 or its partial peptide, ④ an antibody to a G protein-coupled receptor protein containing the same or substantially the same amino acid sequence as the amino acid sequence represented by SEQ ID NO: 1, its partial peptide, or a salt thereof, or ⑤ a polynucleotide comprising a whole or part of the base sequence complementary to a polynucleotide comprising a polynucleotide encoding a G protein-coupled receptor protein containing the same or substantially the same amino acid sequence as the amino acid sequence represented by SEQ ID NO: 1 or its partial peptide; and,

(28) Use of ① a compound or its salt that alters the binding properties of an isoprenoid-related substance to a G protein-coupled receptor protein containing the same or substantially the same amino acid sequence as the amino acid sequence represented by SEQ ID NO: 1 or its salt, ② a G protein-coupled receptor protein containing the same or substantially the same amino acid sequence as the amino acid sequence represented by SEQ ID NO: 1, its partial peptide, or a salt thereof, ③ a polynucleotide containing a polynucleotide encoding a G protein-coupled receptor protein containing the same or substantially the same amino acid sequence as the amino acid sequence represented by SEQ ID NO: 1 or its partial peptide, ④ an antibody to a G protein-coupled receptor protein containing the same or substantially the same amino acid sequence as the amino acid sequence represented by SEQ ID NO: 1, its partial peptide, or a salt thereof, or ⑤ a polynucleotide comprising a whole or part of the base sequence complementary to a polynucleotide comprising a polynucleotide encoding a G protein-coupled receptor protein containing the same or substantially the same amino acid sequence as the amino acid sequence represented by SEQ ID NO: 1 or its partial peptide; for manufacturing a cell growth regulator, a cell differentiation regulator or an apoptosis regulator.

The present invention further provides the following features.

(29) A pharmaceutical according to (6), which is a preventive/therapeutic agent for central dysfunctions (e.g., Alzheimer's disease, senile dementia, suppression of eating, etc.), inflammatory diseases (e.g., allergic disease, asthma, rheumatism, etc.), cardiovascular diseases (e.g., hypertension, cardiac hypertrophy, angina pectoris, arteriosclerosis, etc.), cancer (e.g., non-small cell lung carcinoma, ovarian cancer, prostate cancer, stomach cancer, bladder cancer, breast cancer, uterocervical cancer, colon cancer, rectum cancer, etc.), mellitus diabetes, immune system diseases (e.g., autoimmune disease, AIDS, atopic dermatitis, allergic disease, immunodeficiency, asthma, rheumatic arthritis, psoriasis, arteriosclerosis, mellitus diabetes, Alzheimer's disease, etc.), liver/gall bladder diseases (e.g., hepatic cirrhosis, hepatitis, hepatic insufficiency, cholestasis, calculus, etc.), alimentary disorders (e.g., ulcer, enteritis, apepsia, irritable colon syndrome, ulcerative colitis, diarrhea, ileus, etc.), anxiety, pains or obesity;

(30) An agent for the prevention/treatment of central dysfunctions (e.g., Alzheimer's disease, senile dementia, suppression of eating, etc.), inflammatory diseases (e.g., allergic disease, asthma, rheumatism, etc.), cardiovascular diseases (e.g., hypertension, cardiac hypertrophy, angina pectoris, arteriosclerosis, etc.), cancer (e.g., non-small cell lung carcinoma, ovarian cancer, prostate cancer, stomach cancer, bladder cancer, breast cancer, uterocervical cancer, colon cancer, rectum cancer, etc.), mellitus diabetes, immune system diseases (e.g., autoimmune disease, AIDS, atopic dermatitis, allergic disease, immunodeficiency, asthma, rheumatic arthritis, psoriasis, arteriosclerosis, mellitus diabetes, Alzheimer's disease, etc.), liver/gall bladder diseases (e.g., hepatic cirrhosis, hepatitis, hepatic insufficiency, cholestasis, calculus, etc.), alimentary disorders (e.g., ulcer, enteritis, apepsia, irritable colon syndrome, ulcerative colitis, diarrhea, ileus, etc.), anxiety, pains or obesity, comprising an antibody to a G protein-coupled receptor protein containing the same or substantially the same amino acid sequence as the amino acid sequence represented by SEQ ID NO: 1, its partial peptide, or a salt thereof;

(31) A method for diagnosis of central dysfunctions (e.g., Alzheimer's disease, senile dementia, suppression of eating, etc.), inflammatory diseases (e.g., allergic disease, asthma, rheumatism, etc.), cardiovascular diseases (e.g., hypertension, cardiac hypertrophy, angina pectoris, arteriosclerosis, etc.), cancer (e.g., non-small cell lung carcinoma, ovarian cancer, prostate cancer, stomach cancer, bladder cancer, breast cancer, uterocervical cancer, colon cancer, rectum cancer, etc.), mellitus diabetes, immune system diseases (e.g., autoimmune disease, AIDS, atopic dermatitis, allergic disease, immunodeficiency, asthma, rheumatic arthritis, psoriasis, arteriosclerosis, mellitus diabetes, Alzheimer's disease, etc.), liver/gall bladder diseases (e.g., hepatic cirrhosis, hepatitis, hepatic insufficiency, cholestasis, calculus, etc.), alimentary disorders (e.g., ulcer, enteritis, apepsia, irritable colon syndrome, ulcerative colitis, diarrhea, ileus, etc.), anxiety, pains or obesity, which comprises using a method of quantifying a G protein-coupled receptor protein containing the same or substantially the same amino acid sequence as the amino acid sequence represented by SEQ ID NO: 1, using an antibody to said receptor protein, its partial peptide, or a salt thereof;

(32) An agent for the prevention/treatment of central dysfunctions (e.g., Alzheimer's disease, senile dementia, suppression of eating, etc.), inflammatory diseases (e.g., allergic disease, asthma, rheumatism, etc.), cardiovascular diseases (e.g., hypertension, cardiac hypertrophy, angina pectoris, arteriosclerosis, etc.), cancer (e.g., non-small cell lung carcinoma, ovarian cancer, prostate cancer, stomach cancer, bladder cancer, breast cancer, uterocervical cancer, colon cancer, rectum cancer, etc.), mellitus diabetes, immune system diseases (e.g., autoimmune disease, AIDS, atopic dermatitis, allergic disease, immunodeficiency, asthma, rheumatic arthritis, psoriasis, arteriosclerosis, mellitus diabetes, Alzheimer's disease, etc.), liver/gall bladder diseases (e.g., hepatic cirrhosis, hepatitis, hepatic insufficiency, cholestasis, calculus, etc.), alimentary disorders (e.g., ulcer, enteritis, apepsia, irritable colon syndrome, ulcerative colitis, diarrhea, ileus, etc.), anxiety, pains or obesity, comprising a DNA containing a DNA encoding a G protein-coupled receptor protein containing the same or substantially the same amino acid sequence as the amino acid sequence represented by SEQ ID NO: 1;

(33) A method for diagnosis of central dysfunctions (e.g., Alzheimer's disease, senile dementia, suppression of eating, etc.), inflammatory diseases (e.g., allergic disease, asthma, rheumatism, etc.), cardiovascular diseases (e.g., hypertension, cardiac hypertrophy, angina pectoris, arteriosclerosis, etc.), cancer (e.g., non-small cell lung carcinoma, ovarian cancer, prostate cancer, stomach cancer, bladder cancer, breast cancer, uterocervical cancer, colon cancer, rectum cancer, etc.), mellitus diabetes, immune system diseases (e.g., autoimmune disease, AIDS, atopic dermatitis, allergic disease, immunodeficiency, asthma, rheumatic arthritis, psoriasis, arteriosclerosis, mellitus diabetes, Alzheimer's disease, etc.), liver/gall bladder diseases (e.g., hepatic cirrhosis, hepatitis, hepatic insufficiency, cholestasis, calculus, etc.), alimentary disorders (e.g., ulcer, enteritis, apepsia, irritable colon syndrome, ulcerative colitis, diarrhea, ileus, etc.), anxiety, pains or obesity, which comprises using a DNA containing a DNA encoding a G protein-coupled receptor protein containing the same or substantially the same amino acid sequence as the amino acid sequence represented by SEQ ID NO: 1;

(34) An agent for the prevention/treatment of central dysfunctions (e.g., Alzheimer's disease, senile dementia, suppression of eating, etc.), inflammatory diseases (e.g., allergic disease, asthma, rheumatism, etc.), cardiovascular diseases (e.g., hypertension, cardiac hypertrophy, angina pectoris, arteriosclerosis, etc.), cancer (e.g., non-small cell lung carcinoma, ovarian cancer, prostate cancer, stomach cancer, bladder cancer, breast cancer, uterocervical cancer, colon cancer, rectum cancer, etc.), mellitus diabetes, immune system diseases (e.g., autoimmune disease, AIDS, atopic dermatitis, allergic disease, immunodeficiency, asthma, rheumatic arthritis, psoriasis, arteriosclerosis, mellitus diabetes, Alzheimer's disease, etc.), liver/gall bladder diseases (e.g., hepatic cirrhosis, hepatitis, hepatic insufficiency, cholestasis, calculus, etc.), alimentary disorders (e.g., ulcer, enteritis, apepsia, irritable colon syndrome, ulcerative colitis, diarrhea, ileus, etc.), anxiety, pains or obesity, comprising a compound or its salt that alters the expression level of a G protein-coupled receptor protein containing the same or substantially the same amino acid sequence as the amino acid sequence represented by SEQ ID NO: 1, or its salt;

(35) A screening method according to (1), wherein the protein contains a) the amino acid sequence represented by SEQ ID NO: 1 or SEQ ID NO: 6, wherein 1 or at least 2 (preferably about 1-30, more preferably about 1-10 and most preferably several (1-5) amino acids are deleted, b) the amino acid sequence represented by SEQ ID NO: 1 or SEQ ID NO: 6, wherein 1 or at least 2 (preferably about 1-30, more preferably about 1-10 and most preferably several (1-5) amino acids are added, c) the amino acid sequence represented by SEQ ID NO: 1 or SEQ ID NO: 6, wherein 1 or at least 2 (preferably about 1-30, more preferably about 1-10 and most preferably several (1-5) amino acids are replaced by other amino acids, or d) a combination of these amino acid sequences;

(36) A screening kit according to (5), which comprises a cell containing the G protein-coupled receptor protein according to (1);

(37) A screening kit according to (5), which comprises a cell membrane containing the G protein-coupled receptor protein according to (1);

(38) A screening kit according to (5), which comprises a G protein-coupled receptor protein expressed in a cell membrane of a transformant by culturing the transformant, which is transformed with a recombinant vector containing a DNA containing a DNA encoding the G protein-coupled receptor protein according to (1); and the like.

BRIEF DESCRIPTION OF THE DRAWINGS

FIG. 1 shows hydrophobicity plotting of the G protein-coupled receptor protein of the present invention, prepared based on the amino acid sequence represented by SEQ ID NO: 1.

FIG. 2 shows comparison in homology on the amino acid sequence between the G protein-coupled receptor protein of the present invention having the amino acid sequence represented by SEQ ID NO: 1 and MAS, wherein the upper column and the lower column represent the amino acid sequences of the G protein-coupled receptor protein of the present invention and MAS, respectively.

FIG. 3 shows the amino acid sequence represented by SEQ ID NO: 6 and the base sequence represented by SEQ ID NO: 7 (wherein the upper column is the base sequence and the lower column is the amino acid sequence) (continued on FIG. 4).

FIG. 4 shows the amino acid sequence represented by SEQ ID NO: 6 and the base sequence represented by SEQ ID NO: 7 (wherein the upper column is the base sequence and the lower column is the amino acid sequence) (continued from FIG. 3).

FIG. 5 shows the results of the ligand activity by reporter assay using HEK cells in which H7TMB56 reporter is not expressed. On the abscissa, FSK- denotes the results when no forskolin was added and FSK+ denotes the results when forskolin was added. On the abscissa, the symbols denote the results when the following were added, respectively; Basal: neither forskolin nor test compound; 1C7: 15(S)-HPEDE; 1D3: 5(S), 6(R)-DIHETE; 4B10: ADP; 4B11: testosterone; 4E8: sphingosine-1-phosphate; 5H4 farnesyl pyrophosphate, none: no test compound; FSK: forskolin; 2F6: 1,2-dioctanoyl-SN-glycerol; 4G9: sphingomyelin; and 5B3: geranylgeranyl pyrophosphate. Luciferase on the ordinate denotes the luciferase activity (cps).

FIG. 6 shows the results obtained by measuring the ligand activity by reporter assay using HEK cells, in which H7TMB56 is not expressed. On the abscissa, FSK- denotes the results when no forskolin was added and FSK+ denotes when forskolin was added. On the abscissa, the symbols denote the results when the following were added, respectively; Basal: neither forskolin nor test compound; 1C7: 15(S)-HPEDE; 1D3: 5(S), 6(R-DIHETE; 4B10: ADP; 4B11: testosterone; 4E8: sphingosine-1-phosphate; 5H4: farnesyl pyrophosphate, none: no test compound; FSK: forskolin; 2F6: 1,2-dioctanoyl-SN-glycerol; 4G9: sphingomyelin; and 5B3: geranylgeranyl pyrophosphate. Luciferase on the ordinate denotes the luciferase activity (cps).

FIG. 7 shows the results obtained by measuring the ligand activity by reporter assay using HEK cells, in which H7TMB56 is expressed. On the abscissa, FSK- denotes the results when no forskolin was added and FSK+ represents the results when forskolin was added. On the abscissa, the symbols denote the results when the following were added, respectively; Basal: neither forskolin nor test compound; 1C7: 15(S)-HPEDE; 1D3: 5(S), 6(R)-DIHETE; 4B10: ADP; 4B11: testosterone; 4E8: sphingosine-1 -phosphate; 5H4: farnesyl pyrophosphate, none: no test compound; FSK: forskolin; 2F6: 1,2-dioctanoyl-SN-glycerol; 4G9: sphingomyelin; and 5B3: geranylgeranyl pyrophosphate. Luciferase on the ordinate denotes the luciferase activity (cps).

FIG. 8 shows the results obtained by assaying the cAMP production promoting activity of farnesyl pyrophosphate and geranyl geranyl pyrophosphate on mock cells. The ordinate shows the cAMP level (unit: pmol) per well when cAMP was quantified with its assay kit. Numerals on the abscissa denote the levels of farnesyl pyrophosphate and geranyl geranyl pyrophosphate in a logarithmic way. On the abscissa, basal and FSK denote the results when none was added and when foskolin was added, respectively.

FIG. 9 shows the results obtained by assaying the cAMP production promoting activity of farnesyl pyrophosphate and geranyl geranyl pyrophosphate on H7TMB56 receptor-producing cells on a high level. The ordinate shows the cAMP level (unit: pmol) per well when cAMP was quantified with its assay kit. Numerical values on the abscissa denote the levels of farnesyl pyrophosphate and geranyl geranyl pyrophosphate by logarithm. On the abscissa, basal and FSK represent the results when none was added and when foskolin was added, respectively.

FIG. 10 shows the results obtained by assaying the agonist activity of various isoprenoid-related compounds on CHO cell clones capable of producing H7TMB56 receptor on a high level. The ordinate shows the cAMP level (unit: pmol) per well when cAMP was quantified with its assay kit. On the abscissa; numerals represent 1: isopentenyl pyrophosphate, 2: dimethylallyl pyrophosphate, 3: geranyl pyrophosphate, 4: farnesyl pyrophosphate, 5: geranylgeranyl pyrophosphate, 6: geraniol, 7: farnesol, 8: geranylgeraniol, 9: trans, trans-farnesol, 10: trans, trans-farnesyl bromide, 11: geranyl acetone, 12: geranyl acetate, 13: geranylamine and 14: geranyl chloride, respectively. On the abscissa, basal and FSK represent the results when added with none and when added with foskolin, respectively.

FIG. 11 shows the results obtained by assaying the agonist activity of various isoprenoid-related compounds on CHO-mock cell clones. The ordinate shows the CAMP level (unit: pmol) per well when cAMP was quantified with its assay kit. On the abscissa, numerals represent 1: isopentenyl pyrophosphate, 2: dimethylallyl pyrophosphate, 3: geranyl pyrophosphate, 4: farnesyl pyrophosphate, 5: geranylgeranyl pyrophosphate, 6: geraniol, 7: farnesol, 8: geranylgeraniol, 9: trans, trans-famesol, 10: trans, trans-farnesyl bromide, 11: geranyl acetone, 12: geranyl acetate, 13: geranylamine and 14: geranyl chloride, respectively. On the abscissa, basal and FSK represent the results when added with none and when added with foskolin, respectively.

FIG. 12 shows the results obtained by assaying the intracellular calcium ion level change stimulating activity by geranyl pyrophosphate (GPP; open circle, closed circle), farnesyl pyrophosphate (FPP; open triangle, closed triangle) and geranylgeranyl pyrophosphate (GGPP; open square, closed square) on H7TMB56 receptor-highly expressing CHO cell clones (open circle, open triangle, open square) and mock cells (closed circle, closed triangle, closed square). Numerical values on the abscissa represent the doses of GPP, FPP and GGPP in a logarithmic way. The ordinate represents the integrated values of fluorescence counts.

FIG. 13 shows the results obtained by assaying the intracellular cAMP production promoting activities of various isoprenoid-related compounds on the CHO cell clone highly expressing H7TMB56 receptor. The ordinate represents the cAMP level (unit: pmol) per well when cAMP was quantified by an assay kit. Concentration (M) on the abscissa represents concentrations of various isoprenoid-related compounds. Symbols denote open circle: the results when added with farnesyl pyrophosphate (farnesyl PP), closed square: with geranyl pyrophosphate and closed triangle: with geranylgeranyl pyrophosphate (geranylgeranyl PP), respectively.

FIG. 14 shows the results of detection of active MAP kinase in mock cells (lanes 1 to 3) and H7TMB56 expression cells (lanes 4 to 6). Lanes 1 and 4 denote the results obtained by treating farnesyl pyrophosphate for 0 minute, subjecting the resulting cell extract to electrophoresis and then detecting by western blotting; lanes 2 and 5 denote the results likewise obtained 5 minutes after the farnesyl pyrophosphate treatment; and lanes 3 and 6 denote the results obtained 10 minutes after the farnesyl pyrophosphate treatment.

FIG. 15 shows tissue distribution in the expression of H7TMB56 gene mRNA in human by RT-PCR.

## BEST MODE FOR CARRYING OUT THE INVENTION

The G protein-coupled receptor protein of the present invention (hereinafter sometimes merely referred to also as the receptor protein) is a receptor protein, which contains the same or substantially the same amino acid sequence as the amino acid sequence shown by SEQ ID NO: 1.

The receptor protein of the present invention may be any protein derived from any cells (e.g., splenocytes, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells, the corresponding precursor cells, stem cells, cancer cells, etc.), hemocyte type cells, or any tissues where such cells are present, e.g., brain or any region of the brain (e.g., olfactory bulb, amygdaloid nucleus, basal ganglia, hippocampus, thalamus, hypothalamus, subthalamic nucleus, cerebral cortex, medulla oblongata, cerebellum, occipital pole, frontal lobe, temporal lobe, putamen, caudate nucleus, corpus callosum, substantia nigra), spinal cord, pituitary gland, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, peripheral blood cells, prostate, testicles, testis, uterus, placenta, uterus, bone, joint, skeletal muscle, etc., from human and other mammals (e.g., guinea pigs, rats, mice, rabbits, swine, sheep, bovine, monkeys, etc.). The receptor protein may also be a synthetic protein.

The amino acid sequence which has substantially the same amino acid sequence as that represented by SEQ ID NO: 1 includes an amino acid sequence having at least about 50% homology, preferably at least about 70% homology, more preferably at least about 80% homology, much more preferably at least about 90% homology, and most preferably at least about 95% homology, to the amino acid sequence represented by SEQ ID NO: 1; etc.

Examples of the protein which contains substantially the same amino acid sequence as that shown by SEQ ID NO: 1 include a protein having substantially the same amino acid sequence as that shown by SEQ ID NO: 1 and having the activity substantially equivalent to the amino acid sequence represented by SEQ ID NO: 1, etc.

More specifically, the protein containing substantially the same amino acid sequence as that shown by SEQ ID NO: 1 is, e.g., a protein containing the amino acid sequence represented by SEQ ID NO: 6, etc.

Examples of the substantially equivalent activity include a ligand binding activity, a signal transduction activity, etc. The term substantially equivalent is used to mean that the nature of the activity is the same. Therefore, although it is preferred that activities such as the ligand binding, signal transduction activities, etc. be equivalent (e.g., about 0.01- to about 100-fold, preferably about 0.5- to about 20-fold, more preferably about 0.5- to about 2-fold), quantitative factors such as a level of the activity, a molecular weight of the protein, etc. may differ.

The activities such as ligand binding, signal transduction activities or the like can be determined according to a publicly known method with some modifications, for example, by the ligand determination methods or the screening methods that will be later described.

Proteins containing the following amino acid sequences may also be used as the receptor protein of the present invention: ① amino acid sequences represented by SEQ ID NO: 1 or SEQ ID NO: 6, wherein at least 1 or 2 amino acids (preferably approximately 1 to 30 amino acids, more preferably approximately 1 to 10 amino acids, most preferably several (1 to 5) amino acids) are deleted; ② amino acid sequences represented by SEQ ID NO: 1 or SEQ ID NO: 6, to which at least 1 or 2 amino acids (preferably approximately 1 to 30 amino acids, more preferably approximately 1 to 10 amino acids, and most preferably several (1 to 5) amino acids) are added; ③ amino acid sequences represented by SEQ ID NO: 1 or SEQ ID NO: 6, in which at least 1 or 2 amino acids (preferably approximately 1 to 30 amino acids, more preferably approximately 1 to 10 amino acids, and most preferably several (1 to 5) amino acids) are substituted by other amino acids; or ④ combination of the amino acid sequences described in the above.

Throughout the present specification, the receptor proteins are represented in accordance with the conventional way of describing peptides, that is, the N-terminus (amino terminus) at the left hand and the C-terminus (carboxyl terminus) at the right hand. In the receptor proteins of the present invention including the receptor proteins containing the amino acid sequence shown by SEQ ID NO: 1, the C-terminus may be in any form of a carboxyl group (-COOH), a carboxylate ($-COO^-$), an amide ($-CONH_2$) and an ester (-COOR).

Examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, α-naphthyl, etc.; a $C_{7-14}$ aralkyl group such as a phenyl-$C_{1-2}$-alkyl group, e.g., benzyl, phenethyl, etc., or an α-naphthyl-$C_{1-2}$-alkyl group such as α-naphthylmethyl, etc.; and the like. In addition, pivaloyloxymethyl or the like, which is used widely as an ester for oral administration, may also be used.

Where the receptor protein of the present invention contains a carboxyl group (or a carboxylate) at a position other than the C-terminus, it may be amidated or esterified and such an amide or ester is also included within the receptor protein of the present invention. The ester group may be the same group as that described with respect to the C-terminus described above.

Furthermore, examples of the receptor protein of the present invention include variants of the above receptor proteins, wherein the amino group at the N-terminal methionine residue of the protein supra is protected with a protecting group (for example, a $C_{1-6}$ acyl group such as a $C_{2-6}$ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{2-6}$ alkanoyl group, e.g., formyl group, acetyl group, etc.), or conjugated proteins such as glycoproteins bound to sugar chains.

Specific examples of the receptor protein of the present invention which can be used include a receptor protein derived from human (more preferably derived from human hippocampus) containing an amino acid sequence represented by SEQ ID NO: 1 or SEQ ID NO: 6, etc.

As partial peptides of the receptor protein of the present invention (hereinafter sometimes referred to as the partial peptides), any partial peptide can be used so long as it can be a partial peptide of the receptor protein of the present invention. Among the receptor protein molecules of the present invention, for example, those having a site exposed to the outside of a cell membrane and having a receptor binding activity can be used.

Specifically, the partial peptide of the receptor protein having the amino acid sequence represented by SEQ ID NO: 1 is a peptide containing the parts analyzed to be extracellular domains (hydrophilic domains) in the hydrophobic plotting analysis shown in FIG. 1. A peptide containing a hydrophobic domain in part can be used as well. In addition, the peptide may contain each domain separately or plural domains together.

In the receptor protein of the present invention, preferred partial peptides are those having at least 20, preferably at least 50, and more preferably at least 100 amino acids, in the amino acid sequence which constitutes the receptor protein of the present invention described above.

Herein, the term "activity substantially equivalent" refers to the same significance as defined above. The "activity substantially equivalent" can be assayed in the same manner as given above.

The partial peptide of the present invention may contain the amino acid sequence described above, in which at least 1 or 2 amino acids (preferably approximately 1 to 10 amino acids, more preferably several (1 to 5) amino acids) are deleted; to which at least 1 or 2 amino acids (preferably approximately I to 20 amino acids, more preferably approximately 1 to 10 amino acids, and most preferably several (1 to 5) amino acids) are added; or, in which at least 1 or 2 amino acids (preferably approximately 1 to 10 amino acids, more preferably several and most preferably approximately 1 to 5 amino acids) are substituted by other amino acids.

In the partial peptide of the present invention, the C-terminus may be in any form of a carboxyl group (-COOH), a carboxylate ($-COO^-$), an amide ($-CONH_2$) and an ester (-COOR).

As in the receptor protein of the present invention described above, the partial peptide of the present invention further includes those in which the amino, group of the amino acid residue in the N-terminal methionine residue is protected by a protecting group, those in which the N-terminal region is cleaved in vivo and the produced Gln is pyroglutaminated, those in which substituents on the side chains of amino acids in the molecule are protected by appropriate protecting groups, conjugated peptides such as so-called glycopeptides, to which sugar chains are bound, and the like.

For salts of the receptor protein of the present invention or its partial peptides, there are salts with physiologically acceptable acids, especially preferred are physiologically acceptable acid addition salts. Examples of these salts include salts with, for example, inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The receptor protein of the present invention or salts thereof may be manufactured by a publicly known method used to purify a receptor protein from human and other mammalian cells or tissues described above, or by culturing a transformant that contains the DNA encoding the receptor protein of the present invention, as will be later described. Furthermore, the receptor protein or its salts may also be manufactured by the methods for synthesizing proteins or by modifications thereof, which will also be described hereinafter.

Where the receptor protein or its salts are manufactured from human or other mammalian tissues or cells, human or other mammalian tissues or cells are homogenized, then extracted with an acid or the like, and the extract is isolated and purified by a combination of chromatography techniques such as reverse phase chromatography, ion exchange chromatography, and the like.

To synthesize the receptor protein of the present invention, its partial peptide, or salts or amides thereof, commercially available resins that are used for protein synthesis may be used in general. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmehtylphenyl acetamidomethyl resin, polyacrylamide resin, 4-(2',4'-dimethoxyphenylhydroxymethyl)phenoxy resin, 4-(2',4'-dimethoxyphenyl-Fmoc-aminoethyl) phenoxy resin, etc. Using these resins, amino acids in which $\alpha$-amino groups and functional groups on the side chains are appropriately protected are condensed on the resin in the order of the sequence of the objective protein according to various condensation methods publicly known in the art. At the end of the reaction, the protein is cut out from the resin and at the same time, the protecting groups are removed. Then, intramolecular disulfide bond-forming reaction is performed in a highly diluted solution to obtain the objective protein or amides thereof.

For condensation of the protected amino acids described above, a variety of activation reagents for protein synthesis may be used, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; pyridine; ethers such as dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein bond-forming reactions and is usually selected in the range of approximately -20°C to 50°C. The activated amino acid derivatives are used generally in an excess of 1.5 to 4 times. The condensation is examined by a test using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole.

Examples of the protecting groups used to protect the amino groups of the starting compounds include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of the groups appropriately used for the esterification include a lower alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting compounds include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the activated amino acids, in which the amino groups are activated in the starting material, the corresponding phosphoric amides are employed.

To eliminate (split off) the protecting groups, there are used catalytic reduction under hydrogen gas flow in the presence of a catalyst such as Pd-black, Pd-carbon, etc.; an acid treatment with anhydrous hydrofluoric acid, methanesulfonic acid, trifluoromethane-sulfonic acid or trifluoroacetic acid, or a mixture solution of these acids; a treatment with a base such as diisopropylethylamine, triethylamine, piperidine, piperazine, etc.; and reduction with sodium in liquid ammonia; or the like. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Fonnyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of the functional groups that should not be involved in the reaction of the starting materials, protecting groups, elimination of the protecting groups and activation of the functional groups involved in the reaction may be appropriately selected from publicly known groups and publicly known means.

In another method for obtaining the amides of the protein, for example, the α-carboxyl group of the carboxy terminal amino acid is first protected by amidation; the peptide (protein) chain is then extended to a desired length toward the amino group side. Thereafter, a protein in which only the protecting group of the N-terminal α-amino group in the peptide chain has been eliminated from the protein and a protein in which only the protecting group of the C-terminal carboxyl group has been eliminated are prepared. The two proteins are condensed in a mixture of the solvents described above. The details of the condensation reaction are the same as described above. After the protected protein obtained by the condensation is purified, all the protecting groups are eliminated by the method described above to give the desired crude protein. This crude protein is purified by various known purification means. Lyophilization of the major fraction gives the amide of the desired protein.

To prepare the esterified protein, for example, the α-carboxyl group of the carboxy terminal amino acid is condensed with a desired alcohol to prepare the amino acid ester, which is followed by procedure similar to the preparation of the amidated protein above to give the ester form of the desired protein.

The partial peptide or its salts in the protein of the present invention can be manufactured by publicly known methods for peptide synthesis, or by cleaving the protein of the present invention with an appropriate peptidase. For the methods for peptide synthesis, for example, either solid phase synthesis or liquid phase synthesis may be used. That is, the partial peptide or amino acids that can construct the protein of the present invention are condensed with the remaining part. Where the product contains protecting groups, these protecting groups are removed to give the desired peptide. Publicly known methods for condensation and elimination of the protecting groups are described in ① - ⑤ below.

① M. Bodanszky & M.A. Ondetti: Peptide Synthesis, Interscience Publishers, New York (1966)
② Schroeder & Luebke: The Peptide, Academic Press, New York (1965)
③ Nobuo Izumiya, et al.: Peptide Gosei-no-Kiso to Jikken (Basics and experiments of peptide synthesis), published by Maruzen Co. (1975)
④ Haruaki Yajima & Shunpei Sakakibara: Seikagaku Jikken Koza (Biochemical Experiment) 1, Tanpakushitsu no Kagaku (Chemistry of Proteins) IV, 205 (1977)
⑤ Haruaki Yajima, ed.: Zoku Iyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography, recrystallization, etc. to give the partial peptide of the present invention. When the partial peptide obtained by the above methods is in a free form, the peptide can be converted into an appropriate salt by a publicly known method; when the protein is obtained in a salt form, it can be converted into a free form by a publicly known method.

The polynucleotide encoding the receptor protein of the present invention may be any polynucleotide so long as it contains the base sequence (DNA or RNA, preferably DNA) encoding the receptor protein of the present invention described above. The polynucleotide may also be any one of DNA encoding the receptor protein of the present invention, RNA such as mRNA, etc., and may be double-stranded or single-stranded. Where the polynucleotide is double-stranded, it may be double-stranded DNA, double-stranded RNA or DNA:RNA hybrid. Where the polynucleotide is single-stranded, it may be a sense strand (i.e., a coding strand) or an antisense strand (i.e., a non-coding strand).

The DNA encoding the receptor protein of the present invention may be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid, phagemid, etc. The DNA may also be directly amplified by reverse transcriptase polymerase chain reaction (hereinafter abbreviated as RT-PCR) using the total RNA or mRNA fraction prepared from the cells and tissues described above.

Specifically, the DNA encoding the receptor protein of the present invention may be DNA having the base sequence shown by SEQ ID NO: 2, SEQ ID NO: 3 or SEQ ID NO: 7, or DNA having the base sequence hybridizable to the base sequence represented by SEQ ID NO: 2, SEQ ID NO: 3 or SEQ ID NO: 7 under highly stringent conditions and encoding a receptor protein having the activities substantially equivalent to those of the receptor protein of the present invention (e.g., a ligand binding activity, a signal transduction activity, etc.).

Examples of the DNA hybridizable to the base sequence represented by SEQ ID NO: 2, SEQ ID NO: 3 or SEQ ID NO: 7 under highly stringent conditions include DNA containing a base sequence having at least about 70% homology, preferably at least about 80% homology, more preferably at least about 90% homology and further more preferably at least about 95 % homology, to the base sequence represented by SEQ ID NO: 2, SEQ ID NO: 3 or SEQ ID NO: 7.

As is clear from REFERENCE EXAMPLES 1 to 3 later described, the presence of DNA containing the base sequence represented by SEQ ID NO: 2, SEQ ID NO: 3 or SEQ ID NO: 7 suggests genetic polymorphism and utilization of this polymorphism enables to apply to diagnostics, etc.

The hybridization can be carried out by publicly known methods or by modifications of these methods, for example, according to the method described in Molecular Cloning, 2nd (J. Sambrook et al., Cold Spring Harbor Lab. Press, 1989). A commercially available library may also be used according to the instructions of the attached manufacturer's protocol. Preferably, the hybridization can be carried out under highly stringent conditions.

The highly stringent conditions are, for example, those in a sodium concentration at about 19 - 40 mM, preferably about 19 - 20 mM at a temperature of about 50 - 70°C, preferably about 60 - 65°C. In particular, hybridization conditions in a sodium concentration of about 19 mM at a temperature of about 65°C are most preferred.

More specifically, for the DNA encoding the receptor protein containing the amino acid sequence represented by SEQ ID NO: 1, there may be employed DNA containing the base sequence represented by SEQ ID NO: 2 or SEQ ID NO: 3; and for the DNA encoding the receptor protein containing the amino acid sequence represented by SEQ ID NO: 6, DNA containing the base sequence represented by SEQ ID NO: 7 is used.

The polynucleotide comprising a part of the base sequence of the DNA encoding the receptor protein of the present invention or a part of the base sequence complementary to the DNA is used to mean to include not only the DNA encoding the partial peptide of the present invention described below but also RNA.

According to the present invention, antisense polynucleotides (nucleic acids) that can inhibit the replication or expression of G protein-coupled receptor protein genes can be designed and synthesized based on the base sequence information of the cloned or determined DNA encoding the G protein-coupled receptor protein and non-translated region thereof. Such a polynucleotide (nucleic acid) is capable of hybridizing to RNA of G protein-coupled receptor protein gene and its non-translated region to inhibit the synthesis or function of said RNA or capable of modulating or controlling the expression of G protein-coupled receptor protein gene via interaction with G protein-coupled receptor protein-associated RNA. When the polynucleotides complementary to the selected sequences of RNA associated with the G protein-coupled receptor protein and polynucleotides specifically hybridizable to the G protein-coupled receptor protein-associated RNA are used, the expression level of the G protein-coupled receptor protein gene can be determined or modulated (control of overexpression, potentiation of expression, etc., depending on necessity) in vivo and in vitro by publicly known methods. That is, the polynucleotides are useful for the diagnosis, prevention or/and treatment of diseases associated with deficiency or dysfunction of the G protein-coupled receptor protein. The said term "corresponding" is used to mean homologous to or complementary to a particular sequence of the nucleotide, base sequence or nucleic acid, including the gene. The term "corresponding" between nucleotides, base sequences or nucleic acids and peptides (proteins) usually refer to amino acids of a peptide (protein) under the order derived from the sequence of nucleotides (nucleic acids) or their complements. In the G protein-coupled receptor protein genes, the 5' end hairpin loop, 5' end 6-base-pair repeats, 5' end untranslated region, polypeptide translation initiation codon, protein coding region, ORF translation termination codon, 3' end untranslated region, 3' end palindrome region, and 3' end hairpin loop, may be selected as preferred target regions, though any other region may be selected as a target in the G protein-coupled receptor protein genes.

The relationship between the targeted nucleic acids and the polynucleotides complementary to at least a part of the target region, specifically the relationship between the target and the polynucleotides hybridizable to the target, can be denoted to be "antisense". Examples of the antisense polynucleotides include polydeoxynucleotides containing 2-deoxy-D-ribose, polynucleotides containing D-ribose, any other type of polynucleotides which are N-glycosides of a purine or pyrimidine base, or other polymers containing non-nucleotide backbones (e.g., proteins, nucleic acids and synthetic sequence-specific nucleic acid polymers, commercially available) or other polymers containing special linkages (provided that the polymers contain nucleotides having such a configuration that allows base pairing or base stacking, as found in DNA or RNA), etc. The antisense polynucleotides may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with markers known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., $\alpha$ anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide (nucleic acid) of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleic acids of the present invention can be modified preferably based on the following design, that is, by increasing the intracellular stability of the antisense nucleic acid, increasing the cellular permeability of the antisense nucleic acid, increasing the affinity of the nucleic acid to the targeted sense strand to a higher level, or minimizing the toxicity, if any, of the antisense nucleic acid.

Many of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense nucleic acid of the present invention may contain altered or modified sugars, bases or linkages. The antisense nucleic acid may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory action of the antisense nucleic acid can be examined using the transformant of the present invention, the gene expression system of the present invention in vivo and in vitro, or the translation system of the G protein-coupled receptor protein in vivo and in vitro. The nucleic acid can be applied to cells by a variety of publicly known methods.

The DNA encoding the partial peptide of the present invention may be any DNA so long as it contains the base sequence encoding the partial peptide of the present invention described above. The DNA may also be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid, phagemid, etc. The DNA may also be directly amplified by reverse transcriptase polymerase chain reaction (hereinafter abbreviated as RT-PCR) using mRNA fraction prepared from the cells and tissues described above.

Specifically, the DNA encoding the partial peptide of the present invention may be any one of, for example, (1) a DNA containing a partial base sequence of the DNA having the base sequence represented by SEQ ID NO: 2, SEQ ID NO: 3 or SEQ ID NO: 7, or (2) a DNA containing a partial base sequence of the DNA having a DNA hybridizable to the DNA represented by SEQ ID NO: 2, SEQ ID NO: 3 or SEQ ID NO: 7 under highly stringent conditions and encoding a protein which has the activities (e.g., a ligand-biding activity, a signal transduction activity, etc.) substantially equivalent to those of the receptor protein peptide of the present invention; etc.

Specific examples of the DNA that is hybridizable to the DNA represented by SEQ ID NO: 2, SEQ ID NO: 3 or SEQ ID NO: 7 under highly stringent conditions include DNA containing a base sequence having at least about 70% homology, preferably at least about 80% homology, more preferably at least about 90% homology and further more preferably at least about 95% homology, to the base sequence represented by SEQ ID NO: 2, SEQ ID NO: 3 or SEQ ID NO: 7.

For cloning of the DNA that completely encodes the receptor protein of the present invention or its partial peptide (hereinafter sometimes collectively referred to as the receptor protein of the present invention), the DNA may be either amplified by PCR using synthetic DNA primers containing a part of the base sequence of the receptor protein of the present invention, or the DNA inserted into an appropriate vector can be selected by hybridization with a labeled DNA fragment or synthetic DNA that encodes a part or entire region of the receptor protein of the present invention. The hybridization can be carried out, for example, according to the method described in Molecular Cloning, 2nd (J. Sambrook et al., Cold Spring Harbor Lab. Press, 1989). The hybridization may also be performed using commercially available library in accordance with the protocol described in the attached instructions.

Conversion of the base sequence of the DNA can be effected by publicly known methods such as the ODA-LA PCR method, the Gapped duplex method or the Kunkel method, or its modification by using a publicly known kit available as Mutant™-super Express Km (Takara Shuzo Co., Ltd.), Mutan™-K (Takara Shuzo Co., Ltd.), etc.

The cloned DNA encoding the receptor protein can be used as it is, depending upon purpose or, if desired, after digestion with a restriction enzyme or after addition of a linker thereto. The DNA may contain ATG as a translation initiation codon at the 5' end thereof and may further contain TAA, TGA or TAG as a translation termination codon at the 3' end thereof. These translation initiation and termination codons may also be added by using an appropriate synthetic DNA adapter.

The expression vector for the receptor protein of the present invention can be manufactured, for example, by (a) excising the desired DNA fragment from the DNA encoding the receptor protein of the present invention, and then (b) ligating the DNA fragment with an appropriate expression vector downstream a promoter in the vector.

Examples of the vector include plasmids derived form E. coli (e.g., pCR4, pCR2.1, pBR322, pBR325, pUC12, pUC13), plasmids derived from Bacillus subtilis (e.g., pUB110, pTP5, pC 194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, CMV promoter or SRα promoter is preferably used. Where the host is bacteria of the genus Escherichia, preferred examples of the promoter include trp promoter, lac promoter, recA promoter, λPL promoter, lpp promoter, etc. In the case of using bacteria of the genus Bacillus as the host, preferred example of the promoter are SPO1 promoter, SPO2 promoter and penP promoter. When yeast is used as the host, preferred examples of the promoter are PHO5 promoter, PGK promoter, GAP promoter and ADH promoter. When insect cells are used as the host, preferred examples of the promoter include polyhedrin prompter, P10 promoter, etc.

In addition to the foregoing examples, the expression vector may further optionally contain an enhancer, a splicing signal, a polyA addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as $Amp^r$), neomycin resistant gene (hereinafter sometimes abbreviated as $Neo^r$, G418 resistance), etc. In particular, when dhfr gene is used as the selection marker in CHO ($dhfr^-$) cells, selection can also be made on thymidine free media.

If necessary, a signal sequence that matches with a host is added to the N-terminus of the receptor protein of the present invention. Examples of the signal sequence that can be used are Pho A signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector containing the DNA encoding the receptor protein of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, $AH22R^-$, NA87-11A, DKD-5D, 20B-12, Schizosaccharomyces pombe NCYC1913, NCYC2036, Pichia pastoris, etc.

Examples of insect cells include, for the virus AcNPV, Spodoptera frugiperda cells (Sf cells), MG1 cells derived from mid-intestine of Trichoplusia ni, High FiveTM cells derived from egg of Trichoplusia ni, cells derived from Mamestra brassicae, cells derived from Estigmena acrea, etc.; and for the virus BmNPV, Bombyx mori N cells (BmN cells), etc. are used. Examples of the Sf cell which can be used are Sf9 cells (ATCC CRL1711), Sf21 cells (both cells are described in Vaughn, J. L. et al., In Vivo, 13, 213-217 (1977), etc.

As the insect, for example, a larva of Bombyx mori, etc. can be used [Maeda, et al., Nature, 315, 592 (1985)].

Examples of animal cells include monkey cells COS-7, Vero, Chinese hamster cells CHO (hereinafter referred to as CHO cells), dhfr gene deficient Chinese hamster cells CHO (hereinafter simply referred to as CHO ($dhfr^-$) cell), mouse L cells, mouse AtT-20, mouse myeloma cells, rat GH3, human FL cells, etc.

Bacteria belonging to the genus Escherichia can be transformed, for example, by the method described in Proc. Natl. Acad. Sci. U.S.A., 69, 2110 (1972), Gene, 17, 107 (1982), etc. Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995), published by Shujunsha, or Virology, 52, 456 (1973).

Thus, the transformant transformed with the expression vector containing the DNA encoding the G protein-coupled receptor protein can be obtained.

Where the host is bacteria belonging to the genus Escherichia or the genus Bacillus, the transformant can be appropriately incubated in a liquid medium which contains materials required for growth of the transformant such as carbon sources, nitrogen sources, inorganic materials, and so on. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast extract, vitamins, growth promoting factors etc. may also be added to the medium. Preferably, pH of the medium is adjusted to about 5 to about 8.

A preferred example of the medium for incubation of the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15 - 43° C for about 3 - 24 hours. If necessary, the culture may be aerated or agitated.

Where the bacteria belonging to the genus Bacillus are used as the host, the transformant is cultivated generally at about 30 - 40° C for about 6 - 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids (Bitter, G. A. et al., Proc. Natl. Acad. Sci. U.S.A., 81, 5330 (1984)). Preferably, pH of the medium is adjusted to about 5 - 8. In general, the transformant is cultivated at about 20 - 35°C for about 24 - 72 hours. If necessary, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

Where animal cells are employed as the host, the transformant is cultivated in, for example, MEM medium containing about 5% to about 20% fetal bovine serum [Science, 122,501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 hours to about 60 hours and, if necessary, the culture can be aerated or agitated.

As described above, the G protein-coupled receptor protein of the present invention can be produced on the cell membrane of the transformant.

The receptor protein of the present invention can be separated and purified from the culture described above by the following procedures.

When the receptor protein of the present invention is extracted from the culture or cells, after incubation the transformants or cells are collected by a publicly known method and suspended in an appropriate buffer. The transformants or cells are then disrupted by publicly known methods such as ultrasonication, a treatment with lysozyme and/or freeze-thaw cycling, followed by centrifugation, filtration, etc. Thus, the crude extract of the receptor protein of the present invention can be obtained. The buffer used for the procedures may contain a protein modifier such as urea, guanidine hydrochloride, etc., or a surfactant such as Triton X-100™, etc. When the receptor protein is secreted in the culture, after completion of the cultivation the supernatant can be separated from the transformants or cells and collected by publicly known methods.

The receptor protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method utilizing mainly difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the receptor protein thus obtained is in a free form, it can be converted into the salt by publicly known methods or modifications thereof. On the other hand, when the receptor protein is obtained in the form of a salt, it can be converted into the free form or in the form of a different salt by publicly known methods or modifications thereof.

The receptor protein produced by the recombinant can be treated, prior to or after the purification, with an appropriate protein modifying enzyme so that the receptor protein can be appropriately modified to partially remove a polypeptide. Examples of the protein-modifying enzyme include trypsin, chymotrypsin, arginyl endopeptidase, protein kinase, glycosidase, or the like.

The activity of the thus produced receptor protein of the present invention or salts thereof can be determined by a test binding to a labeled ligand, by an enzyme immunoassay using a specific antibody; or the like.

Antibodies to the receptor protein of the present invention, its partial peptides, or salts thereof may be any of polyclonal antibodies and monoclonal antibodies, as long as they are capable of recognizing the receptor protein of the present invention, its partial peptides, or salts thereof.

The antibodies to the receptor protein of the present invention, its partial peptides, or salts thereof (hereinafter sometimes merely referred to as the receptor protein, etc. of the present invention) may be manufactured by publicly known methods for manufacturing antibodies or antisera, using as antigens the receptor protein, etc. of the present invention.

[Preparation of monoclonal antibody]

(a) Preparation of monoclonal antibody-producing cells

The receptor protein, etc. of the present invention is administered to mammals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once in every 2 to 6 weeks and approximately 2 to 10 times in total. Examples of the applicable mammals are monkeys, rabbits, dogs, guinea pigs, mice, rats, sheep and goats, with mice and rats being preferred.

In the preparation of monoclonal antibody-producing cells, from warm-blooded animals, e.g., mice immunized with an antigen, the animal wherein the antibody titer is noted is selected, then the spleen or lymph node is collected after 2 to 5 days from the final immunization and antibody-producing cells contained therein are fused with myeloma cells to give monoclonal antibody-producing hybridomas. Measurement of the antibody titer in antisera may be made, for example, by reacting a labeled form of the receptor protein, etc., which will be described later, with the antiserum followed by assaying the binding activity of a marker bound to the antibody. The fusion may be operated, for example, by the known Koehler and Milstein method [Nature, 256, 495 (1975)]. Examples of fusion accelerators are polyethylene glycol (PEG), Sendai virus, etc., of which PEG is preferably employed.

Examples of the myeloma cells are NS-1, P3U1, SP2/0, etc. In particular, P3U1 is preferably employed. A preferred ratio in count of the antibody-producing cells (spleen cells) to the myeloma cells used is within a range of approximately 1:1 to 20:1. When PEG (preferably, PEG 1000 to PEG 6000) is added in a concentration of approximately 10 to 80% followed by incubating at about 20 to about 40°C, preferably at about 30 to about 37°C for about 1 to about 10 minutes, an efficient cell fusion can be carried out.

Various methods can be used for screening of the monoclonal antibody-producing hybridomas. Examples of such methods include a method which comprises adding the hybridoma culture supernatant to a solid phase (e.g., microplate) adsorbed with the receptor protein, etc. as an antigen directly or together with a carrier, adding an anti-immunoglobulin antibody (when mouse cells are used for the cell fusion, anti-mouse immunoglobulin antibody is used) labeled with a radioactive substance or an enzyme, or Protein A and detecting the monoclonal antibody bound to the solid phase; a method which comprises adding the hybridoma culture supernatant to a solid phase adsorbed with an anti-immunoglobulin antibody or Protein A, adding the receptor protein, etc. labeled with a radioactive substance or an enzyme and detecting the monoclonal antibodies bound to the solid phase; and the like.

The monoclonal antibodies can be screened by publicly known methods or by modifications of these methods. In general, the screening can be made in a medium for animal cells supplemented with HAT (hypoxanthine, aminopterin and thymidine). Any screening and growth medium can be employed as far as the hybridoma can grow therein. For example, RPMI 1640 medium containing 1% to 20%, preferably 10% to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.) and the like can be used for the screening and growth medium. The cultivation is carried out generally at 20°C to 40°C, preferably at about 37°C, for 5 days to 3 weeks, preferably 1 to 2 weeks. The incubation can be carried out normally in 5% $CO_2$. The antibody titer in the hybridomas culture supernatant can be assayed as in the assay for the antibody titer in the antisera described above.

(b) Purification of monoclonal antibody

Separation and purification of the monoclonal antibody can be carried out by methods applied to conventional separation and purification of immunoglobulins, as in the conventional methods for separation and purification of polyclonal antibodies [e.g., salting-out, alcohol precipitation, isoelectric point precipitation, electrophoresis, adsorption and desorption with ion exchangers (e.g., DEAE), ultracentrifugation, gel filtration, or a specific purification method which involves collecting only an antibody with an activated adsorbent such as an antigen-binding solid phase, Protein A, Protein G, etc. and dissociating the binding to obtain the antibody].

[Preparation of polyclonal antibody]

The polyclonal antibody of the present invention can be manufactured by publicly known methods or modifications thereof. For example, a complex of immunogen (antigen such as the receptor protein, etc.) and a carrier protein is prepared, and a mammal is immunized with the complex in a manner similar to the method described above for the manufacture of monoclonal antibodies. The product containing the antibody to the receptor protein, etc. of the present invention is collected from the immunized animal followed by separation and purification of the antibody.

In the complex of an immunogen and a carrier protein used to immunize a mammal, the type of carrier protein and the mixing ratio of a carrier to hapten may be any type and in any ratio, as long as the antibody is efficiently produced to the hapten immunized by crosslinking to the carrier. For example, bovine serum albumin, bovine thyroglobulins, keyhole limpet hemocyanin, etc. is coupled to hapten in a carrier-to-hapten weight ratio of approximately 0.1 to 20, preferably about 1 to about 5.

A variety of condensing agents can be used for the coupling of a carrier to hapten. Glutaraldehyde, carbodiimide, maleimide activated ester, activated ester reagents containing thiol group or dithiopyridyl group, etc. are used for the coupling.

The condensation product is administered to warm-blooded animals either solely or together with carriers or diluents to the site in which the antibody can be produced by the administration. In order to potentiate the antibody productivity upon the administration, complete Freund's adjuvant or incomplete Freund's adjuvant may be administered. The administration is usually made once approximately in every 2 to 6 weeks and about 3 to about 10 times in total.

The polyclonal antibody can be collected from the blood, ascites, etc., preferably from the blood of mammals immunized by the method described above.

The polyclonal antibody titer in antisera can be assayed by the same procedure as that for the determination of serum antibody titer described above. The separation and purification of the polyclonal antibody can be carried out, following the method for the separation and purification of immunoglobulins performed as applied to the separation and purification of monoclonal antibodies described hereinabove.

One of the ligands to the receptor protein of the present invention or its salts is a compound, in which a pyrophosphate group is bound to the tail of the recurring structure of at least 2 (preferably 2 to 5) isoprenic units. More specifically, farnesyl pyrophosphate (FPP), geranyl pyrophosphate (GPP), geranylgeranyl pyrophosphate (GGPP), or the like is used as the isoprenoid-related substance.

Thus, the receptor protein of the present invention, its partial peptides, or salts thereof (hereinafter sometimes referred to as the receptor protein of the present invention), the DNA encoding the receptor protein of the present invention or its partial peptides (hereinafter sometimes referred to as the DNA of the present invention) and the antibodies to the receptor protein of the present invention or its partial peptides (hereinafter sometimes referred to as the antibody of the present invention) possess applications below.

(1) Preventive and/or therapeutic agent for diseases associated with dysfunction of the G protein-coupled receptor protein of the present invention

a) The receptor protein of the present invention or b) the DNA encoding the receptor protein can be used for pharmaceuticals such as a preventive and/or therapeutic agent for diseases associated with dysfunction of the receptor protein of the present invention, a cell growth regulator, a cell differentiation regulator, an apoptosis regulator, etc.

For example, when the physiological activity of the ligand cannot be expected in a patient (with deficiency of the receptor protein) due to a decrease in the receptor protein of the present invention in the body, the activity of the ligand can be exhibited sufficiently: a) by administering the receptor protein of the present invention to the patient thereby to supplement the amount of the receptor protein; or b) by increasing the amount of the receptor protein in the patient: (a) through administration of the DNA encoding the receptor protein of the present invention to express the same in the patient; or (b) through insertion and expression of the DNA encoding the receptor protein of the present invention in the objective cells to transplant the cells to the patient, etc. That is, the DNA encoding the receptor protein of the present invention is useful as a safe and low toxic preventive and/or therapeutic agent for diseases associated with dysfunction of the receptor protein of the present invention, a cell growth regulator, a cell differentiation regulator, an apoptosis regulator, etc.

Specifically, the receptor protein of the present invention or the DNA of the present invention is useful for the prevention and/or treatment of diseases, for example, central dysfunctions (e.g., Alzheimer's disease, senile dementia, suppression of eating, etc.), inflammatory diseases (e.g., allergic disease, asthma, rheumatism, etc.), cardiovascular diseases (e.g., hypertension, cardiac hypertrophy, angina pectoris, arteriosclerosis, etc.), cancer (e.g., non-small cell lung carcinoma, ovarian cancer, prostate cancer, stomach cancer, bladder cancer, breast cancer, uterocervical cancer, colon cancer, rectum cancer, etc.), mellitus diabetes, immune system diseases (e.g., autoimmune disease, AIDS, atopic dermatitis, allergic disease, immunodeficiency, asthma, rheumatic arthritis, psoriasis, arteriosclerosis, mellitus diabetes, Alzheimer's disease, etc.), liver/gall bladder diseases (e.g., hepatic cirrhosis, hepatitis, hepatic insufficiency, cholestasis, calculus, etc.), alimentary disorders (e.g., ulcer, enteritis, apepsia, irritable colon syndrome, ulcerative colitis, diarrhea, ileus, etc.), anxiety, pains, obesity, etc.

Where the receptor protein of the present invention is used as the preventive/therapeutic agent or regulator described above, the receptor protein can be prepared into a pharmaceutical composition in a conventional manner.

On the other hand, where the DNA of the present invention is used as the preventive/therapeutic agent or regulator described above, the DNA itself is administered; alternatively, the DNA is inserted into an appropriate vector such as retrovirus vector, adenovirus vector, adenovirus-associated virus vector, etc., which is then administered in a conventional manner. The DNA of the present invention may also be administered as intact DNA, or with adjuvants to assist its uptake by gene gun or through a catheter such as a catheter with a hydrogel.

For example, a) the receptor protein of the present invention or b) the DNA of the present invention can be used orally, for example, in the form of tablets which may be sugar coated if necessary, capsules, elixirs, microcapsules, etc., or parenterally in the form of injectable preparations such as a sterile solution, a suspension, etc. in water or with other pharmaceutically acceptable liquid. These preparations can be manufactured by mixing, e.g., a) the receptor protein of the present invention or b) the DNA e of the present invention, with a carrier, flavoring agent, excipient, vehicle, antiseptic agent, stabilizer, binder, etc., which is physiologically acceptable and publicly known, in a unit dosage form required in a generally accepted manner that is applied to making pharmaceutical preparations. The effective component in these preparations is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin, alginic acid, etc., a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil, coconut oil, etc., or the like. Examples of an aqueous medium for injection include physiological saline and an isotonic solution containing glucose and other auxiliary agents (e.g., D-sorbitol, D-mannitol, sodium chloride, etc.) or the like and may be used in combination with an appropriate dissolution aid such as an alcohol (e.g., ethanol), a polyalcohol (e.g., propylene glycol and polyethylene glycol), a nonionic surfactant (e.g., polysorbate 80™ and HCO-50), etc. Examples of the oily medium include sesame oil, soybean oil, etc., which may also be used in combination with a dissolution aid such as benzyl benzoate, benzyl alcohol, etc.

The preventive/therapeutic agent or regulator described above may further be formulated with a buffer (e.g., phosphate buffer or sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

Since the thus obtained pharmaceutical preparation is safe and low toxic, the preparation can be administered to human or mammals (e.g., rats, mice, rabbits, sheep, swine, bovine, cats, dogs, monkeys, etc.).

The dose of the receptor protein of the present invention varies depending on subject to be administered, target organs, conditions, routes for administration, etc.; in oral administration, e.g., to the patient (as 60 kg body weight) with hypertension, the dose is normally about 0.1 mg to about 100 mg, preferably about 1.0 to about 50 mg, and more preferably about 1.0 to about 20 mg per day. In parenteral administration, the single dose varies depending on subject to be administered, target organ, conditions, routes for administration, etc. but it is advantageous, e.g., for the patient (as 60 kg body weight) with hypertension, to administer the receptor protein intravenously in a daily dose of about 0.01 to about 30 mg, preferably about 0.1 to about 20 mg, and more preferably about 0.1 to about 10 mg. For other animal species, the corresponding dose as converted per 60 kg body weight can be administered.

The dose of the DNA of the present invention varies depending on subject to be administered, target organs, conditions, routes for administration, etc.; in oral administration, e.g., to the patient (as 60 kg body weight) with hypertension, the dose is normally about 0.1 mg to about 100 mg, preferably about 1.0 to about 50 mg, and more preferably about 1.0 to about 20 mg per day. In parenteral administration, the single dose varies depending on subject to be administered, target organ, conditions, routes for administration, etc. but it is advantageous, e.g., for the patient (as 60 kg body weight) with hypertension, to administer the DNA intravenously in a daily dose of about 0.01 to about 30 mg, preferably about 0.1 to about 20 mg, and more preferably about 0.1 to about 10 mg. For other animal species, the corresponding dose as converted per 60 kg body weight can be administered.

(2) Gene diagnostic agent

By using the DNA of the present invention as a probe, an abnormality (gene abnormality) of the DNA or mRNA encoding the receptor protein of the present invention or its partial peptides in human or other mammals (e.g., rats, mice, rabbits, sheep, swine, bovine, cats, dogs, monkeys, etc.) can be detected. Therefore, the DNA or its partial peptides are useful as gene diagnostics for damages against the DNA or mRNA, its mutation, or its decreased expression, or increased expression or overexpression of the DNA or mRNA, or the like.

The gene diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766 -2770 ( 1989)), etc..

For example, when a decreased level of the receptor protein of the present invention is detected by the Northern hybridization, it can be diagnosed that diseases associated with dysfunction of the receptor protein of the present invention are involved or it is highly likely to suffer from these diseases in the future.

Also, when an overexpression of the receptor protein of the present invention is detected by the Northern hybridization, it can be diagnosed that diseases caused by overexpression of the receptor protein of the present invention are involved or it is highly likely to suffer from these diseases in the future.

The diseases associated with dysfunction of the receptor protein of the present invention or the diseases caused by overexpression of the receptor protein of the present invention include, for example, central dysfunctions (e.g., Alzheimer's disease, senile dementia, suppression of eating, etc.), inflammatory diseases (e.g., allergic disease, asthma, rheumatism, etc.), cardiovascular diseases (e.g., hypertension, cardiac hypertrophy, angina pectoris, arteriosclerosis, etc.), cancer (e.g., non-small cell lung carcinoma, ovarian cancer, prostate cancer, stomach cancer, bladder cancer, breast cancer, uterocervical cancer, colon cancer, rectum cancer, etc.), mellitus diabetes, immune system diseases (e.g., autoimmune disease, AIDS, atopic dermatitis, allergic disease, immunodeficiency, asthma, rheumatic arthritis, psoriasis, arteriosclerosis, mellitus diabetes, Alzheimer's disease, etc.), liver/gall bladder diseases (e.g., hepatic cirrhosis, hepatitis, hepatic insufficiency, cholestasis, calculus, etc.), alimentary disorders (e.g., ulcer, enteritis, apepsia, irritable colon syndrome, ulcerative colitis, diarrhea, ileus, etc.), anxiety, pains, obesity, etc.

(3) Pharmaceutical comprising a compound that alters the expression level of the receptor protein of the present invention or its partial peptides

By using as a probe, the DNA of the present invention can be used to screen a compound that alters the expression level of the receptor protein of the present invention or its partial peptides.

That is, the present invention provides the method of screening a compound that alters the expression level of the receptor protein or its partial peptides, which comprises measuring the amount of mRNA in the receptor protein of the present invention or its partial peptides contained in, for example, (i) a) blood, b) particular organs, c) tissues or cells isolated from the organs of non-human mammals, or in (ii) transformants, etc.

Specifically, the amount of mRNA in the receptor protein of the present invention or its partial peptides can be measured as follows.

(i) Normal non-human mammals or disease models of non-human mammals (e.g., mice, rats, rabbits, sheep, swine, bovine, cats, dogs, monkeys, more specifically, rats with dementia, obese mice, rabbits with arteriosclerosis, tumor-bearing mice, etc.) receive administration of a drug (e.g., anti-dementia agents, hypotensive agents, anticancer agents, antiobestic agents, etc.) or physical stress (e.g., soaking stress, electric shock, light and darkness, low temperature, etc.), and the blood, particular organs (e.g., brain, liver, kidney, etc.), or tissues or cells isolated from the organs are obtained after passage of a specified period of time.

The mRNA of the receptor protein of the present invention or its partial peptides contained in the thus obtained cells is extracted from the cells, for example, in a conventional manner and quantified using, e.g., TaqManPCR, etc., or may also be analyzed by the Northern blotting by publicly known methods.

(ii) Transformants that express the receptor protein of the present invention or its partial peptides are prepared according to the methods described above, and the mRNA of the receptor protein of the present invention or its partial peptides can be quantified and analyzed, as described above.

The compound that alters the expression level of the receptor protein of the present invention or its partial peptides can be screened by the following procedures.

(i) Normal non-human mammal or disease model of non-human mammal is administered with a test compound at a specified period of time before (30 minutes to 24 hours before, preferably 30 minutes to 12 hours before, more preferably 1 hour to 6 hours before), at a specified time after (30 minutes to 3 days after, preferably 1 hour to 2 days after, more preferably 1 hour to 24 hours after), or simultaneously with a drug or physical stress. At a specified time (30 minute to 3 days, preferably 1 hour to 2 days, more preferably 1 hour to 24 hours) after administration of the test compound, the amount of mRNA in the receptor protein of the present invention or its partial peptides contained in the cells are quantified and analyzed.

(ii) When transformants are cultured in a conventional manner, a test compound is mixed in the culture medium. After a specified time (after 1 day to 7 days, preferably after 1 day to 3 days, more preferably after 2 to 3 days), the amount of mRNA in the receptor protein of the present invention or its partial peptides contained in the transformants can be quantified and analyzed.

The compound or its salts obtained by the screening method of the present invention are compounds that alter the expression level of the receptor protein of the present invention or its partial peptides. Specifically, (a) compounds that potentiate the G protein-coupled receptor-mediated cell stimulating activities (e.g., activities that promote or suppress arachidonic acid release, acetylcholine release, intracellular $Ca^{2+}$ release, intracellular CAMP production, intracellular cGMP production, inositol phosphate production, changes in cell membrane potential, phosphorylation of intracellular proteins, activation of c-fos, pH reduction, etc.) by increasing the expression level of the receptor protein of the present invention or its partial peptides; and (b) compounds that decrease the cell-stimulating activities by reducing the expression level of the receptor protein of the present invention or its partial peptides.

The compounds include peptides, proteins, non-peptide compounds, synthetic compounds, fermentation products, etc. These compounds may be novel or known compounds.

The ligands to the receptor protein of the present invention are the isoprenoid-related substances as described above. Thus, the compounds, which are obtained by the screening method described above and change the expression level of the receptor protein of the present invention or its partial peptides, can be used as a preventive/therapeutic agent for diseases associated with dysfunction of the receptor protein of the present invention, a cell growth regulator, a cell differentiation regulator, an apoptosis regulator, etc.

Specifically, the compounds that increase the expression level of the receptor protein of the present invention or its partial peptides thereby to potentiate the cell stimulating activities are useful as safe and low toxic preventive/therapeutic agents for diseases associated with dysfunction of the receptor protein of the present invention, cell growth regulators, cell differentiation regulators, apoptosis regulators, etc.

The compounds that decrease the expression level of the receptor protein of the present invention or its partial peptides thereby to lower the cell stimulating activities are useful as safe and low toxic preventive and/or therapeutic agents for diseases caused by overexpression of the receptor protein of the present invention, cell growth regulators, cell differentiation regulators, apoptosis regulators, etc.

The diseases associated with dysfunction of the receptor protein of the present invention or the diseases caused by overexpression of the receptor protein of the present invention include, for example, central dysfunctions (e.g., Alzheimer's disease, senile dementia, suppression of eating, etc.), inflammatory diseases (e.g., allergic disease, asthma, rheumatism, etc.), cardiovascular diseases (e.g., hypertension, cardiac hypertrophy, angina pectoris, arteriosclerosis, etc.), cancer (e.g., non-small cell lung carcinoma, ovarian cancer, prostate cancer, stomach cancer, bladder cancer, breast cancer, uterocervical cancer, colon cancer, rectum cancer, etc.), mellitus diabetes, immune system diseases (e.g., autoimmune disease, AIDS, atopic dermatitis, allergic disease, immunodeficiency, asthma, rheumatic arthritis, psoriasis, arteriosclerosis, mellitus diabetes, Alzheimer's disease, etc.), liver/gall bladder diseases (e.g., hepatic cirrhosis, hepatitis, hepatic insufficiency, cholestasis, calculus, etc.), alimentary disorders (e.g., ulcer, enteritis, apepsia, irritable colon syndrome, ulcerative colitis, diarrhea, ileus, etc.), anxiety, pains, obesity, etc.

Where the compounds or their salt forms obtained by the screening method of the present invention are used as the preventive/therapeutic agents or regulators described above, the compounds can be manufactured into pharmaceutical preparations in a conventional manner.

For example, the compounds can be used orally in the form of tablets which may be sugar coated if necessary, capsules, elixirs, microcapsules, etc., or parenterally in the form of injectable preparations such as a sterile solution, a suspension, etc. in water or with other pharmaceutically acceptable liquid can be formulated. These preparations can be manufactured, e.g., by mixing the compounds with a carrier, flavoring agent, excipient, vehicle, antiseptic agent, stabilizer, binder, etc., which is physiologically acceptable and publicly known, in a unit dosage form required in a generally accepted manner that is applied to making pharmaceutical preparations. The effective component in these preparations is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin, alginic acid, etc., a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil, coconut oil, etc., or the like. Examples of an aqueous medium for injection include physiological saline and an isotonic solution containing glucose and other auxiliary agents (e.g., D-sorbitol, D-mannitol, sodium chloride, etc.) or the like and may be used in combination with an appropriate dissolution aid such as an alcohol (e.g., ethanol), a polyalcohol (e.g., propylene glycol and polyethylene glycol), a nonionic surfactant (e.g., polysorbate 80™ and HCO-50), etc. Examples of the oily medium include sesame oil, soybean oil, etc., which may also be used in combination with a dissolution aid such as benzyl benzoate, benzyl alcohol, etc.

The preventive/therapeutic agent or regulator described above may further be formulated with a buffer (e.g., phosphate buffer or sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

Since the thus obtained pharmaceutical preparation is safe and low toxic, the preparation can be administered to human or mammals (e.g., rats, mice, rabbits, sheep, swine, bovine, cats, dogs, monkeys, etc.).

The dose of the compounds or their salts varies depending on subject to be administered, target organs, conditions, routes for administration, etc.; in oral administration, e.g., to the patient (as 60 kg body weight) with hypertension, the dose is normally about 0.1 mg to about 100 mg, preferably about 1.0 to about 50 mg, and more preferably about 1.0 to about 20 mg per day. In parenteral administration, the single dose varies depending on subject to be administered, target organ, conditions, routes for administration, etc. but it is advantageous, e.g., for the patient (as 60 kg body weight) with hypertension, to administer intravenously in a daily dose of about 0.01 to about 30 mg, preferably about 0. 1 to about 20 mg, and more preferably about 0.1 to about 10 mg. For other animal species, the corresponding dose as converted per 60 kg body weight can be administered.

(4) Method of quantifying the G protein-coupled receptor protein and method for diagnosis

The antibodies of the present invention are capable of specifically recognizing the G protein-coupled receptor protein of the present invention. Thus, the antibodies can be used to quantify the G protein-coupled receptor protein of the present invention in a test fluid, especially for quantification by the sandwich immunoassay, etc.

That is, the present invention provides:

(i) a method of quantifying the receptor protein of the present invention in a test fluid, which comprises competitively reacting the antibody of the present invention with the test fluid and a labeled form of the receptor protein of the present invention, and measuring a ratio of the labeled form of the receptor protein of the present invention bound to the antibody; and,

(ii) a method of quantifying the receptor protein of the present invention in a test fluid, which comprises reacting the test fluid with the antibody of the present invention immobilized on a carrier and a labeled form of another antibody of the present invention simultaneously or sequentially, and measuring the activity of a marker on the immobilized carrier.

In the method of quantification (ii) described above, it is preferred that one antibody recognizes the N-terminal region of the receptor protein of the present invention, and another antibody reacts with the C-terminal region of the receptor protein of the present invention.

Using monoclonal antibodies to the receptor protein of the present invention, the receptor protein of the present invention can be assayed and also detected by tissue staining, or the like. For these purposes, the antibody molecule itself may be used, or F(ab')2, Fab' or Fab fractions of the antibody molecule may be used.

The method of quantifying the receptor protein of the present invention using the antibody of the present invention is not particularly limited. Any quantification method can be used, so long as the amount of antibody, antigen, or antibody-antigen complex corresponding to the amount of antigen (e.g., the amount of the receptor protein of the present invention) in a fluid to be tested can be detected by chemical or physical means and the amount of the antigen can be calculated from a standard curve prepared from standard solutions containing known amounts of the antigen. For example, nephrometry, the competitive method, the immunometric method, and the sandwich method are appropriately used, with the sandwich method described below being most preferable in terms of sensitivity and specificity.

As the marker for the methods using labeled substances, there are employed, for example, radioisotopes, enzymes, fluorescent substances, luminescent substances, etc. For the radioisotope, for example, [$^{125}$I], [$^{131}$I], [$^{3}$H], [$^{14}$C], etc. are used. As the enzyme described above, stable enzymes with high specific activity are preferred; for example, β-galactosidase, β-glucosidase, alkaline phosphatase, peroxidase, malate dehydrogenase and the like are used. Examples of the fluorescent substances used are fluorescamine, fluorescein isothiocyanate, etc. are used. For the luminescent substances, for example, luminol, luminol derivatives, luciferin, lucigenin, etc. are used. The biotin-avidin system may also be used for binding antibody or antigen to the marker.

For immobilization of antigen or antibody, physical adsorption may be used. Chemical binding techniques conventionally used for insolubilization or immobilization of proteins or enzymes may also be used. For carriers, there are used, e.g., insoluble polysaccharides such as agarose, dextran, cellulose, etc.; synthetic resin such as polystyrene, polyacrylamide, silicon, etc., and glass or the like.

In the sandwich method, a test fluid is reacted with the immobilized monoclonal antibody of the present invention (primary reaction), then with a labeled form of another monoclonal antibody of the present invention (secondary reaction), and the activity of a marker on the immobilizing carrier is measured, whereby the amount of the receptor protein of the present invention in the test fluid can be quantified. The order of the primary and secondary reactions may be reversed, and the reactions may be performed simultaneously or with an interval. The markers and methods of immobilization may be based on those described above. In the immunoassay by the sandwich method, the antibodies used for solid phase or antibodies for labeling are not necessarily one species, but a mixture of two or more species of antibodies may be used for purposes of increasing the measurement sensitivity, etc.

In the method of assaying the receptor protein of the present invention by the sandwich method, antibodies that bind to the receptor protein of the present invention at the different sites are preferably used as the monoclonal antibodies of the present invention for the primary and secondary reactions. That is, in the antibodies used for the primary and secondary reactions are, for example, when the antibody used in the secondary reaction recognizes the C-terminal region of the receptor protein, it is preferred to use the antibody recognizing the region other than the C-terminal region for the primary reaction, e.g., the antibody recognizing the N-terminal region.

The monoclonal antibodies of the present invention can be used for the assay systems other than the sandwich method, for example, competitive method, immunometric method, nephrometry, etc.

In the competitive method, antigen in a test fluid and labeled antigen are competitively reacted with antibody, and the unreacted labeled antigen (F) and the labeled antigen bound to the antibody (B) are separated (B/F separation). The amount of a marker in B or F is measured, and the amount of the antigen in the test fluid is quantified. This reaction method includes a liquid phase technique using a soluble antibody as an antibody, polyethylene glycol for B/F separation and a secondary antibody to the soluble antibody, and an immobilization technique either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of a marker in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying these immunological methods to the method for quantification of the present invention, any particular conditions, procedures, etc. are not required. Systems for measuring the receptor protein of the present invention are constructed by adding the usual technical consideration in the art to the conventional conditions and procedures. For the details of these general technical means, reference can be made to the following reviews and texts.

Reference can be made, for example, to Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing).

As described above, the receptor protein of the present invention can be quantified with high sensitivity, using the antibodies of the present invention.

Further where a decreased level of the receptor protein of the present invention is detected by quantifying the level of the receptor protein of the present invention using the antibodies of the present invention, it can be diagnosed that diseases associated with dysfunction of the receptor protein of the present invention are involved or it is highly likely to suffer from these diseases in the future.

Also, when an increased level of the receptor protein of the present invention is detected, it can be diagnosed that diseases caused by overexpression of the receptor protein of the present invention are involved or it is highly likely to suffer from these diseases in the future.

The diseases associated with dysfunction of the receptor protein of the present invention or the diseases caused by overexpression of the receptor protein of the present invention include, for example, central dysfunctions (e.g., Alzheimer's disease, senile dementia, suppression of eating, etc.), inflammatory diseases (e.g., allergic disease, asthma, rheumatism, etc.), cardiovascular diseases (e.g., hypertension, cardiac hypertrophy, angina pectoris, arteriosclerosis, etc.), cancer (e.g., non-small cell lung carcinoma, ovarian cancer, prostate cancer, stomach cancer, bladder cancer, breast cancer, uterocervical cancer, colon cancer, rectum cancer, etc.), mellitus diabetes, immune system diseases (e.g., autoimmune disease, AIDS, atopic dermatitis, allergic disease, immunodeficiency, asthma, rheumatic arthritis, psoriasis, arteriosclerosis, mellitus diabetes, Alzheimer's disease, etc.), liver/gall bladder diseases (e.g., hepatic cirrhosis, hepatitis, hepatic insufficiency, cholestasis, calculus, etc.), alimentary disorders (e.g., ulcer, enteritis, apepsia, irritable colon syndrome, ulcerative colitis, diarrhea, ileus, etc.), anxiety, pains, obesity, etc.

(5) Method of determining other ligands than the isoprenoid-related substance to the receptor protein of the present invention

By binding of the isoprenoid-related substance to the receptor protein of the present invention, an increased intracellular $Ca^{2+}$ level and promotion of cAMP production are observed. Thus, the receptor protein of the present invention is useful as a reagent for searching or determining ligands other than the isoprenoid-related substance, using this intracellular signal as an indicator.

That is, the present invention provides a method of determining a ligand to the receptor protein of the present invention, which comprises assaying the intracellular $Ca^{2+}$ level increasing activity or the intracellular cAMP production promoting activity mediated by the receptor protein of the present invention, when a test compound is brought in contact with a cell containing the receptor protein of the present invention.

Examples of the test compounds include known ligands (e.g., angiotensin, bombesin, canavinoid, cholecystokinin, glutamine, serotonin, melatonin, neuropeptide Y, opioids, purines, vasopressin, oxytocin, PACAP (e.g., PACAP27, PACAP38), secretin, glucagon, calcitonin, adrenomedulin, somatostatin, GHRH, CRF, ACTH, GRP, PTH, VIP (vasoactive intestinal and related polypeptide), somatostatin, dopamine, motilin, amylin, bradykinin, CGRP (calcitonin gene-related peptide), leukotriens, pancreastatin, prostaglandins, thromboxane, adenosine, adrenaline, a chemokine superfamily (e.g., CXC chemokine subfamily such as IL-8, GROα, CROβ, GROγ, NAP-2, ENA-78, GCP-2, PF4, IP10, Mig, PBSF/SDF-1, etc.; CC chemokine subfamily such as MCAF/MCP-1, MCP-2, MCP-3, MCP-4, eotaxin, RANTES, MIP1-α, MIP-1β, HCC-1, MIP-3α/LARC, MIP-3β/ELC, I-309, TARC, MIPF-1, MIPF-2/eotaxin-2, MDC, DC-CK1/PARC, SLC, etc.; C chemokine subfamily such as lymphotactin; CX3C chemokine subfamily such as fractalkine, etc.), endothelin, enterogastrin, histamine, neurotensin, TRH, pancreatic polypeptide, galanin, lysophosphatidic acid (LPA) or sphingosine 1-phosphate, etc.); in addition, tissue extracts, cell culture supernatants, etc. from human or mammals (e.g., mice, rats, swine, bovine, sheep, monkeys, etc.) are also employed. For example, the tissue extracts, cell culture supernatants, etc. are added to the receptor protein of the present invention, and while assaying cell stimulating activities, etc., the mixture is fractionated; finally a single ligand can be obtained.

Specifically, the method of the present invention for determining ligands is a method of determining a compound or its salt having the intracellular $Ca^{2+}$ level increasing activity or the intracellular cAMP production promoting activity mediated by the receptor protein of the present invention, which comprises constructing the recombinant receptor protein expression system and using the receptor-binding assay system using the expression system.

More specifically, the present invention provides the following method for determination:

(1) a method of determining a ligand to the receptor protein of the present invention which comprises assaying the intracellular $Ca^{2+}$ level increasing activity or the intracellular cAMP production promoting activity, when a test compound is brought in contact with a cell containing the receptor protein of the present invention; and,
(2) a method of determining a ligand to the receptor protein of the present invention which comprises assaying the intracellular $Ca^{2+}$ level increasing activity or the intracellular cAMP production promoting activity mediated by the receptor protein of the present invention, when a test compound is brought in contact with the receptor protein expressed on a cell membrane by culturing a transformant containing a DNA encoding the receptor protein of the present invention.

In particular, it is preferred to perform the test described above after confirmation that the test compound is bound to the receptor protein of the present invention.
In the method of the present invention for determining ligands where cells containing the receptor protein of the present invention are used, the cells may be fixed with glutaraldehyde, formalin, etc. The fixation may be made by publicly known methods.
The cell membrane fraction containing the receptor protein of the present invention refers to a fraction abundant in cell membrane obtained by cell disruption and subsequent fractionation by a publicly known method. Useful cell disruption methods include cell squashing using a Potter-Elvehjem homogenizer, disruption using a Waring blender or Polytron (manufactured by Kinematica, Inc.), disruption by ultrasonication, disruption by cell spraying through thin nozzles under an increased pressure using a French press, or the like. Fractionation of cell membranes is effected mainly by fractionation using a centrifugal force, such as centrifugation for fractionation and density gradient centrifugation. For example, the cell lysate is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the receptor protein expressed and membrane components such as cell-derived phospholipids, membrane proteins, etc.
The level of the receptor protein of the present invention in the cells containing the receptor protein and in the membrane fraction is preferably $10^3$ to $10^8$ molecules per cell, more preferably $10^5$ to $10^7$ molecules per cell. As the expression level increases, the ligand binding activity per unit of the membrane fraction (specific activity) increases so that not only the highly sensitive screening system can be constructed but also large quantities of samples can be assayed with the same lot.
To carry out the method of the present invention for determining the ligand, the intracellular $Ca^{2+}$ level increasing activity or the intracellular cAMP production promoting activity mediated by the receptor protein of the present invention may be assayed by a publicly known method, or using an assay kit commercially available. Specifically, cells containing the receptor protein of the present invention are first cultured on a multi-well plate, etc. Prior to the ligand determination, the medium is replaced by fresh medium or with an appropriate non-cytotoxic buffer, followed by incubation for a given period of time in the presence of a test compound, etc. Subsequently, the cells are extracted or the supernatant is recovered and the resulting product is quantified by respective procedures. Where it is difficult to detect the production of the indicator substance (e.g., $Ca^{2+}$,cAMP, etc.) for the cell-stimulating activities due to a degrading enzyme contained in the cells, an inhibitor against such a degrading enzyme may be added prior to the assay. For detecting the activity such as the cAMP production promotion, the baseline production in cells is previously increased by forskolin or the like and the activity can be detected in terms of the production promoting effect on the cells.
The kit of the present invention for determining the ligand comprises the cells containing the receptor protein of the present invention, or the cell membrane fraction.
The ligand to the receptor protein of the present invention thus determined binds to the receptor protein of the present invention to regulate its physiological functions, and can thus be used as a preventive/therapeutic agent for diseases associated with the functions of the receptor protein of the present invention.

(6) Method of screening a compound (agonist, antagonist, etc.) that alters the binding properties of the G protein-coupled receptor protein of the present invention to a ligand, and pharmaceutical comprising a compound that alters the binding properties of the G protein-coupled receptor protein of the present invention to a ligand

Either by using the receptor protein of the present invention or by constructing the expression system of a recombinant receptor protein and using the receptor-binding assay system using the expression system, a compound (e.g., a peptide, protein, non-peptide compound, synthetic compound, fermentation product, etc.) that alters the binding properties of the isoprenoid-related substance, which is a ligand, to the receptor protein of the present invention, or its salt can be efficiently screened.

Such compounds include (a) compounds having the cell stimulating activities (e.g., activities that promote or suppress arachidonic acid release, acetylcholine release, intracellular $Ca^{2+}$ release (intracellular $Ca^{2+}$ level increase), intracellular cAMP production, intracellular cGMP production, inositol phosphate production, changes in cell membrane potential, phosphorylation of intracellular proteins, activation of c-fos, pH reduction, etc.) mediated by the G protein-coupled receptor protein (so-called agonists to the receptor protein of the present invention); (b) compounds having no such cell-stimulating activities (so-called antagonists to the receptor protein of the present invention); (c) compounds that potentiate the binding force of the ligand to the G protein-coupled receptor protein of the present invention; or (d) compounds that reduce the binding force of the ligand to the G protein-coupled receptor protein of the present invention; etc. (preferably, the compounds (a) are screened by the method of determining the ligand described above).

Thus, the present invention provides a method of screening a compound or its salt that alters the binding properties of the ligand to the receptor protein of the present invention, it partial peptide, or a salt thereof, which comprises comparing (i) the case where the receptor protein of the present invention, it partial peptide, or a salt thereof is brought in contact with the ligand and (ii) the case where the receptor protein of the present invention, it partial peptide, or a salt thereof is brought in contact with the ligand and a test compound.

The screening method of the present invention is characterized by measuring, e.g., an amount of the ligand bound to the receptor protein, a cell stimulating activity, etc. in (i) and (ii) and comparing the same between (i) and (ii).

More specifically, the present invention provides:

a) a method of screening a compound or its salt that alters the binding properties of a ligand to the receptor protein of the present invention, which comprises measuring binding amounts of a labeled form of the ligand to the receptor protein in the case where a labeled form of the ligand is brought in contact with the receptor protein of the present invention and in the case where a labeled form of the ligand and a test compound are brought in contact with the receptor protein of the present invention, and comparing the binding amounts;

b) a method of screening a compound or its salt that alters the binding properties of a ligand to the receptor protein of the present invention, which comprises measuring binding amounts of a labeled form of the ligand to a cell containing the receptor protein of the present invention or a membrane fraction of the cell, in the case where a labeled form of the ligand is brought in contact with the cell or the membrane fraction of the cell and in the case where a labeled form of the ligand and a test compound are brought in contact with the cell or the membrane fraction of the cell, and comparing the binding amounts;

c) a method of screening a compound or its salt that alters the binding properties of a ligand to the receptor protein of the present invention, which comprises measuring binding amounts of a labeled form of the ligand to the receptor protein, in the case where a labeled form of the ligand is brought in contact with the receptor protein expressed on a cell membrane by culturing a transformant containing the DNA of the present invention and in the case where a labeled form of the ligand and a test compound are brought in contact with the receptor protein expressed on a cell membrane by culturing a transformant containing the DNA of the present invention, and comparing the binding amounts;

d) a method of screening a compound or its salt that alters the binding properties of a ligand to the receptor protein of the present invention, which comprises assaying receptor-mediated cell stimulating activities (e.g., activities that promote or suppress arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, pH reduction, etc.), in the case where a compound that activates the receptor protein of the present invention (e.g., a ligand to the receptor protein of the present invention, etc.) is brought in contact with a cell containing the receptor protein of the present invention and in the case where the compound that activates the receptor protein of the present invention and a test compound are brought in contact with a cell containing the receptor protein of the present invention, and comparing the activities; and,

e) a method of screening a compound or its salt that alters the binding properties of a ligand to the receptor protein of the present invention, which comprises assaying receptor-mediated cell stimulating activities (e.g., activities that promote or suppress arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, pH reduction, etc.), in the case where a compound that activates the receptor protein of the present invention (e.g., a ligand to the receptor protein of the present invention, etc.) is brought in contact with the receptor protein of the present invention expressed on a cell membrane by culturing a transformant containing the DNA of the present invention and in the case where the compound that activates the receptor protein of the present invention and a test compound are brought in contact with the receptor protein of the present invention expressed on a cell membrane by culturing a transformant containing the DNA of the present invention, and comparing the activities.

As the ligand, there are used the isoprenoid-related substances described above, such as farnesyl pyrophosphate (FPP), geranyl pyrophosphate (GPP), geranylgeranyl pyrophosphate (GGPP), etc.

In addition, the compound or its salt that alters the binding properties of the isoprenoid-related substance to the receptor protein of the present invention may also be used. The compound or its salt that alters the binding properties of the isoprenoid-related substance to the receptor protein of the present invention can be obtained by the screening method of the present invention, which will be later described, e.g., using the isoprenoid-related substance as a ligand.

Before the receptor protein of the present invention was obtained, it was required for screening G protein-coupled receptor agonists or antagonists to obtain candidate compounds first, using cells or tissues containing the G protein-coupled receptor protein or membrane fractions of the cells, from rats or other animals (primary screening), and then examine the candidate compounds whether the compounds actually inhibit the binding of human G protein-coupled receptor protein to the ligand (secondary screening). When cells, tissues, or the cell membrane fractions were directly used, it was practically difficult to screen agonists or antagonists to the objective receptor protein, since other receptor proteins were present together.

However, using, for example, the human-derived receptor protein of the present invention, the primary screening becomes unnecessary, and the compound that inhibits the binding of the ligand to the G protein-coupled receptor protein can be efficiently screened. Furthermore, it is easy to assess whether the obtained compound is an agonist or antagonist.

Hereinafter, the screening methods of the present invention are described more specifically.

First, for the receptor protein of the present invention used in the screening methods of the present invention, any substance may be used so long as it contains the receptor protein of the present invention described above. The cell membrane fraction from mammalian organs containing the receptor protein of the present invention is preferred. However, it is very difficult to obtain human organs. It is thus preferable to use human-derived receptor proteins or the like, produced by large-scale expression using recombinants.

To manufacture the receptor protein of the present invention, the methods described above are used, and it is preferred to express the DNA of the present invention in mammalian and insect cells. For the DNA fragment encoding the objective protein region, the complementary DNA, but not necessarily limited thereto, is employed. For example, the gene fragments and synthetic DNA may also be used. To introduce a DNA fragment encoding the receptor protein of the present invention into host animal cells and efficiently express the DNA there, it is preferred to insert the DNA fragment downstream of a polyhedorin promoter of nuclear polyhedrosis virus (NPV) belonging to baculovirus hosted by insects, SV40-derived promoter, retrovirus promoter, metallothionein promoter, human heat shock promoter, cytomegalovirus promoter, SR$\alpha$ promoter, etc.. The quantity and quality of the expressed receptor are examined by publicly known methods, for example, the method described in the literature [Nambi, P. et al., The Journal of Biological Chemistry (J. Biol. Chem.), 267, 19555-19559, 1992].

Therefore, in the screening methods of the present invention, those containing the receptor protein of the present invention may be a receptor protein purified by publicly known methods, cells containing the receptor protein, or the cell membrane fraction containing the receptor protein.

In the screening methods of the present invention where cells containing the receptor protein of the present invention are used, the cells may be fixed with glutaraldehyde, formalin, etc. The fixation may be made by publicly known methods.

The cells containing the receptor protein of the present invention are host cells that express the receptor protein. For the host cells, Escherichia coli, Bacillus subtilis, yeast, insect cells, animal cells and the like are preferred.

The cell membrane fraction refers to a fraction abundant in cell membrane obtained by cell disruption and subsequent fractionation by a publicly known method. Useful cell disruption methods include cell squashing using a Potter-Elvehjem homogenizer, disruption using a Waring blender or Polytron (manufactured by Kinematica, Inc.), disruption by ultrasonication, disruption by cell spraying through thin nozzles under an increased pressure using a French press, or the like. Fractionation of cell membranes is effected mainly by fractionation using a centrifugal force, such as centrifugation for fractionation and density gradient centrifugation. For example, the cell lysate is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the receptor protein expressed and membrane components such as cell-derived phospholipids, membrane proteins, etc.

The level of the receptor protein of the present invention in the cells containing the receptor protein and in the membrane fraction is preferably $10^3$ to $10^8$ molecules per cell, more preferably $10^5$ to $10^7$ molecules per cell. As the expression level increases, the ligand binding activity per unit of the membrane fraction (specific activity) increases so that not only the highly sensitive screening system can be constructed but also large quantities of samples can be assayed with the same lot.

To screen the compound that alters the binding properties of the ligand to the receptor protein of the present invention described in a) to c), for example, an appropriate receptor protein fraction and a labeled ligand are required.

The receptor protein fraction is preferably a fraction of naturally occurring receptor protein or a recombinant receptor fraction having an activity equivalent to that of the natural protein. Herein, the equivalent activity is intended to mean a ligand binding activity, a signal transduction activity or the like, which is equivalent to that possessed by naturally occurring receptor proteins.

As the labeled ligand, there is employed a labeled ligand, a labeled ligand analog compound, etc. For example, ligands labeled with [$^3$H], [$^{125}$I], [$^{14}$C], [$^{35}$S], etc, are used.

Specifically, to screen the compounds that alter the binding properties of ligands to the receptor protein of the present invention, first, the receptor protein standard is prepared by suspending cells or cell membrane fraction containing the receptor protein of the present invention in a buffer appropriate for the screening. For the buffer, any buffer that does not interfere with the binding of ligands to the receptor protein is usable and examples of such a buffer are phosphate buffer, Tris-hydrochloride buffer, etc., having pH of 4 to 10 (preferably pH of 6 to 8). To minimize non-specific binding, a surfactant such as CHAPS, Tween-80™ (Kao-Atlas Co.), digitonin, deoxycholate, etc. may be added to the buffer. To inhibit degradation of the receptor or ligands by proteases, protease inhibitors such as PMSF, leupeptin, E-64 (manufactured by Peptide Research Laboratory, Co.), and pepstatin may be added. To 0.01 ml to 10 ml of the receptor solution, a given amount (5,000 cpm to 500,000 cpm) of labeled ligand is added, and $10^{-4}$ M - $10^{-10}$ M of a test compound is simultaneously added to be co-present. To examine non-specific binding (NSB), a reaction tube containing an unlabeled test compound in a large excess is also prepared. The reaction is carried out at approximately 0 to 50° C, preferably about 4 to 37° C for about 20 minutes to about 24 hours, preferably about 30 minutes to about 3 hours. After the reaction, the reaction mixture is filtrated through glass fiber filter paper, etc. and washed with an appropriate volume of the same buffer. The residual radioactivity on the glass fiber filter paper is then measured by means of a liquid scintillation counter or γ-counter. Regarding the count obtained by subtracting the amount of non-specific binding (NSB) from the count obtained in the absence of any competitive substance ($B_0$) as 100%, when the amount of specific binding (B-NSB) is, for example, 50% or less, the test compound can be selected as a candidate substance having a potential of competitive inhibition.

To perform the methods d) and e) supra of screening the compounds that alter the binding properties of ligands to the receptor protein of the present invention, for example, the receptor protein-mediated cell-stimulating activity (e. g., activity that promotes or inhibits arachidonic acid release, acetylcholine release, intracellular Ca release, intracellular CAMP production, intracellular cGMP production, inositol phosphate production, changes in cell membrane potential, phosphorylation of intracellular proteins, activation of c-fos, pH reduction, etc.) can be assayed using publicly known methods or commercially available kits.

Specifically, the cells containing the receptor protein of the present invention are first cultured on a multi-well plate, etc. Prior to screening, the medium is replaced by fresh medium or with an appropriate non-cytotoxic buffer, followed by incubation for a given period of time in the presence of a test compound, etc. Subsequently, the cells are extracted or the supernatant is recovered and the resulting product is quantified by appropriate procedures. Where it is difficult to detect the production of the indicator substance (e.g., arachidonic acid, etc.) for the cell-stimulating activity due to a degrading enzyme contained in the cells, an inhibitor against such a degrading enzyme may be added prior to the assay. For detecting activities such as the cAMP production suppression activity, the baseline production in the cells is increased by forskolin, etc. and the suppressing effect on the increased baseline production may then be detected.

For the screening by assaying the cell-stimulating activity, cells that have expressed an appropriate receptor protein are required. For the cells that have expressed the receptor protein of the present invention, the cell line having a native form of the receptor protein of the present invention, the cell line expressing the recombinant receptor protein described above, etc. are desired.

For the test compound, for example, peptides, proteins, non-peptide compounds, synthetic compounds, fermentation products, cell extracts, plant extracts, animal tissue extracts, etc. are used. These compounds may be novel or known compounds.

The test compound which is preferably used is a compound designed to bind to the ligand-binding pocket, based on the atomic coordinate and the position of the ligand-binding pocket in the active site of the receptor protein of the present invention. The atomic coordinate and the position of the ligand-binding pocket in the active site of the receptor protein of the present invention can be determined by publicly known methods or modifications thereof.

The kits for screening the compound or its salt that alters the binding properties of ligands to the receptor protein of the present invention comprise the receptor protein of the present invention, cells containing the receptor protein of the present invention, or membrane fractions of the cells containing the receptor protein of the present invention; etc.

Examples of the screening kits of the present invention are as follow,

1. Reagent for screening

a) Assay buffer and wash buffer

Hanks' Balanced Salt Solution (manufactured by Gibco Co.) supplemented with 0.05% bovine serum albumin (Sigma Co.).

The solution is sterilized by filtration through a 0.45 μm filter and stored at 4°C. Alternatively, the solution may be prepared at use.

b) G protein-coupled receptor standard

CHO cells on which the receptor of the present invention has been expressed are subcultured in a 12-well plate at the rate of 5 x $10^5$ cells/well and then cultured at 37°C under 5% $CO_2$ and 95% air for 2 days.

c) Labeled ligand

Ligand labeled with commercially available [$^3$H], [$^{125}$I], [$^{14}$C], [$^{35}$S], etc.

The ligand is stored in its aqueous solution at 4°C or -20°C, which is diluted to 1 μM with an assay buffer at use.

d) Standard ligand solution

The ligand is dissolved in PBS supplemented with 0.1 % bovine serum albumin (manufactured by Sigma, Inc.) in a concentration of 1 mM, and the solution is stored at -20°C.

2. Assay method

a) Cells are cultured in a 12-well tissue culture plate to express the receptor of the present invention. After washing the cells twice with 1 ml of the assay buffer, 490 μl of the assay buffer is added to each well.
b) After 5 μl of a test compound solution of $10^{-3}$ to $10^{-10}$ M is added, 5 μl of the labeled ligand is added to the system followed by reacting at room temperature for an hour. To determine the amount of the non-specific binding, the ligand of $10^{-3}$ M is added in an amount of 5 μl, instead of the test compound.
c) The reaction solution is removed and washed 3 times with 1 ml each of the wash buffer. The labeled ligand bound to the cells is dissolved in 0.2N NaOH-1% SDS and mixed with 4 ml of a liquid scintillator A (manufactured by Wako Pure Chemical Industries, Ltd.).
d) Radioactivity is measured using a liquid scintillation counter (manufactured by Beckmann) and PMB (percent of the maximum binding) is calculated in accordance with the following equation 1:

$$PMB = [(B-NSB)/(B_0 - NSB)] \times 100$$

wherein:

PMB: percent of the maximum binding
B: value when a sample is added
NSB: non-specific binding
$B_0$: maximum binding

The compounds or their salts obtained by the screening methods or the screening kits of the present invention are compounds having the activity that alters the binding properties of the ligand to the receptor protein of the present invention. Specifically, these compounds include (a) compounds having the cell stimulating activities (e.g., activities that promote or suppress arachidonic acid release, acetylcholine release, intracellular $Ca^{2+}$ release (intracellular $Ca^{2+}$ level increase), intracellular cAMP production, intracellular cGMP production, inositol phosphate production, changes in cell membrane potential, phosphorylation of intracellular proteins, activation of c-fos, pH reduction, etc.) mediated by the G protein-coupled receptor protein (so-called agonists to the receptor protein of the present invention); (b) compounds having no such cell-stimulating activities (so-called antagonists to the receptor protein of the present invention); (c) compounds that potentiate the binding force of the ligand to the G protein-coupled receptor protein of the present invention; or (d) compounds that reduce the binding force of the ligand to the G protein-coupled receptor protein of the present invention; etc.

Examples of such compounds include peptides, proteins, non-peptide compounds, synthetic compounds, fermentation products, etc. These compounds may be either novel or publicly known compounds.

The agonists to the receptor protein of the present invention have similar physiological activities to those of the isoprenoid-related substance, which is a ligand to the receptor protein of the present invention, and are useful as safe and low toxic pharmaceuticals depending on the physiological activities the isoprenoid-related substance has.

The antagonists to the receptor protein of the present invention can suppress the physiological activities of the isoprenoid-related substance, which is a ligand to the receptor protein of the present invention, and are useful as safe and low toxic pharmaceuticals to suppress the physiological activities of the isoprenoid-related substance.

The compounds that potentiate the binding force of the ligand to the G protein-coupled receptor protein of the present invention can potentiate the physiological activities of the ligand to the receptor protein of the present invention, and are useful as safe and low toxic pharmaceuticals depending on the physiological activities the isoprenoid-related substance has.

Since the compounds that reduce the binding force of the ligand to the G protein-coupled receptor protein of the present invention can reduce the physiological activities the ligand to the receptor protein of the present invention has, the compounds are useful as safe and low toxic pharmaceuticals to suppress the physiological activities of the isoprenoid-related substance.

Specifically, the compounds or their salts obtained by the screening methods or screening kits of the present invention are useful as preventive/therapeutic agents for diseases, for example, central dysfunctions (e.g., Alzheimer's disease, senile dementia, suppression of eating, etc.), inflammatory diseases (e.g., allergic disease, asthma, rheumatism, etc.), cardiovascular diseases (e.g., hypertension, cardiac hypertrophy, angina pectoris, arteriosclerosis, etc.), cancer (e.g., non-small cell lung carcinoma, ovarian cancer, prostate cancer, stomach cancer, bladder cancer, breast cancer, uterocervical cancer, colon cancer, rectum cancer, etc.), mellitus diabetes, immune system diseases (e.g., autoimmune disease, AIDS, atopic dermatitis, allergic disease, immunodeficiency, asthma, rheumatic arthritis, psoriasis, arteriosclerosis, mellitus diabetes, Alzheimer's disease, etc.), liver/gall bladder diseases (e.g., hepatic cirrhosis, hepatitis, hepatic insufficiency, cholestasis, calculus, etc.), alimentary disorders (e.g., ulcer, enteritis, apepsia, irritable colon syndrome, ulcerative colitis, diarrhea, ileus, etc.), anxiety, pains, obesity, etc.; cell growth regulators, cell differentiation regulators, apoptosis regulators, etc.

Where the compounds or their salt forms obtained by the screening methods or screening kits of the present invention are used as the preventive/therapeutic agents or regulators described above, the compounds can be manufactured into pharmaceutical preparations in a conventional manner.

For example, the compounds can be used orally in the form of tablets which may be sugar coated if necessary, capsules, elixirs, microcapsules, etc., or parenterally in the form of injectable preparations such as a sterile solution, a suspension, etc. in water or with other pharmaceutically acceptable liquid can be formulated. These preparations can be manufactured, e.g., by mixing the compounds with a carrier, flavoring agent, excipient, vehicle, antiseptic agent, stabilizer, binder, etc., which is physiologically acceptable and publicly known, in a unit dosage form required in a generally accepted manner that is applied to making pharmaceutical preparations. The effective component in these preparations is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin, alginic acid, etc., a lubricant such as magnesium stearate, a sweetening agent such as sucrose, lactose or saccharin, and a flavoring agent such as peppermint, akamono oil and cherry, etc. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil, coconut oil, etc., or the like. Examples of an aqueous medium for injection include physiological saline and an isotonic solution containing glucose and other auxiliary agents (e.g., D-sorbitol, D-mannitol, sodium chloride, etc.) or the like and may be used in combination with an appropriate dissolution aid such as an alcohol (e.g., ethanol), a polyalcohol (e.g., propylene glycol and polyethylene glycol), a nonionic surfactant (e.g., polysorbate 80™ and HCO-50), etc. Examples of the oily medium include sesame oil, soybean oil, etc., which may also be used in combination with a dissolution aid such as benzyl benzoate, benzyl alcohol, etc.

The preventive/therapeutic agent or regulator described above may further be formulated with a buffer (e.g., phosphate buffer or sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

Since the thus obtained pharmaceutical preparation is safe and low toxic, the preparation can be administered to human or mammals (e.g., rats, mice, rabbits, sheep, swine, bovine, cats, dogs, monkeys, etc.).

The dose of the compounds or their salts varies depending on subject to be administered, target organs, conditions, routes for administration, etc.; in oral administration, e.g., to the patient (as 60 kg body weight) with hypertension, the dose is normally about 0.1 mg to about 100 mg, preferably about 1.0 to about 50 mg, and more preferably about 1.0 to about 20 mg per day. In parenteral administration, the single dose varies depending on subject to be administered, target organ, conditions, routes for administration, etc. but it is advantageous, e.g., for the patient (as 60 kg body weight) with hypertension, to administer intravenously in a daily dose of about 0.01 to about 30 mg, preferably about 0.1 to about 20 mg, and more preferably about 0.1 to about 10 mg. For other animal species, the corresponding dose as converted per 60 kg body weight can be administered.

(7) Compound that alters the amount of the receptor protein of the present invention or its partial peptides on cell membranes

The antibodies of the present invention are capable of specifically recognizing the receptor protein of the present invention, its partial peptides, or salts thereof and thus can be used for screening a compound that alters the amount of the receptor protein of the present invention or its partial peptides on cell membranes.
That is, the present invention provides, for example, the following methods:

(i) a method of screening a compound that alters the amount of the receptor protein of the present invention or its partial peptides on cell membranes, which comprises disrupting a) blood, b) particular organs, or c) tissues or cells isolated from the organs of non-human mammals, isolating the cell membrane fraction and then quantifying the receptor protein of the present invention or its partial peptides contained in the cell membrane fraction;
(ii) a method of screening a compound that alters the amount of the receptor protein of the present invention or its partial peptides on cell membranes, which comprises disrupting a transformant, etc. expressing the receptor protein of the present invention or its partial peptides, isolating the cell membrane fraction, and then quantifying the receptor protein of the present invention or its partial peptides contained in the cell membrane fraction;
(iii) a method of screening a compound that alters the amount of the receptor protein of the present invention or its partial peptides on cell membranes, which comprises sectioning (a) blood, (b) particular organs, or (c) tissues or cells isolated from the organs of non-human mammals, immunostaining, and then quantifying the staining intensity of the receptor protein at the cell surface layer to confirm the protein on the cell membranes; and,
(iv) a method of screening a compound that alters the amount of the receptor protein of the present invention or its partial peptides on cell membranes, which comprises sectioning a transformant, etc. expressing the receptor protein of the present invention or its partial peptides, immunostaining, and then quantifying the staining intensity of the receptor protein at the cell surface layer to confirm the protein on the cell membranes.

Specifically, the receptor protein of the present invention or its partial peptides contained in cell membrane fractions is quantified as follows.

(i) Normal non-human mammals or disease models of non-human mammals (e.g., mice, rats, rabbits, sheep, swine, bovine, cats, dogs, monkeys, more specifically, rats with dementia, obese mice, rabbits with arteriosclerosis, tumor-bearing mice, etc.) are administered with drugs (e.g., anti-dementia agents, hypotensive agents, anticancer agents, antiobestic agents, etc.) or physical stress (e.g., soaking stress, electric shock, light and darkness, low temperature, etc.), and the blood, particular organs (e.g., brain, liver, kidney, etc.), or tissue or cells isolated from the organs are obtained after a specified period of time. The obtained organs, tissues, cells or the like are suspended in, for example, an appropriate buffer (e.g., Tris hydrochloride buffer, phosphate buffer, Hepes buffer, etc.), and the organs, tissues or cells are disrupted, and the cell membrane fraction is obtained using surfactants (e.g., Triton-X 100™, Tween 20™) and further using techniques such as centrifugal separation, filtration, column fractionation, etc.

The cell membrane fraction refers to a fraction abundant in cell membranes obtained by cell disruption and subsequent fractionation by a publicly known method. Useful cell disruption methods include cell squashing using a Potter-Elvehjem homogenizer, disruption using a Waring blender or Polytron (manufactured by Kinematica Inc.), disruption by ultrasonication, disruption by cell spraying through thin nozzles under an increased pressure using a French press, and the like. Cell membrane fractionation is effected mainly by fractionation using a centrifugal force, such as centrifugation for fractionation and density gradient centrifugation. For example, cell lysates are centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the receptor protein expressed and membrane components such as cell-derived phospholipids, membrane proteins, etc.

The receptor protein of the present invention or its partial peptides contained in the cell membrane fraction can be quantified by, for example, the sandwich immunoassay, western blot analysis, etc. using the antibody of the present invention.

The sandwich immunoassay can be performed as described above, and the western blot can be performed by publicly known methods.

(ii) Transformants expressing the receptor protein of the present invention or its partial peptides are prepared by the method described above, and the receptor protein of the present invention or its partial peptides contained in the cell membrane fraction can be quantified.

The compound that alters the amount of the receptor protein of the present invention or its partial peptides in cell membranes can be screened as follows.

(i) Normal non-human mammal or disease model of non-human mammal is administered with a test compound at a specified period of time before (30 minutes to 24 hours before, preferably 30 minutes to 12 hours before, more preferably 1 hour to 6 hours before), at a specified time after (30 minutes to 3 days after, preferably 1 hour to 2 days after, more preferably 1 hour to 24 hours after), or simultaneously with a drug or physical stress. At a specified time (30 minute to 3 days, preferably 1 hour to 2 days, more preferably 1 hour to 24 hours) after administration of the test compound, the amount of the receptor protein of the present invention or its partial peptides contained in cell membranes can be quantified.

(ii) Transformants are cultured in a conventional manner and a test compound is mixed in the culture medium. After a specified time (after 1 day to 7 days, preferably after 1 day to 3 days, more preferably after 2 to 3 days), the amount of the receptor protein of the present invention or its partial peptides contained in the cell membranes can be quantified.

Specifically, the receptor protein of the present invention or its partial peptides contained in cell membrane fractions is confirmed as follows.

(iii) Normal non-human mammals or disease models of non-human mammals (e.g., mice, rats, rabbits, sheep, swine, bovine, cats, dogs, monkeys, etc., more specifically, rats with dementia, obese mice, rabbits with arteriosclerosis, tumor-bearing mice, etc.) are administered with drugs (e.g., anti-dementia agents, hypotensive agents, anticancer agents, antiobestic agents, etc.) or physical stress (e.g., soaking stress, electric shock, light and darkness, low temperature, etc.) or the like, and blood or particular organ (e.g., brain, liver, kidney, etc.), or the tissues or cells isolated from the organ are obtained after a specified period of time. Tissue sections are prepared from the thus obtained organs, tissues, cells, etc. in a conventional manner followed by immunostaining with the antibody of the present invention. The staining intensity of the receptor protein on the cell surface layer is quantified to confirm the protein on the cell membrane, whereby the amount of the receptor protein of the present invention or its partial peptides on the cell membrane can be confirmed quantitatively or qualitatively.

(iv) The confirmation can also be made by the similar method, using transformants expressing the receptor protein of the present invention or its partial peptides.

The compounds or its salts obtained by the screening methods of the present invention are the compounds that have an effect of changing the amount of the receptor protein or its partial peptides. Specifically, these compounds are: (a) compounds that increase the amount of the receptor protein of the present invention or its partial peptides on cell membranes thereby to potentiate the G protein-coupled receptor-mediated cell-stimulating activities (e.g., the activities that promote or inhibit arachidonic acid release, acetylcholine release, intracellular $Ca^{2+}$ release (intracellular $Ca^{2+}$ level increase), intracellular cAMP production, intracellular cGMP production, inositol phosphate production, changes in cell membrane potential, phosphorylation of intracellular proteins, activation of c-fos, pH reduction, etc.); and (b) compounds that decrease the amount of the receptor protein of the present invention or its partial peptides thereby to lower the cell stimulating-activities.

The compounds may be peptides, proteins, non-peptide compounds, synthetic compounds, fermentation products, and may be novel or publicly known compounds.

The compounds that increase the amount of the receptor protein of the present invention or its partial peptides thereby to potentiate the cell-stimulating activities are useful as safe and low toxic pharmaceuticals for the prevention/treatment of diseases associated with dysfunction of the receptor protein of the present invention.

The compounds that decrease the amount of the receptor protein of the present invention or its partial peptides thereby to lower the cell-stimulating activities are useful as safe and low toxic pharmaceuticals for the prevention/treatment of diseases caused by overexpression of the receptor protein of the present invention.

Specifically, the compounds or their salts having the effects that alter the amount of the receptor protein of the present invention or its partial peptides can be used as preventive/therapeutic agents for diseases, for example, central dysfunctions (e.g., Alzheimer's disease, senile dementia, suppression of eating, etc.), inflammatory diseases (e.g., allergic disease, asthma, rheumatism, etc.), cardiovascular diseases (e.g., hypertension, cardiac hypertrophy, angina pectoris, arteriosclerosis, etc.), cancer (e.g., non-small cell lung carcinoma, ovarian cancer, prostate cancer, stomach cancer, bladder cancer, breast cancer, uterocervical cancer, colon cancer, rectum cancer, etc.), mellitus diabetes, immune system diseases (e.g., autoimmune disease, AIDS, atopic dermatitis, allergic disease, immunodeficiency, asthma, rheumatic arthritis, psoriasis, arteriosclerosis, mellitus diabetes, Alzheimer's disease, etc.), liver/gall bladder diseases (e.g., hepatic cirrhosis, hepatitis, hepatic insufficiency, cholestasis, calculus, etc.), alimentary disorders (e.g., ulcer, enteritis, apepsia, irritable colon syndrome, ulcerative colitis, diarrhea, ileus, etc.), anxiety, pains, obesity, etc.; cell growth regulators, cell differentiation regulators, apoptosis regulators, etc.

Where the compounds or their salt forms obtained by the screening methods of the present invention are used in the form of pharmaceutical compositions as the preventive/therapeutic agents or the regulators described above, the compounds can be manufactured into pharmaceutical preparations by conventional means.

For example, the compounds can be used orally in the form of tablets which may be sugar coated if necessary, capsules, elixirs, microcapsules, etc., or parenterally in the form of injectable preparations such as a sterile solution, a suspension, etc. in water or with other pharmaceutically acceptable liquid can be formulated. These preparations can be manufactured, e.g., by mixing the compounds with a carrier, flavoring agent, excipient, vehicle, antiseptic agent, stabilizer, binder, etc., which is physiologically acceptable and publicly known, in a unit dosage form required in a generally accepted manner that is applied to making pharmaceutical preparations. The effective component in these preparations is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin, alginic acid, etc., a lubricant such as magnesium stearate, a sweetening agent such as sucrose, lactose or saccharin, a flavoring agent such as peppermint, akamono oil and cherry, and the like. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil, coconut oil, etc., or the like. Examples of an aqueous medium for injection include physiological saline and an isotonic solution containing glucose and other auxiliary agents (e.g., D-sorbitol, D-mannitol, sodium chloride, etc.) or the like and may be used in combination with an appropriate dissolution aid such as an alcohol (e.g., ethanol), a polyalcohol (e.g., propylene glycol and polyethylene glycol), a nonionic surfactant (e.g., polysorbate 80™ and HCO-50), etc. Examples of the oily medium include sesame oil, soybean oil, etc., which may also be used in combination with a dissolution aid such as benzyl benzoate, benzyl alcohol, etc.

The preventive/therapeutic agent or regulator described above may further be formulated with a buffer (e.g., phosphate buffer or sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

Since the thus obtained pharmaceutical preparation is safe and low toxic, the preparation can be administered to, e.g., human or mammals (e.g., rats, mice, rabbits, sheep, swine, bovine, cats, dogs, monkeys, etc.).

The dose of the compounds or their salts varies depending on subject to be administered, target organs, conditions, routes for administration, etc.; in oral administration, e.g., to the patient (as 60 kg body weight) with hypertension, the dose is normally about 0.1 mg to about 100 mg, preferably about 1.0 to about 50 mg, and more preferably about 1.0 to about 20 mg per day. In parenteral administration, the single dose varies depending on subject to be administered, target organ, conditions, routes for administration, etc. but it is advantageous, e.g., for the patient (as 60 kg body weight) with hypertension, to administer intravenously in a daily dose of about 0.01 to about 30 mg, preferably about 0.1 to about 20 mg, and more preferably about 0.1 to about 10 mg. For other animal species, the corresponding dose as converted per 60 kg body weight can be administered.

(8) Pharmaceutical comprising a neutralizing antibody to the receptor protein of the present invention, its partial peptides or salts thereof

The neutralizing activity of the antibodies to the receptor protein of the present invention, its partial peptides or salts thereof refers to an activity of inactivating the signal transduction functions in which the receptor protein participates. Therefore, where the antibodies have the neutralizing activity, the signal transduction in which the receptor protein participates, for example, the receptor protein-mediated cell stimulating activities (e.g., the activities that promote or inhibit arachidonic acid release, acetylcholine release, intracellular $Ca^{2+}$ release (intracellular $Ca^{2+}$ level increase), intracellular cAMP production, intracellular cGMP production, inositol phosphate production, changes in cell membrane potential, phosphorylation of intracellular proteins, activation of c-fos, pH reduction, etc.) can be inactivated.

Thus, the neutralizing antibodies to the receptor protein of the present invention, its partial peptides or salts thereof can be used as preventive/therapeutic agents for diseases caused by overexpression of the receptor protein, etc. (diseases, for example, central dysfunctions (e.g., Alzheimer's disease, senile dementia, suppression of eating, etc.), inflammatory diseases (e.g., allergic disease, asthma, rheumatism, etc.), cardiovascular diseases (e.g., hypertension, cardiac hypertrophy, angina pectoris, arteriosclerosis, etc.), cancer (e.g., non-small cell lung carcinoma, ovarian cancer, prostate cancer, stomach cancer, bladder cancer, breast cancer, uterocervical cancer, colon cancer, rectum cancer, etc.), mellitus diabetes, immune system diseases (e.g., autoimmune disease, AIDS, atopic dermatitis, allergic disease, immunodeficiency, asthma, rheumatic arthritis, psoriasis, arteriosclerosis, mellitus diabetes, Alzheimer's disease, etc.), liver/gall bladder diseases (e.g., hepatic cirrhosis, hepatitis, hepatic insufficiency, cholestasis, calculus, etc.), alimentary disorders (e.g., ulcer, enteritis, apepsia, irritable colon syndrome, ulcerative colitis, diarrhea, ileus, etc.), anxiety, pains, obesity, etc.); cell growth regulators, cell differentiation regulators or apoptosis regulators.

(9) Method of elucidating the action mechanism of various drugs

By using the receptor protein of the present invention, it can be confirmed whether or not various drugs exert the pharmacological effects mediated by the receptor protein of the present invention.
Thus, the present invention provides:

(1) a method of confirmation, which is characterized by using the receptor protein of the present invention thereby to confirm that a preventive/therapeutic drug for diseases such as central dysfunctions (e.g., Alzheimer's disease, senile dementia, suppression of eating, etc.), inflammatory diseases (e.g., allergic disease, asthma, rheumatism, etc.), cardiovascular diseases (e.g.; hypertension, cardiac hypertrophy, angina pectoris, arteriosclerosis, etc.), cancer (e.g., non-small cell lung carcinoma, ovarian cancer, prostate cancer, stomach cancer, bladder cancer, breast cancer, uterocervical cancer, colon cancer, rectum cancer, etc.), mellitus diabetes, immune system diseases (e.g., autoimmune disease, AIDS, atopic dermatitis, allergic disease, immunodeficiency, asthma, rheumatic arthritis, psoriasis, arteriosclerosis, mellitus diabetes, Alzheimer's disease, etc.), liver/gall bladder diseases (e.g., hepatic cirrhosis, hepatitis, hepatic insufficiency, cholestasis, calculus, etc.), alimentary disorders (e.g., ulcer, enteritis, apepsia, irritable colon syndrome, ulcerative colitis, diarrhea, ileus, etc.), anxiety, pains, obesity, etc., binds to the receptor protein or its salts; and,
(2) a screening method according to (1), which is characterized by determining the binding amount of the receptor protein of the present invention when each drug is contacted with the receptor protein of the present invention.

This confirmation method can be carried out by using the drug described above in the method of screening the compound that alters the binding properties of the ligand to the receptor protein of the present invention described above, in place of the test compound.
Also, a kit for the confirmation method of the present invention comprises the drug described above in the kit for screening the compound that alters the binding properties of the ligand to the receptor protein of the present invention described above.
As such, by using the confirmation method of the present invention, it can be confirmed that various drugs commercially available or in the developing process exhibit their pharmacological effects mediated by the receptor protein of the present invention.

(10) Pharmaceutical comprising the antisense DNA of the present invention

The antisense DNA of the present invention can be used as a preventive/therapeutic agent for diseases caused by overexpression of the receptor protein of the present invention, for example, central dysfunctions (e.g., Alzheimer's disease, senile dementia, suppression of eating, etc.), inflammatory diseases (e.g., allergic disease, asthma, rheumatism, etc.), cardiovascular diseases (e.g., hypertension, cardiac hypertrophy, angina pectoris, arteriosclerosis, etc.), cancer (e.g., non-small cell lung carcinoma, ovarian cancer, prostate cancer, stomach cancer, bladder cancer, breast cancer, uterocervical cancer, colon cancer, rectum cancer, etc.), mellitus diabetes, immune system diseases (e.g., autoimmune disease, AIDS, atopic dermatitis, allergic disease, immunodeficiency, asthma, rheumatic arthritis, psoriasis, arteriosclerosis, mellitus diabetes, Alzheimer's disease, etc.), liver/gall bladder diseases (e.g., hepatic cirrhosis, hepatitis, hepatic insufficiency, cholestasis, calculus, etc.), alimentary disorders (e.g., ulcer, enteritis, apepsia, irritable colon syndrome, ulcerative colitis, diarrhea, ileus, etc.), anxiety, pains, obesity, etc.; cell growth regulators, cell differentiation regulators, apoptosis regulators, etc.
For example, where the antisense DNA is used, the antisense DNA itself may be administered solely; alternatively, the antisense DNA may be inserted into an appropriate vector such as retrovirus vector, adenovirus vector, adenovirus-associated virus vector, etc., which is then administered in a conventional manner. The antisense DNA may also be administered as intact DNA, or with adjuvants to assist its uptake by gene gun or through a catheter such as a catheter with a hydrogel.

Moreover, the antisense DNA may also be used as an oligonucleotide probe for diagnosis to investigate the presence of the DNA of the present invention or the state of its expression in tissues or cells.

(11) Preparation of transgenic animals carrying the DNA of the present invention

The present invention provides a non-human mammal bearing an exogenous DNA of the present invention (hereinafter merely referred to as the exogenous DNA of the present invention) or its variant DNA (sometimes simply referred to as the exogenous variant DNA of the present invention).

Thus, the present invention provides:

[1] a non-human mammal bearing the exogenous DNA of the present invention or its variant DNA;
[2] the mammal according to [1], wherein the non-human mammal is a rodent;
[3] the mammal according to [2], wherein the rodent is mouse or rat; and,
[4] a recombinant vector bearing the exogenous DNA of the present invention or its variant DNA and capable of being expressed in a mammal.

The non-human mammal bearing the exogenous DNA of the present invention or its variant DNA (hereinafter simply referred to as the DNA transgenic animal of the present invention) can be prepared by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method, etc. Also, the exogenous DNA of the present invention can be transfected into a somatic cell, a living organ, a tissue cell, or the like, by the DNA transfection methods, and the transformants can be utilized for cell culture, tissue culture, etc. In addition, these cells can be fused with the above-described germinal cell by a publicly known cell fusion method to prepare the transgenic animal of the present invention.

Examples of the non-human mammal used include bovine, swine, sheep, goats, rabbits, dogs, cats, guinea pigs, hamsters, mice, rats, and the like. Above all, preferred are rodents, especially mice (e.g., C57BL/6 strain, DBA2 strain, etc. for a pure line and for a cross line, B6C3F1 strain, BDF1 strain B6D2F1 strain, BALB/c strain, ICR strain, etc.) or rats (Wistar, SD, etc.) or the like, since they are relatively short in ontogeny and life cycle from a standpoint of preparing model animals for human disease.

"Mammals" in a recombinant vector that can be expressed in "mammals" include the aforesaid non-human mammals and human.

The exogenous DNA of the present invention refers to the DNA of the present invention that is once isolated/extracted from mammals, not the DNA of the present invention inherently possessed by the non-human mammals.

The mutant DNA of the present invention includes mutants resulting from variation (e.g., mutation, etc.) in the base sequence of the original DNA of the present invention, specifically DNAs resulting from base addition, deletion, substitution with other bases, etc. and further including abnormal DNA.

The abnormal DNA is intended to mean such a DNA that expresses the abnormal receptor protein of the present invention and exemplified by the DNA that expresses a receptor protein to suppress the functions of the normal receptor protein of the present invention.

The exogenous DNA of the present invention may be any one of those derived from a mammal of the same species as, or a different species from, the mammal as the target animal. In transfecting the DNA of the present invention to a target animal, it is generally advantageous to use the DNA as a DNA construct in which the DNA is ligated downstream a promoter capable of expressing the DNA in the target animal. For example, in the case of transfecting the human DNA of the present invention, a DNA transgenic mammal that expresses the DNA of the present invention to a high level can be prepared by microinjecting a DNA construct (e.g., vector, etc.) ligated with the human DNA of the present invention into a fertilized egg of the target non-human mammal downstream various promoters, which are capable of expressing the DNAs derived from various mammals (e.g., rabbits, dogs, cats, guinea pigs, hamsters, rats, mice, etc.) bearing the DNA of the present invention highly homologous to the human DNA.

As expression vectors for the receptor protein of the present invention, there are Escherichia coli-derived plasmids, Bacillus subtilis-derived plasmids, yeast-derived plasmids, bacteriophages such as λ phage, retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used.

Examples of these promoters for regulating the DNA expression described above include (1) promoters for the DNA derived from viruses (e.g., simian virus, cytomegalovirus, Moloney leukemia virus, JC virus, breast cancer virus, poliovirus, etc.), and (2) promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase 1 tissue inhibitor, MHC class I antigen (H-2L), H-ras, renin, dopamine β-hydroxylase, thyroid peroxidase (TPO), peptide chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among others them, cytomegalovirus promoters, human peptide elongation factor 1α (EF-1α) promoters, human and chicken β actin promoters, etc., which protein can highly express in the whole body are preferred.

It is preferred that the vectors described above have a sequence for terminating the transcription of the desired messenger RNA in the DNA transgenic animal (generally called a terminator); for example, a sequence of each DNA derived from viruses and various mammals. SV40 terminator of the simian virus, etc. are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for the normal receptor protein of the present invention can be acquired as a whole or a part of DNA derived from liver, kidney, thyroid cell or fibroblast of human or various mammals (e.g., rabbits, dogs, cats, guinea pigs, hamsters, rats, mice, etc.) or of the genomic DNA from various commercially available genomic DNA libraries, or using as a starting material complementary DNA prepared by a publicly known method from RNA derived from liver, kidney, thyroid cell or fibroblast. Also, an exogenous abnormal DNA can produce a translational region, which is obtained by point mutagenesis variation of the translational region in a normal receptor protein obtained from the cells or tissues described above.

The said translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA of the present invention is transfected at the fertilized egg cell stage in such a manner that the DNA is certainly present in all the germinal cells and somatic cells of the target mammal. The fact that the exogenous DNA of the present invention is present in the germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA in all of the germinal cells and somatic cells thereof.

The non-human mammal in which the normal exogenous DNA of the present invention has been transfected can be passaged as the DNA-bearing animal under ordinary rearing environment, by confirming that the exogenous DNA is stably retained by mating.

By the transfection of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfection means that the exogenous DNA of the present invention is excessively present in all of the germinal cells and somatic cells thereof. The offspring of the animal that inherits the exogenous DNA of the present invention have excessively the exogenous DNA of the present invention in all of the germinal cells and somatic cells thereof.

By obtaining a homozygotic animal having the transfected DNA in both of homologous chromosomes and mating a male and female of the animal, all offspring can be passaged to retain the DNA.

In a non-human mammal bearing the normal DNA of the present invention, the normal DNA of the present invention is expressed to a high level, and may eventually develop the hyperfunction of the receptor protein of the present invention by promoting the function of endogenous normal DNA. Therefore, the animal can be utilized as a pathologic model animal for such a disease. Specifically, using the normal DNA transgenic animal of the present invention, it is possible to elucidate the mechanism of the hyperfunction of the receptor protein of the present invention and the pathological mechanism of the disease associated with the receptor protein of the present invention and to determine how to treat the disease.

Furthermore, a mammal transfected the exogenous normal DNA of the present invention exhibits an increasing symptom of the receptor protein of the present invention librated, the animal is usable for screening therapeutic agents for the disease associated with the receptor protein of the present invention.

On the other hand, non-human mammal bearing the exogenous abnormal DNA of the present invention can be passaged under normal breeding conditions as the DNA-bearing animal by confirming the stable retaining of the exogenous DNA via crossing. Further, the objective exogenous DNA can be used as a starting material by inserting the DNA into the plasmid described above. The DNA construct with a promoter can be prepared by conventional DNA engineering techniques. The transfection of the abnormal DNA of the present invention at the fertilized egg cell stage is preserved to be present in all of the germinal and somatic cells of the mammals to be subjected. The fact that the abnormal DNA of the present invention is present in the germinal cells of the animal after DNA transfection means that all of the offspring of the prepared animal have the abnormal DNA of the present invention in all of the germinal and somatic cells. Such an offspring passaged the exogenous DNA of the present invention contains the abnormal DNA of the present invention in all of the germinal and somatic cells. A homozygous animal having the introduced DNA on both of homologous chromosomes can be acquired and then by mating these male and female animals, all the offspring can be bled to have the DNA.

Non-human mammal bearing the abnormal DNA of the present invention may express the abnormal DNA of the present invention at a high level, may be the function inactivation type inadaptability to the receptor protein of the present invention by inhibiting functions of the endogenous normal DNA, and may be utilized as its disease model animal. For example, using the abnormal DNA transgenic animal of the present invention, it is possible to elucidate the mechanism of inadaptability to the receptor protein of the present invention and study a method for the treatment of this disease.

More specifically, the transgenic animal of the present invention expressing the abnormal DNA of the present invention to a high level is also expected to serve as an experimental model to elucidate the mechanism of the functional inhibition (dominant negative effect) of normal receptor protein by the abnormal receptor protein of the present invention in the function inactive type inadaptability to the receptor protein of the present invention.

A mammal bearing the abnormal exogenous DNA of the present invention is also expected to serve as a screening test on therapeutic drugs for the function inactive type inadaptability to the receptor protein of the present invention, since the receptor protein of the present invention increases in such animal in its free form.

Other potential applications of two kinds of the transgenic animals described above include, for example:

(1) use as a cell source for tissue culture;
(2) elucidation of relation to a receptor protein specifically expressed or activated by the receptor protein of the present invention, through direct analysis of DNA or RNA in tissues of the DNA transgenic animal of the present invention or by analysis of tissues of the receptor protein expressed by the DNA;
(3) research on functions of the cells derived from tissues that are cultured usually only with difficulty, using cells of a tissue bearing the DNA cultured by a standard tissue culture technique;
(4) screening of such a drug that enhances the functions of cells using the cells described in (3) above; and,
(5) isolation and purification of the variant receptor protein of the present invention and preparation of antibodies thereto; etc.

Furthermore, clinical conditions of a disease associated wit the receptor protein of the present invention, including the function inactive type inadaptability to the receptor protein of the present invention can be determined using the DNA transgenic animal of the present invention. Also, pathological findings on each organ in a disease model associated with the receptor protein of the present invention are obtained in more detail, which can contribute to development of a new method for treatment as well as the research and therapy of any secondary diseases associated with the disease.

It is also possible to obtain a free DNA-transfected cell by withdrawing each organ from the DNA transgenic animal of the present invention, mincing the organ and digesting with a proteinase such as trypsin, etc., followed by establishing the line of culturing or cultured cells. Furthermore, the DNA transgenic animal can serve as identification of a cell capable of producing the receptor 'protein of the present invention, and as studies on relevancy to apoptosis, differentiation or proliferation, or on the mechanism of signal transduction in these properties to inspect any abnormality therein. Thus, the DNA transgenic animal can provide an effective research material to elucidate the receptor protein of the present invention and its function and effect.

To develop a therapeutic drug for the treatment of diseases associated with the receptor protein of the present invention, including the function inactive type inadaptability to the receptor protein of the present invention, using the DNA transgenic animal of the present invention, an effective and rapid method for screening can be provided by using the method for inspection and the method for quantification, etc. described above. It is also possible to investigate and develop a method of DNA therapy for the treatment of diseases associated with the receptor protein of the present invention, using the DNA transgenic animal of the present invention or a vector capable of expressing the exogenous DNA of the present invention.

(12) Knockout animal

The present invention provides a non-human mammal embryonic stem cell bearing the DNA of the present invention inactivated and a non-human mammal deficient in expressing the DNA of the present invention.

Thus, the present invention provides:

[1] a non-human embryonic stem cell in which the DNA of the present invention is inactivated;
[2] an embryonic stem cell according to [1], wherein the DNA is inactivated by introducing a reporter gene (e.g., β-galactosidase gene derived from Escherichia coli);
[3] an embryonic stem cell according to [1], which is resistant to neomycin;
[4] an embryonic stem cell according to [1], wherein the non-human mammal is a rodent;
[5] an embryonic stem cell according to [4], wherein the rodent is mouse;
[6] a non-human mammal deficient in expressing the DNA of the present invention, wherein the DNA of the present invention is inactivated;
[7] a non-human mammal according to [6], wherein the DNA is inactivated by inserting a reporter gene (e.g., β-galactosidase derived from Escherichia coli) therein and the reporter gene is capable of being expressed under control of a promoter to the DNA of the present invention;
[8] a non-human mammal according to [6], which is a rodent;
[9] a non-human mammal according to [8], wherein the rodent is mouse; and,
[10] a method of screening a compound or its salt that promotes or inhibits the promoter activity to the DNA of the present invention, which comprises administering a test compound to the mammal of [7] and detecting expression of the reporter gene.

The non-human mammal embryonic stem cell in which the DNA of the present invention is inactivated refers to a non-human mammal embryonic stem cell that suppresses the ability of the non-human mammal to express the DNA by artificially mutating the DNA of the present invention, or the DNA has no substantial ability to express the receptor protein of the present invention (hereinafter sometimes referred to as the knockout DNA of the present invention) by substantially inactivating the activity of the receptor protein of the present invention encoded by the DNA (hereinafter merely referred to as ES cell).

The same examples as described above apply to the non-human mammal.

Techniques for artificially mutating the DNA of the present invention include deletion of a part or all of the DNA sequence and insertion of or substitution with other DNA, by genetic engineering. By these variations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention the desired non-human mammal has, inserting a reporter gene, etc. including a neomycin resistant gene or a hygromycin resistant gene, or a reporter gene such as lacZ (β-galactosidase gene) or cat (chloramphenicol acetyltransferase gene), etc. into its exon site thereby to disable the functions of exon; or by inserting a DNA sequence which terminates gene transcription (e.g., polyA additional signal, etc.) in the intron between exons thereby to inhibit the synthesis of complete messenger RNA to eventually destroy the gene, transfecting a DNA strand having the thus constructed DNA strand (hereinafter simply referred to as targeting vector) to a chromosome of the subject animal by, e.g., homologous recombination. The thus obtained ES cells are analyzed by the southern hybridization analysis using a DNA sequence on or near the DNA of the present invention as a probe, or by PCR analysis using a DNA sequence on the targeting vector and another DNA sequence near the DNA of the present invention, which is not included in the targeting vector as primers, thereby to select the knockout ES cell of the present invention.

The parent ES cells to inactivate the DNA of the present invention by homologous recombination, etc. may be the strain already established as described above, or may be originally established by the publicly known method by Evans and Kaufman with modifications. For example, in the case of mouse ES cells, currently it is common practice to use ES cells of the 129 strain. However, since their immunological background is obscure in general, the C57BL/6 mouse or the BDF1 mouse (F1 hybrid between C57BL/6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The BDF1 mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, in addition to the advantages that the ovum availability per animal is high and ova are robust.

In establishing ES cells, blastocytes at 3.5 days after fertilization are commonly used. In the present invention, embryos are preferably collected at the 8-cell stage, after culturing until the blastocyte stage, the embryos are used to efficiently obtain a large number of early stage embryos.

Although the ES cells used may be of either sex, male ES cells are generally more convenient for generation of a germ cell line chimera and are therefore preferred. It is also desirable that sexes are identified as soon as possible to save painstaking culture time.

Methods for sex identification of the ES cell include a method in which a gene in the sex-determining region on the Y-chromosome is amplified by the PCR method and detected. When this method is used, one colony of ES cells (about 50 cells) is sufficient for sex-determination analysis, which karyotype analysis, for example G-banding method, requires about $10^6$ cells; therefore, the first selection of ES cells at the early stage of culture can be based on sex identification, and male cells can be selected early, which saves a significant amount of time at the early stage of culture.

Second selection can be achieved by, for example, the number of chromosome confirmation by the G-banding method. It is usually desirable that the chromosome number of the obtained ES cells be 100% of the normal number. However, when it is difficult to obtain the cells having the normal number of chromosomes due to physical operation etc. in cell establishment, it is desirable that the ES cell be again cloned to a normal cell (e.g., in mouse cells having the number of chromosomes being 2n = 40) after the gene of the ES cells is rendered knockout.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably 5% carbon dioxide and 95% air, or 5% oxygen, 5% carbon dioxide and 90% air) in the presence of LIF (1-10000 U/ml) on appropriate feeder cells such as STO fibroblasts, treated with a trypsin/EDTA solution (normally 0.001 to 0.5% trypsin/ 0.1 to 5 mM EDTA, preferably about 0.1 % trypsin/1 mM EDTA) at the time of passage to obtain separate single cells, which are then seeded on freshly prepared feeder cells. This passage is normally conducted every 1 to 3 other days; it is desirable that cells be observed at passage and cells found to be morphologically abnormal in culture, if any, be abandoned.

Where the ES cells are allowed to reach a high density in mono-layers or to form cell aggregates in suspension under appropriate conditions, they will spontaneously differentiate to various cell types, for example, pariental and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the DNA of the present invention obtained by differentiation of the ES cells of the present invention are useful in studying the receptor protein of the present invention in vitro or the receptor protein of the present invention from an aspect of cell biology.

The non-human mammal deficient in expression of the DNA of the present invention can be identified from a normal animal by measuring the mRNA level in the subject animal by a publicly known method, and indirectly comparing the expression levels.

The same examples as described above apply to the non-human mammal used.

With respect to the non-human mammal deficient in expression of the DNA of the present invention, the DNA of the present invention can be made knockout by transfecting the targeting vector prepared as described above to non-human mammal embryonic stem cells or oocytes thereof, and conducting homologous recombination in which the targeting vector DNA sequence, wherein the DNA of the present invention is inactivated by the transfection, is replaced by the DNA of the present invention on a chromosome of a non-human mammal embryonic stem cell or embryo thereof.

The cells with the DNA of the present invention knockout can be identified by the southern hybridization analysis using a DNA sequence on or near the DNA of the present invention as a probe, or by PCR analysis using as primers a DNA sequence on the targeting vector and another DNA sequence, which is not included in the targeting vector. When non-human mammalian embryonic stem cells are used, a cell line wherein the DNA of the present invention is inactivated by homologous recombination is cloned; the cloned cell is injected to, e.g., a non-human mammalian embryo or blastocyst, at an appropriate stage such as the 8-cell stage. The produced chimeric embryos are transplanted to the uterus of the pseudopregnant non-human mammal. The resulting animal is a chimeric animal composed of both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the receptor protein of the present invention. The individuals deficient in homozygous expression of the receptor protein of the present invention can be obtained from offspring of the intercross between the heterozygotes of the receptor protein of the present invention.

When an oocyte or egg cell is used, a DNA solution may be injected, e.g., to the prenucleus by microinjection to obtain a transgenic non-human mammal having a targeting vector introduced in a chromosome thereof. From such transgenic non-human mammals, those having a mutation at the locus of the DNA of the present invention can be obtained by selection based on homologous recombination.

As described above, individuals in which the DNA of the present invention is rendered knockout permit passage rearing under ordinary rearing conditions, after the individuals obtained by their crossing have proven to have been knockout.

Furthermore, the genital system may be obtained and maintained by conventional methods. That is, by crossing male and female animals each having the inactivated DNA, homozygote animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell, in which the DNA of the present invention is inactivated, is very useful for preparing a non-human mammal deficient in expression of the DNA of the present invention.

Since the non-human mammal, in which the DNA of the present invention is inactivated, lacks various biological activities derived from the receptor of the present invention, such an animal can be a disease model suspected of inactivated biological activities of the receptor of the present invention and thus, offers an effective study to investigate causes for and therapy for these diseases.

(12a) Method of screening compounds having therapeutic/preventive effects on diseases caused by deficiency, damages, etc. of the DNA of the present invention

The non-human mammal deficient in expression of the DNA of the present invention can be used for screening of compounds having therapeutic/preventive effects on diseases caused by deficiency, damages, etc. of the DNA of the present invention, compounds having effects on cell growth regulation, cell differentiation regulation or apoptosis regulation; etc.

That is, the present invention provides a method of screening of a compound or its salt having therapeutic/ preventive effects on diseases caused by deficiency, damages, etc. of the DNA of the present invention, which comprises administering a test compound to the non-human mammal deficient in expression of the DNA of the present invention and observing/measuring changes occurred in the animal.

As the non-human mammal deficient in expression of the DNA of the present invention which can be used for the screening method, the same examples as given hereinabove apply.

Examples of test compounds include peptides, proteins, non-peptide compounds, synthetic compounds, fermentation products, cell extracts, vegetable extracts, animal tissue extracts, blood plasma, etc. These compounds may be novel compounds or publicly known compounds.

Specifically, the non-human mammal deficient in expression of the DNA of the present invention is treated with a test compound, comparison is made with an intact animal for control and changes in the respective organs, tissues, disease conditions, etc. of the animal is used as an indicator to assess the therapeutic/preventive effects of the test compound.

For treating an animal to be tested with a test compound, for example, oral administration, intravenous injection, etc. are applied and the treatment is appropriately selected depending upon conditions of the test animal, properties of the test compound, etc. Furthermore, the dose of a test compound to be administered can be appropriately chosen depending on method of administration, property of test compound, etc.

For example, in the screening method described above, when a test compound is administered to a test animal and found to reduce the blood sugar level of the animal to at least about 10%, preferably at least about 30% and more preferably at least about 50%, the test compound can be selected to be a compound having a therapeutic/ preventive effect for the diseases above.

The compound obtained using the screening method above is a compound selected from the test compounds described above and exhibits therapeutic/preventive effects on diseases caused by deficiencies, damages, etc. of the receptor protein of the present invention (for example, central dysfunctions (e.g., Alzheimer's disease, senile dementia, suppression of eating, etc.), inflammatory diseases (e.g., allergic disease, asthma, rheumatism, etc.), cardiovascular diseases (e.g., hypertension, cardiac hypertrophy, angina pectoris, arteriosclerosis, etc.), cancer (e.g., non-small cell lung carcinoma, ovarian cancer, prostate cancer, stomach cancer, bladder cancer, breast cancer, uterocervical cancer, colon cancer, rectum cancer, etc.), mellitus diabetes, immune system diseases (e.g., autoimmune disease, AIDS, atopic dermatitis, allergic disease, immunodeficiency, asthma, rheumatic arthritis, psoriasis, arteriosclerosis, mellitus diabetes, Alzheimer's disease, etc.), liver/gall bladder diseases (e.g., hepatic cirrhosis, hepatitis, hepatic insufficiency, cholestasis, calculus, etc.), alimentary disorders (e.g., ulcer, enteritis, apepsia, irritable colon syndrome, ulcerative colitis, diarrhea, ileus, etc.), anxiety, pains, obesity, etc.). Therefore, the compound can be used as a safe and low toxic agent for the treatment/prevention of these diseases, and as a drug such as a cell growth regulator, a cell differentiation regulator, an apoptosis regulator, etc. Furthermore, compounds derived from the compound obtained by the screening described above can be similarly used.

The compound obtained by the screening method above may be in the form of salts. As such salts, there may be used salts with physiologically acceptable acids (e.g., inorganic acids, organic acids, etc.) or bases (e.g., alkali metal salts, etc.), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

A pharmaceutical composition comprising the compound obtained by the above screening method, or salts thereof, may be manufactured in a manner similar to the method of preparing the pharmaceutical composition comprising the compound that alters the binding properties of the receptor protein of the present invention to the ligand described hereinabove.

The thus obtained pharmaceutical preparation is safe and low toxic, and can be administered to, e.g., human or mammals (e.g., rats, mice, rabbits, sheep, swine, bovine, cats, dogs, monkeys, etc.).

The dose of the compounds or their salts varies depending on target disease, subject to be administered, routes for administration, etc. For example, when the compound is orally administered generally to the patient (as 60 kg body weight) with hypertension, the dose is normally about 0.1 mg to about 100 mg, preferably about 1.0 to about 50 mg, and more preferably about 1.0 to about 20 mg per day. In parenteral administration, the single dose varies depending on subject to be administered, target disease, etc. but it is advantageous, e.g., for the patient (as 60 kg body weight) with hypertension, to administer intravenously in a daily dose of about 0.01 to about 30 mg, preferably about 0.1 to about 20 mg, and more preferably about 0.1 to about 10 mg. For other animal species, the corresponding dose as converted per 60 kg body weight can be administered.

(12b) Method of screening a compound that promotes or inhibits the activities of a promoter to the DNA of the present invention

The present invention provides a method of screening a compound or its salt that promotes or inhibits the activity of a promoter to the DNA of the present invention, which comprises administering a test compound to a non-human mammal deficient in expression of the DNA of the present invention and detecting expression of the reporter gene.

In the screening method above, the non-human mammal deficient in expression of the DNA of the present invention is selected from the aforesaid non-human mammal deficient in expression of the DNA of the present invention, as an animal in which the DNA of the present invention is inactivated by introducing a reporter gene and the reporter gene is expressed under control of a promoter to the DNA of the present invention.

The same examples of the test compound described above apply to the test compound.

As the reporter gene, the same specific examples apply to this screening method. Preferably employed are β-galactosidase (lacZ), soluble alkaline phosphatase gene, luciferase gene and the like.

In the non-human mammal deficient in expression of the DNA of the present invention wherein the DNA of the present invention is substituted with the reporter gene, a reporter gene is present under control of a promoter to the DNA of the present invention, and thus the activity of the promoter can be detected by tracing expression of a substance encoded by the reporter gene.

When a part of the DNA region encoding the receptor protein of the present invention is substituted with, e.g., β-galactosidase gene (lacZ) derived from Escherichia coli, β-galactosidase is expressed in a tissue where the receptor protein of the present invention should originally be expressed, instead of the receptor protein of the present invention. Thus, the state of expression of the receptor protein of the present invention can be readily observed in vivo of animal by staining with a reagent, e.g., 5-bromo-4-chloro-3-indolyl-p-galactopyranoside (X-gal) which is a substrate for β-galactosidase. Specifically, a mouse deficient in the receptor protein of the present invention, or its tissue slice section is fixed with glutaraldehyde, etc. After washing with phosphate buffered saline (PBS), the system is reacted with a staining solution containing X-gal at room temperature or about 37° C for approximately 30 minutes to an hour. After the β-galactosidase reaction is terminated by washing the tissue preparation with 1 mM EDTA/PBS solution, the color formed is observed. Alternatively, mRNA encoding lacZ may be detected in a conventional manner.

The compound or its salts obtained by using the aforesaid screening method are compounds selected from the test compounds described above and the compounds that promote or inhibit the promoter activity to the DNA of the present invention.

The compound obtained by the screening method above may be in the form of salts. As such salts, there may be used salts with physiologically acceptable acids (e.g., inorganic acids, organic acids, etc.) or bases (e.g., alkali metal salts, etc.), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The compound or its salt that promotes the promoter activity to the DNA of the present invention can promote expression of the receptor protein of the present invention to promote the functions of the receptor protein. Thus, the compound is useful as a drug such as a preventive/therapeutic agent for, e.g., diseases associated with dysfunction of the receptor protein of the present invention, etc.

The compound or its salt that inhibits the promoter activity to the DNA of the present invention can inhibit expression of the receptor protein of the present invention to inhibit the functions of the receptor protein. Thus, the compound is useful as a drug such as a preventive/therapeutic agent for, e.g., diseases related to overexpression of the receptor protein of the present invention, etc.

Specifically, the compound or its salt that promotes or inhibits the promoter activity to the DNA of the present invention can be used as a preventive/therapeutic agent for diseases, for example, central dysfunctions (e.g., Alzheimer's disease, senile dementia, suppression of eating, etc.), inflammatory diseases (e.g., allergic disease, asthma, rheumatism, etc.), cardiovascular diseases (e.g., hypertension, cardiac hypertrophy, angina pectoris, arteriosclerosis, etc.), cancer (e.g., non-small cell lung carcinoma, ovarian cancer, prostate cancer, stomach cancer, bladder cancer, breast cancer, uterocervical cancer, colon cancer, rectum cancer, etc.), mellitus diabetes, immune system diseases (e.g., autoimmune disease, AIDS, atopic dermatitis, allergic disease, immunodeficiency, asthma, rheumatic arthritis, psoriasis, arteriosclerosis, mellitus diabetes, Alzheimer's disease, etc.), liver/gall bladder diseases (e.g., hepatic cirrhosis, hepatitis, hepatic insufficiency, cholestasis, calculus, etc.), alimentary disorders (e.g., ulcer, enteritis, apepsia, irritable colon syndrome, ulcerative colitis, diarrhea, ileus, etc.), anxiety, pains, obesity, etc.; a cell growth regulator, a cell differentiation regulator, an apoptosis regulator, etc.

Furthermore, compounds derived from the compounds obtained by the screening described above may also be used similarly.

The aforesaid preventive/therapeutic agent or regulator comprising the compound obtained by the above screening method, or salts thereof, may be manufactured in a manner similar to the method of preparing the preventive/therapeutic agent or regulator comprising the compound that alters the binding properties of the receptor protein of the present invention to the ligand described hereinabove.

The thus obtained pharmaceutical preparation is safe and low toxic, and can be administered to, e.g., human or mammals (e.g., rats, mice, rabbits, sheep, swine, bovine, cats, dogs, monkeys, etc.).

The dose of the compounds or their salts varies depending on target disease, subject to be administered, routes for administration, etc. For example, when the compound that promotes or inhibits the promoter activity to the DNA of the present invention is orally administered generally to the patient (as 60 kg body weight) with hypertension, the dose is normally about 0.1 mg to about 100 mg, preferably about 1.0 to about 50 mg, and more preferably about 1.0 to about 20 mg per day. In parenteral administration, the single dose varies depending on subject to be administered, target disease, etc. but it is advantageous, e.g., for the patient (as 60 kg body weight) with hypertension, to administer intravenously in a daily dose of about 0.01 to about 30 mg, preferably about 0.1 to about 20 mg, and more preferably about 0.1 to about 10 mg. For other animal species, the corresponding dose as converted per 60 kg body weight can be administered.

As described above, the non-human mammal deficient in expressing the DNA of the present invention is extremely useful for screening a compound or its salt that promotes or inhibits the activity of promoter to the DNA of the present invention, and thus can greatly contribute to investigations of causes for various diseases caused by failure to express the DNA of the present invention or to development of preventive/therapeutic agents for these diseases.

Moreover, when a so-called transgenic animal (gene-transfected animal) is prepared by using a DNA containing the promoter region of the receptor protein of the present invention, ligating genes encoding various proteins downstream the same and injecting the genes into animal oocyte, the receptor protein can be specifically synthesized by the animal so that it becomes possible to investigate the activity in vivo. Furthermore, when an appropriate reporter gene is ligated to the promoter region described above to establish a cell line so as to express the gene, such can be used as a survey system of low molecular weight compounds that specifically promotes or suppresses the ability of producing the receptor protein itself of the present invention in vivo.

In the specification and drawings, the codes of bases, amino acids, etc. are shown by abbreviations and in this case, they are denoted in accordance with the IUPAC-IUB Commission on Biochemical Nomenclature or by the common codes in the art, examples of which are shown below. For amino acids that may have the optical isomer, L form is presented unless otherwise indicated.

| | |
|---|---|
| DNA | : deoxyribonucleic acid |
| cDNA | : complementary deoxyribonucleic acid |
| A | : adenine |
| T | : thymine |
| G | : guanine |
| C | : cytosine |
| RNA | : ribonucleic acid |
| mRNA | : messenger ribonucleic acid |

| | |
|---|---|
| dATP | : deoxyadenosine triphosphate |
| dTTP | : deoxythymidine triphosphate |
| dGTP | : deoxyguanosine triphosphate |
| dCTP | : deoxycytidine triphosphate |
| ATP | : adenosine triphosphate |
| EDTA | : ethylenediaminetetraacetic acid |
| SDS | : sodium dodecyl sulfate |
| Gly | : glycine |
| Ala | : alanine |
| Val | : valine |
| Leu | leucine |
| Ile | : isoleucine |
| Ser | : serine |
| Thr | : threonine |
| Cys | : cysteine |
| Met | : methionine |
| Glu | : glutamic acid |
| Asp | : aspartic acid |
| Lys | :lysine |
| Arg | : arginine |
| His | histidine |
| Phe | : phenylalanine |
| Tyr | : tyrosine |
| Trp | : tryptophan |
| Pro | : proline |
| Asn | : asparagine |
| Gln | : glutamine |
| pGlu | : pyroglutamic acid |
| Me | : methyl |
| Et | : ethyl |
| Bu | : butyl |
| Ph | : phenyl |
| TC | : thiazolidine-4(R)-carboxamide |

The substituents, protective groups and reagents, which are frequently used throughout the specification, are shown by the following abbreviations.

| | |
|---|---|
| Tos | : p-toluenesulfonyl |
| CHO | : formyl |
| Bzl | : benzyl |
| $Cl_2Bzl$ | : 2,6-dichlorobenzyl |
| Bom | : benzyloxymethyl |
| Z | : benzyloxycarbonyl |
| Cl-Z | : 2-chlorobenzyloxycarbonyl |
| Br-Z | : 2-bromobenzyloxycarbonyl |
| Boc | : t-butoxycarbonyl |
| DNP | : dinitrophenol |
| Trt | : trityl |
| Bum | : t-butoxymethyl |
| Fmoc | : N-9-fluorenylmethoxycarbonyl |
| HOBt | : 1-hydroxybenztriazole |
| HOOBt | : 3,4-dihydro-3-hydroxy-4-oxo-1,2,3-benzotriazine |
| HONB | : 1-hydroxy-5-norbornene-2,3-dicarboximide . |
| DCC | : N,N'-dicyclohexylcarbodiimide |

The sequence identification numbers in the sequence listing of the specification indicates the following sequence, respectively.

[SEQ ID NO: 1]

This shows the amino acid sequence of H7TMB56, which is a novel G protein-coupled receptor protein of the present invention derived from human hippocampus.

[SEQ ID NO: 2]

This shows the base sequence of cDNA encoding H7TMB56 having the amino acid sequence represented by SEQ ID NO: 1, which is a novel G protein-coupled receptor protein of the present invention derived from human hippocampus.

[SEQ ID NO: 3]

This shows the base sequence of cDNA encoding H7TMB56 having the amino acid sequence represented by SEQ ID NO: 1, which is a novel G protein-coupled receptor protein of the present invention derived from human hippocampus.

[SEQ ID NO: 4]

This shows the base sequence of primer 1 used for cloning of cDNA encoding H7TMB56 having the amino acid sequence represented by SEQ ID NO: 1, which is a novel G protein-coupled receptor protein of the present invention derived from human hippocampus.

[SEQ ID NO: 5]

This shows the base sequence of primer 2 used for cloning of cDNA encoding H7TMB56 having the amino acid sequence represented by SEQ ID NO: 1, which is a novel G protein-coupled receptor protein of the present invention derived from human hippocampus.

[SEQ ID NO: 6]

This shows the amino acid sequence of H7TMB56C, which is a novel G protein-coupled receptor protein of the present invention derived from human hippocampus.

[SEQ ID NO: 7]

This shows the base sequence of cDNA encoding a G protein-coupled receptor protein having the amino acid sequence represented by SEQ ID NO: 6.

[SEQ ID NO: 8]

This shows the base sequence of the primer used in EXAMPLE 6.

[SEQ ID NO: 9]

This shows the base sequence of the primer used in EXAMPLE 6.

[SEQ ID NO: 10]

This shows the base sequence of the primer used in EXAMPLE 6.
Transformant Escherichia coli DH5$\alpha$/pCRII-H7TMB56 obtained in EXAMPLE 1 described below has been deposited with International Patent Organisms Depository, National Institute of Advanced Industrial Science and Technology (formerly, National Institute of Bioscience and Human-Technology (NIBH), Ministry of International Trade and Industry), located at Central 6, 1-1-1 Higashi, Tsukuba, Ibaraki, Japan, under Accession Number FERM BP-6483 since September 4, 1998 and with Institute for Fermentation (IFO) under Accession Number IFO 16186 since June 19, 1998.

EXAMPLES

The present invention is described in detail below with reference to EXAMPLES, but is not deemed to limit the scope of the present invention thereto. The gene manipulation procedures using Escherichia coli were performed according to the methods described in the Molecular Cloning.

REFERENCE EXAMPLE 1

Cloning of human hippocampus-derived G protein-coupled receptor protein encoding cDNA and determination of its base sequence (1)

Using as a template human hippocampus cDNA (Marathon-Ready™ cDNA, CLONTECH, Inc.), PCR was carried out using 2 primers, i.e., primer 1 (SEQ ID NO: 4) and primer 2 (SEQ ID NO: 5). The reaction solution for PCR was as follows. A 1/10 aliquot of the cDNA described above was used as a template, a 1/50 aliquot of Advantage cDNA Polymerase Mix (CLONTECH, Inc.), 0.2 μM each of primer 1 (SEQ ID NO: 4) and primer 2 (SEQ ID NO: 5), 200 μM of dNTPs and a buffer attached to enzyme were added thereto to make the volume 25 μl. PCR was carried out (1) first at 95°C for 1 minute, then by repeating (2) 3 times one cycle set to include 94°C for 20 seconds and 72°C for 2 minutes, (3) 3 times one cycle set to include 94°C for 20 seconds and 68°C for 2 minutes, (4) 38 times one cycle set to include 94°C for 20 seconds, 63.5°C for 20 seconds and 68°C for 2 minutes and 20 seconds, (5) finally followed by extension at 68°C for 7 minutes. After the reaction, the PCR product was subcloned to plasmid vector pCRII (Invitrogen, Inc.) following the protocols of TA clonking kit (Invitrogen, Inc.), which was transfected to Escherihia coli DH5α. After clones bearing cDNA were selected in an LB agar medium containing ampicillin, the sequences of individual clones were analyzed to acquire cDNA sequence (SEQ ID NO: 2 and SEQ ID NO: 3) encoding a novel G protein-coupled receptor protein. The novel G protein-coupled receptor protein having the amino acid sequence (SEQ ID NO: 1) deduced from this cDNA was named H7TMB56.

Plasmid pCRII-H7TMB56 wherein cDNA (SEQ ID NO: 2) encoding the G protein-coupled receptor protein H7TMB56 of the present invention derived from human hippocampus was subcloned was transfected to Escherichia coli DH5α in accordance with methods publicly known to acquire transformant: Escherichia coli DHSα/pCRII-H7TMB56.

REFERENCE EXAMPLE 2

Cloning of human hippocampus-derived G protein-coupled receptor protein encoding cDNA and determination of its base sequence (2)

Using as a template human hippocampus cDNA (Marathon-Ready™ cDNA, CLONTECH, Inc.), PCR was carried out using 2 primers, i.e., primer 1 (SEQ ID NO: 4) and primer 2 (SEQ ID NO: 5). The reaction solution for PCR was as follows. A 1/10 aliquot of the cDNA described above was used as a template, a 1/50 aliquot of Advantage cDNA Polymerase Mix (CLONTECH, Inc.), 0.2 μM each of primer 1 (SEQ ID NO: 4) and primer 2 (SEQ ID NO: 5) , 200 μM of dNTPs and a buffer attached to enzyme were added thereto to make the volume 25 μl. PCR was carried out (1) first at 95°C for 1 minute, then by repeating (2) 3 times one cycle set to include 94°C for 20 seconds and 72°C for 2 minutes, (3) 3 times one cycle set to include 94°C for 20 seconds and 68°C for 2 minutes, (4) 38 times one cycle set to include 94°C for 20 seconds, 63.5°C for 20 seconds and 68°C for 2 minutes and 20 seconds, (5) finally followed by extension at 68°C for 7 minutes. After the reaction, the PCR product was subcloned to plasmid vector pCRII (Invitrogen, Inc.) following the protocols of TA clonking kit (Invitrogen, Inc.), which was transfected to Escherihia coli DH5α. After clones bearing cDNA were selected in an LB agar medium containing ampicillin, the sequences of individual clones were analyzed to acquire cDNA sequence (SEQ ID NO: 7) encoding a novel G protein-coupled receptor protein. The novel G protein-coupled receptor protein having the amino acid sequence (SEQ ID NO: 6) deduced from this cDNA was named H7TMB56C.

REFERENCE EXAMPLE 3

Cloning of human genome-derived G protein-coupled receptor protein encoding cDNA and determination of its base sequence

Using as a template human genome DNA (about 1ng/μl), PCR was carried out using 2 primers, i.e., primer 1 (SEQ ID NO: 4) and primer 2 (SEQ ID NO: 5). The reaction solution for PCR was as follows. A 1/25 aliquot of the reaction solution using the genome DNA as a template was used, a 1/50 aliquot of Advantage cDNA Polymerase Mix (CLONTECH, Inc.), 0.2 μM each of primer 1 (SEQ ID NO: 4) and primer 2 (SEQ ID NO: 5), 200 μM of dNTPs and a buffer attached to enzyme were added to make the volume 25 μl. PCR was carried out (1) at 94°C for 1 minute, then by repeating (2) 4 times one cycle set to include 94°C for 20 seconds and 72°C for 2 minutes, (3) 4 times one cycle set to include 94°C for 20 seconds and 70°C for 2 minutes, (4) 27 times one cycle set to include 94°C for 20 seconds, 68°C for 2 minutes, (5) finally followed by extension at 68°C for 7 minutes. After the reaction, the PCR product was subcloned to plasmid vector pCRII (Invitrogen, Inc.) following the protocols of TA clonking kit (Invitrogen, Inc.), which was transfected to Escherihia coli DH5α. After clones bearing the genome fragments were selected in an LB agar medium containing ampicillin, the sequences of individual clones were analyzed. From the results it was confirmed that these base sequences were the same as the DNA sequences (SEQ ID NO: 2, SEQ ID NO: 3 and SEQ ID NO: 7) encoding the novel G protein-coupled receptor proteins acquired in EXAMPLES 1 and 2.

REFERENCE EXAMPLE 4

Preparation of H7TMB56 or H7TMB56C expressing CHO cells

In a manner similar to REFERENCE EXAMPLE 1, plasmid pCRII-H7TMB56C, to which cDNA (SEQ ID NO: 7) encoding H7TMB56C was subcloned, was transfected to Escherichia coli DH5α by publicly known methods to acquire transformant: Escherichia coli DHSα/pCRII-H7TMB56C.

After culturing E.coli DHSα/pCRII-H7TMB56 or E.coli DH5α/pCRII-H7TMB56C prepared in REFERENCE EXAMPLE 1, plasmid DNA of pCRII-H7TMB56 or pCRII-H7TMB56C was prepared using Plasmid Mid Kit (Qiagen, Inc.). From these plasmids, cDNA encoding the G protein-coupled receptor protein H7TMB56 or H7TMB56C of the present invention was cloned to plasmid vector pcDNA3.1/V5/His for protein expression to construct plasmid pcDNA3.1-H7TMB56 or H7TMB56C for protein expression. The thus obtained plasmid was, after preparing large quantities of the plasmid DNA using Plasmid Mid Kit (Qiagen, Inc.), transfected to CHO dhfr⁻ cells using Celfect Transfection Kit (Amersham-Pharmacia-Biotech, Inc.) in accordance with the protocols attached. That is, 10 mg of DNA was co-precipitated with calcium phosphate in a suspension. The resulting suspension was added to a 10 cm Petri dish, on which 5 x $10^5$ or 1 x $10^6$ CHO dhfr$^-$ cells had been plated 24 hours before, followed by incubation in 10% fetal bovine serum-containing MEMα medium for a day. After subculture, the cells were cultured in a selective MEMα medium containing 0.4 mg/ml of G418 (GIBCO BRL, Inc., Inc.) and 10% dialyzed fetal bovine serum. The colony of the transformant (CHO/H7TMB56 or CHO/H7TMB56C) which became proliferated in the selective medium was named H7TMB56 or H7TMB56C expressing CHO cells.

After the total RNA was extracted from the selected H7TMB56 expressing CHO cells in a conventional manner, the mRNA level in H7TMB56 was assayed by the TaqMan method to calculate the copy number. The results are shown in Table 1.

[TABLE 1]

| Clone No. | Expression Level (copy/ng total RNA) | |
|---|---|---|
| | First Run | Second Run |
| 7 | 83046<br>104954 | 89931<br>71409 |
| 9 | 32632<br>61153 | 30669<br>28487 |
| 15 | 5146<br>7172 | 7970<br>4882 |

EXAMPLE 1

Increase in expression level of the reporter gene by isoprenoid-related substances in HEK 293 cells in which H7TMB56 was transiently expressed

The H7TMB56-specific stimulating activity by the isoprenoid-related substances was detected using as an indicator the expression level of reporter gene product (luciferase) produced by expression induction of CRE promoter.

HEK293 cells were suspended in growth medium (DMEM (Dulbecco's Modified Eagle Medium) (Gibco BRL) supplemented with 10% fetal bovine serum (Gibco BRL)) and plated on a 96-well plate of black well (Beckton-Dickinson, Inc.) coated with collagen in 1 x $10^5$ cells/well. After incubation overnight under conditions at 37°C in 5 % $CO_2$, the

cells were transfected as follows, using a reporter gene-bearing plasmid pCRE-Luc (Clontech) and an expression vector plasmid together, which was prepared by inserting H7TMB56 gene into expression vector pAKKO-111H (the same plasmid vector as pAKKO-1.11H described in Biochim. Biophys. Acta, Hinuma, S. et al., 1219, 251-259, 1994) in animal cells by a publicly known method, or for control pAKKO- 111H inserted with other G protein-coupled receptor protein gene.

OPTI-MEM-I (Gibco BRL) and LipofectamineTM 2000 Reagent (Gibco BRL) were mixed in 24:1 to prepare the Lipofectamine dilution. Also, OPTI-MEM-I, H7TMB56 expression vector plasmid or other receptor expression vector plasmid (240 ng/μl) and pCRE-Luc (240 ng/μl) were mixed in 24:0.9:0.1 to prepare the DNA dilution. The Lipofectamine dilution and the DNA dilution were equally mixed, and the mixture was allowed to stand for 20 minutes at room temperature to form the complex of DNA and Lipofectamine. Thereafter, 25 μl was added to the HEK293 cell-incubated plate, followed by further incubation overnight at 37°C in 5% $CO_2$.

The transfected HEK293 cells were washed with an assay buffer (DMEM supplemented with 0.1% bovine serum albumin), and commercially available compounds containing farnesyl pyrophosphate (Sigma) and geranylgeranyl pyrophosphate (Sigma) diluted with the assay buffer were added to the cells, respectively, in 2 x $10^{-4}$ to $10^{-5}$ M, followed by incubation at 37°C for 4 hours in 5% $CO_2$. After the supernatant was discarded, 50 μl of PicaGene LT2.0 (Toyo Ink Mfg. Co., Ltd.) was added as a substrate for assaying the luciferase activity and a luminescence level of the luciferase was measured on a plate reader (ARVO sx Multi-Label Counter, Wallac, Inc.).

The results displayed that the luciferase activity was increased by farnesyl pyrophosphate and geranylgeranyl pyrophosphate specifically to the HEK293 cells bearing the H7TMB56 gene having the base sequence represented by SEQ ID NO: 2 (FIGS. 5 to 7).

EXAMPLE 2

Establishment of CHO cells stably expressing H7TMB56 and detection of the cAMP production promoting activity mediated by H7TMB56

The same expression vector as used in EXAMPLE 1 was transfected to CHO dhfr$^-$ cells using Gene Transfer (Wako Pure Chemical Industries, Ltd.), which was cultured in nucleic acid-free medium to select transformants. The cells were further cloned and a highly reactive clone was selected using the promotion of cAMP production as an indicator, since the results of EXAMPLE 1 showed to cause the activation of CRE promoter. The intracellular cAMP level was assayed using cAMP Screen Kit (Applied Biosystems, Inc.). Examination of the selected clone (clone 22) confirmed the specificity in the agonist activities of farnesyl pyrophosphate and geranylgeranyl pyrophosphate to H7TMB56, and its dose dependency (FIGS. 8 and 9). Furthermore, the CAMP production promoting activities on H7TMB56 (FIG. 10) and mock cells (FIG. 11) were assayed with respect to various isoprenoid-related substances in a dose of 10 μM (1: isopentenyl pyrophosphate, 2: dimethylallyl pyrophosphate, 3: geranyl pyrophosphate, 4: farnesyl pyrophosphate, 5: geranylgeranyl pyrophosphate, 6: geraniol, 7: farnesol, 8: geranylgeraniol, 9: trans, trans-farnesol, 10: trans, trans-farnesyl bromide, 11: geranyl acetone, 12: geranyl acetate, 13: geranylamine and 14: geranyl chloride). As a result, the agonist activity specific to geranyl pyrophosphate was detected as well, in addition to farnesyl pyrophosphate and geranylgeranyl pyrophosphate (FIG. 10).

EXAMPLE 3

Detection of the intracellular calcium level change stimulating activity on CHO cells stably expressing H7TMB56

The highly reactive clone described in EXAMPLE 2 and mock cells for control were plated on a 96-well black plate (Coaster Coming) followed by incubation overnight. By pre-incubation with a calcium ion fluorescent indicator dye Fluo-3 AM (Dojindo Laboratories) for an hour, the dye was introduced into the cells. Changes in the intracellular calcium ion level were determined using FLIPR by Molecular Device, Inc. With respect to the fluorescence counts measured with passage of time, comparison was made on the dose dependency of the reaction, based on the integrated values of the counts. As a result, it was found that the intracellular calcium level change stimulating activity was also specific and dose-dependent, as shown in FIG. 12.

EXAMPLE 4

Dose dependency of the cAMP production promotion mediated by H7TMB56

In a manner similar to EXAMPLE 2, the intracellular cAMP production promoting activity was assayed on 5 isoprenoid-related substances. The results are shown in FIG. 13. It is understood from FIG. 13 that geranyl pyrophosphate (geranyl PP), farnesyl pyrophosphate (farnesyl PP) and geranylgeranyl pyrophosphate (geranylgeranyl PP) promoted the production of cAMP dose-dependently.

EXAMPLE 5

Activation of MAP kinase specific to H7TMB56 expression CHO cells

H7TMB56 expression CHO cells and mock cells were plated on a 6-well plate in 3 x $10^5$ cells/well, followed by incubation overnight. In order to make serum free, the medium was replaced by nucleic acid-free α-MEM medium supplemented with 0.1% BSA and incubation was further continued overnight. Immediately before the assay, preincubation was again carried out for 2 hours. After 0.1 μM of farnesyl pyrophosphate was added to the medium to initiate the treatment, the medium was removed by suction at regular time intervals and then a sample buffer for SDS-PAGE was added to terminate the reaction. SDS-PAGE was performed in a conventional manner to transfer the protein onto a nitrocellulose filter. Thereafter, phosphorylated p44/42 MAP kinase was detected following the instructions described in the manual of PhosphoPlus p44/42 MAPK (Thr202/Tyr204) Antibody Kit (Cell Signaling, Inc.). As a result, phosphorylation of MAP kinase was detected specifically to the H7TMB56 expression CHO cells by the treatment with farnesyl pyrophosphate, which revealed that the enzyme was activated (FIG. 14).

EXAMPLE 6

Analysis on tissue distribution of h7TMB56 mRNA in human by RT-PCR

For cDNAs to be used as templates in RT-PCR for detecting the expression level of mRNA, the products synthesized from polyA+ RNA (Clontech) derived from various tissues of human by the following procedure were used. Following the manual attached, the reaction was carried out at 42°C using a random primer from 1 μg of RNA and SuperScriptII reverse transcriptase (GIBCO BRL, Inc.) as a reverse transcriptase. After completion of the reaction, the mixture was precipitated with ethanol and the precipitate was dissolved in 100 μl. The expression level was quantified using Sequence Detection System Prism 7700. The following primers were used; 5'-GGCCCTAATTTACAGGATGCACC-3' (SEQ ID NO: 8) and 5'-GCCCACGAAGAAGTAAATGATGG-3' (SEQ ID NO: 9) for amplification and detection, and 5'-(Fam)TGGTTTGCATGTCCCTGTCCTCTCTAAA-(Tam et al.)-3' (SEQ ID NO: 10) as a TaqMan probe. The RT-PCR reaction solution was prepared by adding to 12.5 μl of TaqMan Universal PCR Master Mix (PE Biosystems) 0.225 μl each of 100 μM primer solutions, 1.25 μl of 5 μM TaqMan probe and 0.5 μl of the cDNA solution prepared above. Distilled water was added to the reaction solution to make the total volume 25 μl. PCR was carried out by maintaining at 50°C for 2 minutes and 95°C for 10 minutes, and then repeating 40 times one cycle to set to include 95°C for 15 seconds and 60°C for 1 minute.

The mRNA expression level of GPR7 obtained in various tissues from human was calculated in terms of the copy number per ng of poly(A)$^+$ RNA (FIG. 15).

EXAMPLE 7

Expression of H7TMB56 on cell membrane

An expression plasmid was constructed to express a fused protein of Green Fluorescent Protein (GFP) cDNA isolated from jelly fish Auquorea victoria, fused to H7TMB56 at the C-terminus to fit the translation frame. In this case, a fragment excised out of GFP expression vector pQBI25 (Takara Shuzo Co., Ltd.) was used as GFP cDNA. In H7TMB56, its termination codon was modified by PCR to recognition sequence with restriction enzyme NheI, and the GFP fragment was ligated thereto, which was inserted into the expression vector pAKKO-111H described in EXAMPLE 1. The thus obtained plasmid of the H7TMB56 and GFP-fused protein expression vector was transfected to CHO cells by the following procedure. CHO cells were suspended in a growth medium [DMEM (Dulbecco's Modified Eagle Medium) (GIBCO BRL, Inc.) supplemented with 10% fetal bovine serum, and plated on a Lab-TekII cover glass chamber with 4 chambers (Nalgen Nunc, Inc.) in a concentration of 0.6 x $10^5$ cells/chamber. After incubation overnight at 37°C in 5% $CO_2$, transfection was performed. For transfection, Lipofectamine™ 2000 Reagent (GIBCO BRL, Inc., Inc.) was used. First, 2 μl of Lipofectamine™ 2000 Reagent was mixed with 50 μl of OPTI-MEM-I (GIBCO BRL, Inc.). The mixture was allowed to stand for 20 minutes at room temperature to form the complex of DNA and Lipofectamine, and 100 μl was added to the CHO cells incubated in the chamber followed by further incubation overnight at 37°C for 5% $CO_2$. The medium was replaced by a medium for confocal microscopic observation [Hanks' Balanced Salt Solution (GIBCO BRL, Inc.) suspended with 0.1 % bovine albumin (Essentially Fatty Acid Free, Sigma)] and the fluorescent images of GFP were observed with a confocal microscope (Leica, Inc.). In this case, GFP was excited at 488 nm.

As a result, the fused protein of H7TMB56 and GFP was observed on the cell membrane. When farnesyl pyrophosphate was added to the medium in $10^{-6}$ M, it was found that the fluorescence of GFP was not observed on the cell membrane, but moved to the cytoplasm. This displayed that H7TMB56 was a G protein-coupled type receptor expressed on the cell membrane and at the same time, H7TMB56 was reacted with farnesyl pyrophosphate to move to the cytoplasm, namely, internalization occurred.

INDUSTRIAL APPLICABILITY

By using the G protein-coupled receptor protein of the present invention, its partial peptides or salts thereof and the isoprenoid-related substances, which are the ligands, compounds that alter the binding properties of the isoprenoid-related substances to the G protein-coupled receptor protein of the present invention, its partial peptides or salts thereof can be screened efficiently.

**Claims**

1. A method of screening a compound or its salt that alters the binding properties of a G protein-coupled receptor protein containing the same or substantially the same amino acid sequence as the amino acid sequence represented by SEQ ID NO: 1 or its salt to an isoprenoid-related substance, which comprises using (1) said receptor protein, its partial peptide, or a salt thereof and (2) the isoprenoid-related substance.

2. A method of screening according to claim 1, wherein the isoprenoid-related substance is a compound, in which a pyrophosphate group is bound to the tail of the recurring structure of at least 2 isoprenic units.

3. A method of screening according to claim 1, wherein the isoprenoid-related substance is farnesyl pyrophosphate (FPP), geranyl pyrophosphate (GPP) or geranylgeranyl pyrophosphate (GGPP).

4. A method of screening according to claim 1, wherein the G protein-coupled receptor protein is a protein consisting the amino acid sequence represented by SEQ ID NO: 1 or SEQ ID NO: 6.

5. A kit for screening a compound or its salt that alters the binding properties of a G protein-coupled receptor protein containing the same or substantially the same amino acid sequence as the amino acid sequence represented by SEQ ID NO: 1 to an isoprenoid-related substance, comprising (1) said receptor protein, its partial peptide, or a salt thereof and (2) the isoprenoid-related substance.

6. A compound or its salt that alters the binding properties of an isoprenoid-related substance to a G protein-coupled receptor protein containing the same or substantially the same amino acid sequence represented by SEQ ID NO: 1, or its salt, which is obtainable using the screening method according to claim 1 or the screening kit according to claim 5.

7. A pharmaceutical comprising a compound or its salt that alters the binding properties of an isoprenoid-related substance to a G protein-coupled receptor protein containing the same or substantially the same amino acid sequence as the amino acid sequence represented by SEQ ID NO: 1, or its salt.

8. A pharmaceutical according to claim 7, which is a cell growth regulator, a cell differentiation regulator or an apoptosis regulator.

9. A method of determining a ligand to a G protein-coupled receptor protein containing the same or substantially the same amino acid sequence as the amino acid sequence represented by SEQ ID NO: 1, or its salt, which comprises assaying the intracellular $Ca^{2+}$ level increasing activity or the intracellular cAMP producing activity, in the case where a test compound is brought in contact with a cell containing said G protein-coupled receptor protein.

10. A ligand obtained by the method according to claim 9.

11. A method of screening a compound or its salt that alters the binding properties of a G protein-coupled receptor protein containing the same or substantially the same amino acid sequence as the amino acid sequence represented by SEQ ID NO: 1 or its salt to an isoprenoid-related substance, which comprises comparing (i) the case where a G protein-coupled receptor protein containing the same or substantially the same amino acid sequence as the amino acid sequence represented by SEQ ID NO: 1, its partial peptide, or a salt thereof, is brought in contact with ① an isoprenoid-related substance or ② a compound or its salt that alters the binding properties of said receptor protein or its salt to the isoprenoid-related substance, and (ii) the case where a G protein-coupled receptor protein containing the same or substantially the same amino acid sequence as the amino acid sequence represented by SEQ ID NO: 1, its partial peptide, or a salt thereof is brought in contact with ① an isoprenoid-related substance or ② a compound or its salt that alters the binding properties of said receptor protein or its salt to the isoprenoid-related substance, and a test compound.

**12.** A method of screening a compound or its salt that alters the binding properties of a G protein-coupled receptor protein containing the same or substantially the same amino acid sequence as the amino acid sequence represented by SEQ ID NO: 1 or its salt to an isoprenoid-related substance, which comprises measuring a binding amount of ① a labeled form of the isoprenoid-related substance or ② a labeled form of the compound or its salt that alters the binding properties of said receptor protein or its salt and the isoprenoid-related substance, to the receptor protein, its partial peptide, or a salt thereof, (i) in the case where ① a labeled form of the isoprenoid-related substance or ② a labeled form of the compound or its salt that alters the binding properties of a G protein-coupled receptor protein containing the same or substantially the same amino acid sequence as the amino acid sequence represented by SEQ ID NO: 1 or its salt to the isoprenoid-related substance, is brought in contact with the receptor protein, its partial peptide, or a salt thereof, and (ii) in the case where ① a labeled form of the isoprenoid-related substance or ② a labeled form of the compound or its salt that alters the binding properties of a G protein-coupled receptor protein containing the same or substantially the same amino acid sequence as the amino acid sequence represented by SEQ ID NO: 1 or its salt to the isoprenoid-related substance, and a test compound are brought in contact with the receptor protein, its partial peptide, or a salt thereof; and comparing the binding amount between (i) and (ii).

**13.** A method of screening a compound or its salt that alters the binding properties of a G protein-coupled receptor protein containing the same or substantially the same amino acid sequence as the amino acid sequence represented by SEQ ID NO: 1 or its salt to an isoprenoid-related substance, which comprises measuring a binding amount of ① a labeled form of the isoprenoid-related substance or ② a labeled form of the compound or its salt that alters the binding properties of said receptor protein or its salt to the isoprenoid-related substance, to a cell or cell membrane fraction containing the receptor protein, (i) in the case where ① a labeled form of the isoprenoid-related substance or ② a labeled form of the compound or its salt that alters the binding properties of a G protein-coupled receptor protein containing the same or substantially the same amino acid sequence as the amino acid sequence represented by SEQ ID NO: 1 or its salt to the isoprenoid-related substance, is brought in contact with the cell or cell membrane fraction containing said receptor protein, and (ii) in the case where ① a labeled form of the isoprenoid-related substance or ② a labeled form of the compound or its salt that alters the binding properties of a G protein-coupled receptor protein containing the same or substantially the same amino acid sequence as the amino acid sequence represented by SEQ ID NO: 1 or its salt to the isoprenoid-related substance, and a test compound are brought in contact with the cell or cell membrane fraction containing said receptor protein; and comparing the binding amount between (i) and (ii).

**14.** A method of screening a compound or its salt that alters the binding properties of a G protein-coupled receptor protein containing the same or substantially the same amino acid sequence as the amino acid sequence represented by SEQ ID NO: 1 or its salt to an isoprenoid-related substance, which comprises measuring a cell stimulating activity mediated by said receptor protein, (i) in the case where ① an isoprenoid-related substance or ② a compound or its salt that alters the binding properties of a G protein-coupled receptor protein containing the same or substantially the same amino acid sequence as the amino acid sequence represented by SEQ ID NO: 1 or its salt to the isoprenoid-related substance, is brought in contact with a cell containing said receptor protein, and (ii) in the case where ① an isoprenoid-related substance or ② a labeled form of the compound or its salt that alters the binding properties of a G protein-coupled receptor protein containing the same or substantially the same amino acid sequence as the amino acid sequence represented by SEQ ID NO: 1 or its salt to the isoprenoid-related substance, and a test compound are brought in contact with the cell containing the receptor protein; and comparing the cell stimulating activity between (i) and (ii).

**15.** A screening method according to claim 14, wherein the cell stimulating activity is an intracellular $Ca^{2+}$ level increasing activity or an intracellular CAMP producing activity.

**16.** A screening method according to claims 11 through 15, wherein the isoprenoid-related substance is a compound, in which a pyrophosphate group is bound to the tail of the recurring structure of at least 2 isoprenic units.

**17.** A screening method according to claims 11 through 16, wherein the isoprenoid-related substance is farnesyl pyrophosphate (FPP), geranyl pyrophosphate (GPP) or geranylgeranyl pyrophosphate (GGPP).

**18.** A screening method according to claims 11 through 17, wherein the test compound is a compound designed to bind, based on the atomic coordinate and the position of the ligand-binding pocket in the active site of the receptor protein of the present invention containing the same or substantially the same amino acid sequence as the amino acid sequence represented by SEQ ID NO: 1, to the ligand-binding pocket.

**19.** A compound or its salt that alters the binding properties of a G protein-coupled receptor protein containing the same or substantially the same amino acid sequence as the amino acid sequence represented by SEQ ID NO: 1 or its salt to an isoprenoid-related substance, which is obtainable by using the screening method according to any one of claims 11 through 18.

**20.** A cell growth regulator, a cell differentiation regulator or an apoptosis regulator, comprising a G protein-coupled receptor protein containing the same or substantially the same amino acid sequence as the amino acid sequence represented by SEQ ID NO: 1, its partial peptide, or a salt thereof.

**21.** A cell growth regulator, a cell differentiation regulator or an apoptosis regulator, comprising a polynucleotide containing a polynucleotide encoding a G protein-coupled receptor protein containing the same or substantially the same amino acid sequence as the amino acid sequence represented by SEQ ID NO: 1 or its partial peptide.

**22.** A diagnostic for central dysfunctions, inflammatory diseases, cardiovascular diseases, cancer, mellitus diabetes, immune system diseases, liver/gall bladder diseases, alimentary disorders, anxiety, pains or obesity, comprising a polynucleotide containing a polynucleotide encoding a G protein-coupled receptor protein containing the same or substantially the same amino acid sequence as the amino acid sequence represented by SEQ ID NO: 1 or its partial peptide.

**23.** A cell growth regulator, a cell differentiation regulator or an apoptosis regulator, comprising an antibody to a G protein-coupled receptor protein containing the same or substantially the same amino acid sequence as the amino acid sequence represented by SEQ ID NO: 1, its partial peptide, or a salt thereof.

**24.** A diagnostic for central dysfunctions, inflammatory diseases, cardiovascular diseases, cancer, mellitus diabetes, immune system diseases, liver/gall bladder diseases, alimentary disorders, anxiety, pains or obesity, comprising an antibody to a G protein-coupled receptor protein containing the same or substantially the same amino acid sequence as the amino acid sequence represented by SEQ ID NO: 1, its partial peptide, or a salt thereof.

**25.** A cell growth regulator, a cell differentiation regulator or an apoptosis regulator, comprising a whole or part of the base sequence complementary to a polynucleotide containing a polynucleotide encoding a G protein-coupled receptor protein containing the same or substantially the same amino acid sequence as the amino acid sequence represented by SEQ ID NO: 1 or its partial peptide.

**26.** A cell growth regulator, a cell differentiation regulator or an apoptosis regulator, comprising a compound or its salt that alters the expression level of a G protein-coupled receptor protein containing the same or substantially the same amino acid sequence as the amino acid sequence represented by SEQ ID NO: 1.

**27.** A method of regulating cell differentiation or apoptosis, which comprises administering to a mammal an effective dose of ① a compound or its salt that alters the binding properties of an isoprenoid-related substance to a G protein-coupled receptor protein containing the same or substantially the same amino acid sequence as the amino acid sequence represented by SEQ ID NO: 1 or its salt, ② a G protein-coupled receptor protein containing the same or substantially the same amino acid sequence as the amino acid sequence represented by SEQ ID NO: 1, its partial peptide, or a salt thereof, ③ a polynucleotide containing a polynucleotide encoding a G protein-coupled receptor protein containing the same or substantially the same amino acid sequence as the amino acid sequence represented by SEQ ID NO: 1 or its partial peptide, ④ an antibody to a G protein-coupled receptor protein containing the same or substantially the same amino acid sequence as the amino acid sequence represented by SEQ ID NO: 1, its partial peptide, or a salt thereof, or ⑤ a polynucleotide comprising a whole or part of the base sequence complementary to a polynucleotide comprising a polynucleotide encoding a G protein-coupled receptor protein containing the same or substantially the same amino acid sequence as the amino acid sequence represented by SEQ ID NO: 1 or its partial peptide.

**28.** Use of ① a compound or its salt that alters the binding properties of an isoprenoid-related substance to a G protein-coupled receptor protein containing the same or substantially the same amino acid sequence as the amino acid sequence represented by SEQ ID NO: 1 or its salt, ② a G protein-coupled receptor protein containing the same or substantially the same amino acid sequence as the amino acid sequence represented by SEQ ID NO: 1, its partial peptide, or a salt thereof, ③ a polynucleotide containing a polynucleotide encoding a G protein-coupled receptor protein containing the same or substantially the same amino acid sequence as the amino acid sequence represented by SEQ ID NO: 1 or its partial peptide, ④ an antibody to a G protein-coupled receptor protein containing the same or substantially the same amino acid sequence as the amino acid sequence represented by SEQ ID NO: 1, its partial peptide, or a salt thereof, or ⑤ a polynucleotide comprising a whole or part of the base sequence complementary to a polynucleotide comprising a polynucleotide encoding a G protein-coupled receptor protein containing the same or substantially the same amino acid sequence as the amino acid sequence represented by SEQ ID NO: 1 or its partial peptide; for manufacturing a cell growth regulator, a cell differentiation regulator or an apoptosis regulator.

Fig. 1

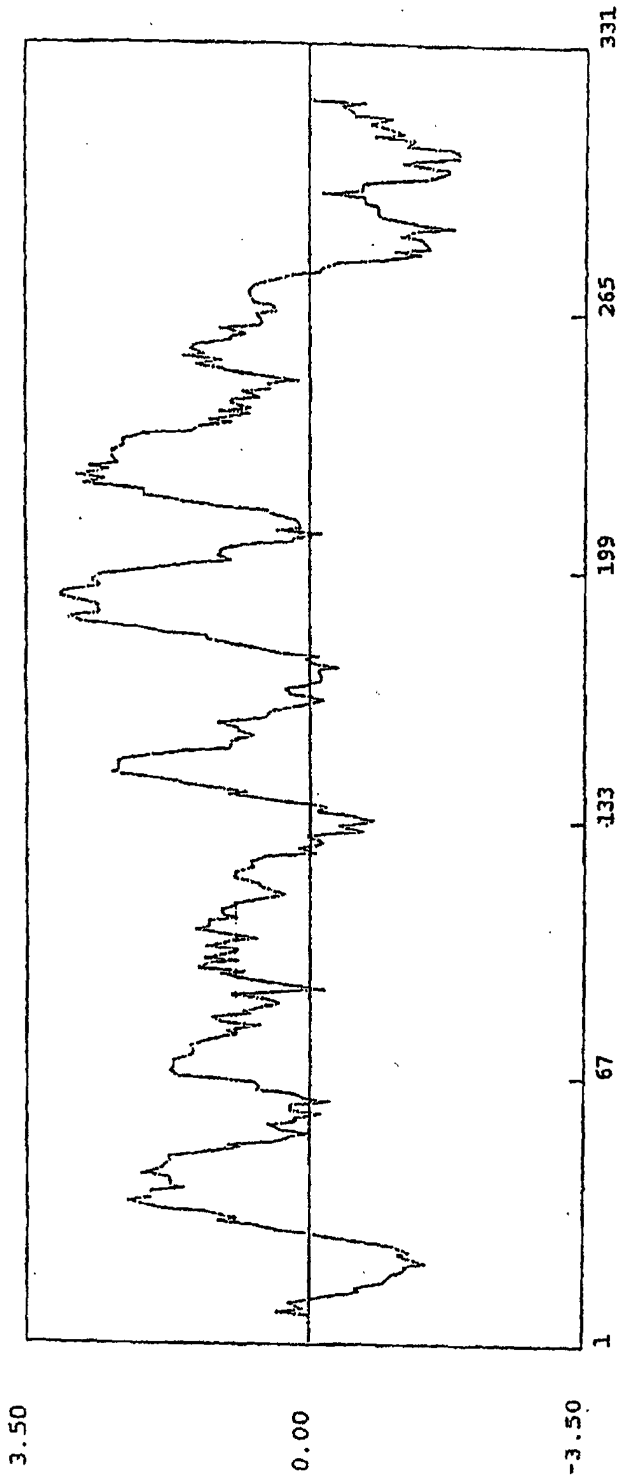

# Fig. 2

```
 37 ISLVGLTGNAVVLWLLGYRMRRNAVSIYILNLAAAD..FLFLSFQIIRSP 84
    || :|:: |:::||:| :|||||  ::|| :|: ||  :||  | |:
 41 ISPLGFVENGILLWFLCFRMRRNPFTVYITHLSIADISLLFCIF.ILSID 89

 85 LRL...INISHLIRKILVSV.MTFPYFTGLSMLSAISTERCLSVLWPIWY 130
      |   :: :|     : :|| : | | ||| :|:|||:|||||||:||||
 90 YALDYELSSGHYYTIVTLSVTFLFGYNTGLYLLTAISVERCLSVLYPIWY 139

131 RCRRPTHLSAVVCVLLWGLSLLFSMLEWRFCDFLFSGADSSWCETSD... 177
    ||:|| | || ||:|||:|| | :.:|: :|  : || :|   : ||
140 RCHRPKHQSAFVCALLWALSCLVTTMEYVMC..IDSGEESH..SQSDCRA 185

178 ...FIPVAWLIFLCVVLCVSSLVLLVRILCGSRKMPLTRLYVTILLTVLV 224
       || :  :: :  :: |||.:|:|:|  ::     ::||::|::|:::
186 VIIFIAILSFLVFTPLMLVSSTILVVKIRKNTWASHSSKLYIVIMVTIII 235

225 FLLCGLPFGILGALIY.......RMHLNLEVLYCHVYLVCMSLSSLNSSA 267
    ||: ::|: :|  | |       ::| |:::|:          |::||||
236 FLIFAMPMRVLYLLYYEYWSTFGNLH.NISLLF..........STINSSA 274

268 NPIIYFFVGSFRQRQNRQNLKLVLQRALQDKPEVDKGEGQLPEESLE 314
    ||:||||||| :::: |::|||| ||::|: :  : ||:     |:|
275 NPFIYFFVGSSKKKRFRESLKVVLTRAFKDEMQPRRQEGNGNTVSIE 321
```

# Fig.- 3

1 ATGGATCCAACCGTCCCAGTCTTGGGTACAAAACTGACACCAATCAACGGACGTGAGGAG 60

MetAspProThrValProValLeuGlyThrLysLeuThrProIleAsnGlyArgGluGlu

61 ACTCCTTGCTACAAGCAGACCCTGAGCTTCACGGTGCTGACGTGCATCATTTCCCTTGTC 120

ThrProCysTyrLysGlnThrLeuSerPheThrValLeuThrCysIleIleSerLeuVal

121 GGACTGACAGGAAACGCGGTTGTGCTCTGGCTCCTGGGCTGCCGCATGCGCAGGAACGCT 180

GlyLeuThrGlyAsnAlaValValLeuTrpLeuLeuGlyCysArgMetArgArgAsnAla

181 GTCTCCATCTACATCCTCAACCTGGCCGCAGCAGACTTCCTCTTCCTCAGCTTCCAGATT 240

ValSerIleTyrIleLeuAsnLeuAlaAlaAlaAspPheLeuPheLeuSerPheGlnIle

241 ATACGTTCGCCATTACGCCTCATCAATATCAGCCATCTCATCCGCAAAATCCTCGTTTCT 300

IleArgSerProLeuArgLeuIleAsnIleSerHisLeuIleArgLysIleLeuValSer

301 GTGATGACCTTTCCCTACTTTACAGGCCTGAGTATGCTGAGCGCCATCAGCACCGAGCGC 360

ValMetThrPheProTyrPheThrGlyLeuSerMetLeuSerAlaIleSerThrGluArg

361 TGCCTGTCTGTTCTGTGGCCCATCTGGTACCGCTGCCGCCGCCCCACACACCTGTCAGCG 420

CysLeuSerValLeuTrpProIleTrpTyrArgCysArgArgProThrHisLeuSerAla

421 GTCGTGTGTGTCCTGCTCTGGGGCCTGTCCCTGCTGTTTAGTATGCTGGAGTGGAGGTTC 480

ValValCysValLeuLeuTrpGlyLeuSerLeuLeuPheSerMetLeuGluTrpArgPhe

# Fig. 4

481 TGTGACTTCCTGTTTAGTGGTGCTGATTCTAGTTGGTGTGAAACGTCAGATTTCATCCCA 540

CysAspPheLeuPheSerGlyAlaAspSerSerTrpCysGluThrSerAspPheIlePro

541 GTCGCGTGGCTGATTTTTTTATGTGTGGTTCTCTGTGTTTCCAGCCTGGTCCTGCTGGTC 600

ValAlaTrpLeuIlePheLeuCysValValLeuCysValSerSerLeuValLeuLeuVal

601 AGGATCCTCTGTGGATCCCGGAAGATGCCGCTGACCAGGCTGTACGTGACCATCCTGCTC 660

ArgIleLeuCysGlySerArgLysMetProLeuThrArgLeuTyrValThrIleLeuLeu

661 ACAGTGCTGGTCTTCCTCCTCTGCGGCCTGCCCTTCGGCATTCTGGGGGCCCTAATTTAC 720

ThrValLeuValPheLeuLeuCysGlyLeuProPheGlyIleLeuGlyAlaLeuIleTyr

721 AGGATGCACCTGAATTTGGAAGTCTTATATTGTCATGTTTATCTGGTTTGCATGTCCCTG 780

ArgMetHisLeuAsnLeuGluValLeuTyrCysHisValTyrLeuValCysMetSerLeu

781 TCCTCTCTAAACAGTAGTGCCAACCCCATCATTTACTTCTTCGTGGGCTCCTTTAGGCAG 840

SerSerLeuAsnSerSerAlaAsnProIleIleTyrPhePheValGlySerPheArgGln

841 CGTCAAAATAGGCAGAACCTGAAGCTGGTTCTCCAGAGGGCTCTGCAGGACAAGCCTGAG 900

ArgGlnAsnArgGlnAsnLeuLysLeuValLeuGlnArgAlaLeuGlnAspLysProGlu

901 GTGGATAAAGGTGAAGGGCAGCTTCCTGAGGAAAGCCTGGAGCTGTCGGGAAGCAGATTG 960

ValAspLysGlyGluGlyGlnLeuProGluGluSerLeuGluLeuSerGlySerArgLeu

961 GGGCCATGA 969

GlyPro***

Fig. 5

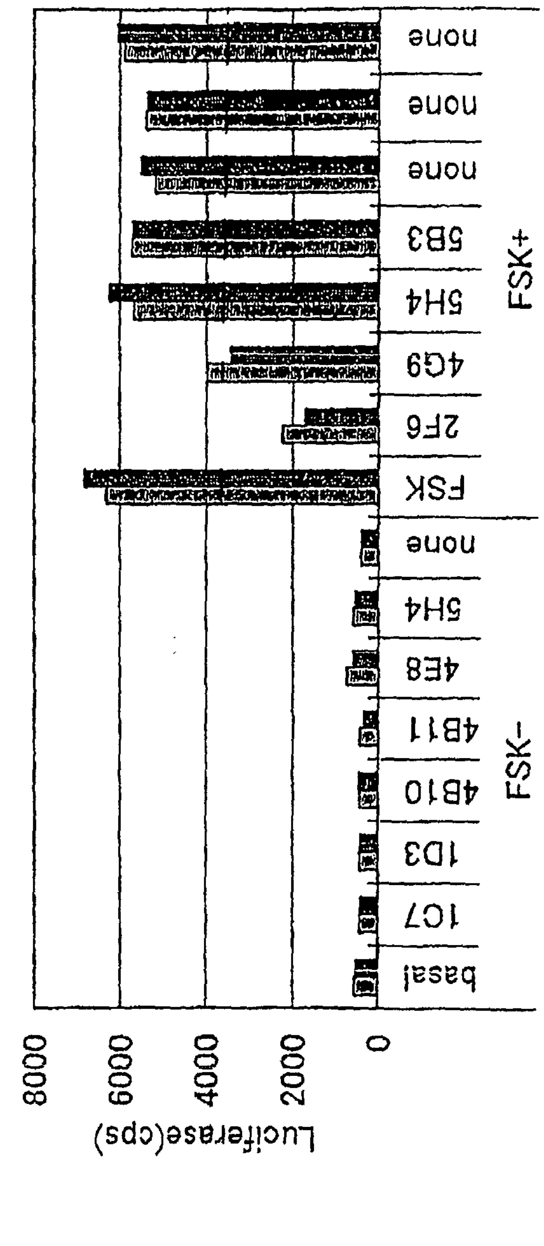
Luciferase(cps)
8000
6000
4000
2000
0
basal
1C7
1D3
4B10
4B11
4E8
5H4
none
FSK
2F6
4G9
5H4
5B3
none
none
none
FSK-
FSK+

Fig. 6

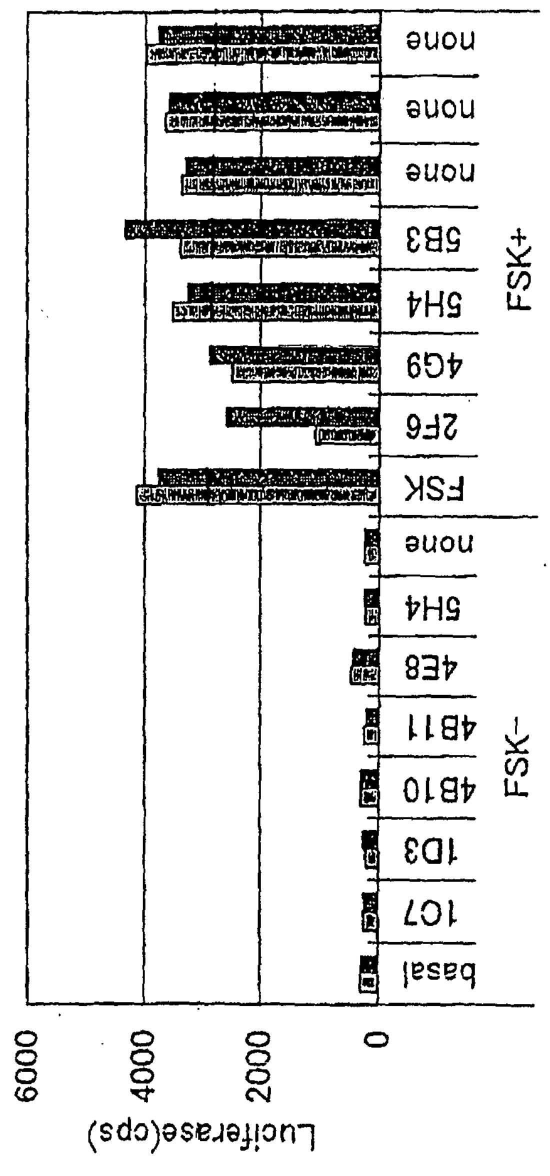
Luciferase(cps)
6000
4000
2000
0
basal
1O7
1D3
4B10
4B11
4E8
5H4
none
FSK
2F6
4G9
5H4
5B3
none
none
none
FSK-
FSK+

Fig. 7

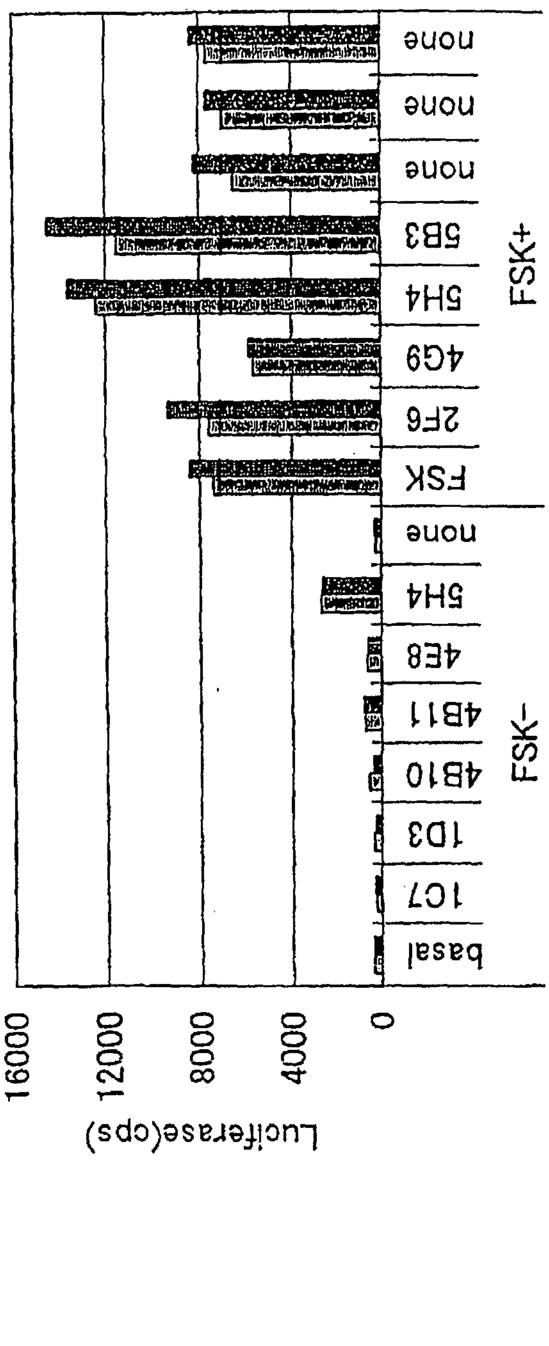

Luciferase(cps)
16000
12000
8000
4000
0
basal
1C7
1D3
4B10
4B11
4E8
5H4
none
FSK
2F6
4G9
5H4
5B3
none
none
none
FSK-
FSK+

Fig. 8

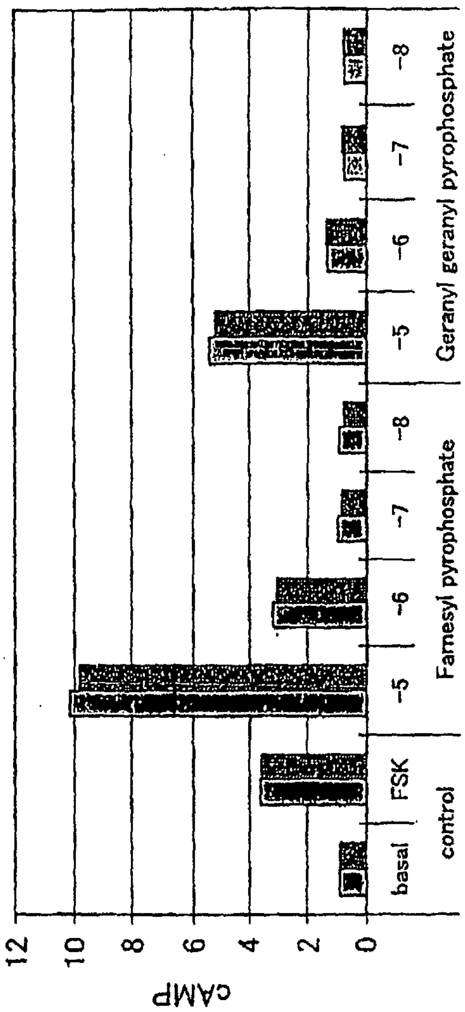
cAMP
12
10
8
6
4
2
0
basal
FSK
control
-5
-6
-7
-8
Farnesyl pyrophosphate
-5
-6
-7
-8
Geranyl geranyl pyrophosphate

Fig. 9

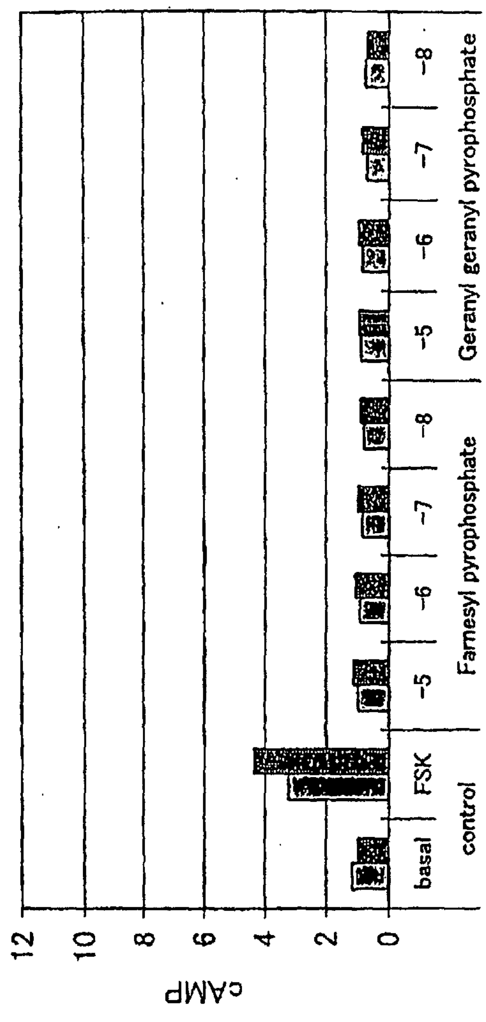
cAMP
12
10
8
6
4
2
0
basal
FSK
control
-5
-6
-7
-8
Farnesyl pyrophosphate
-5
-6
-7
-8
Geranyl geranyl pyrophosphate

Fig. 10

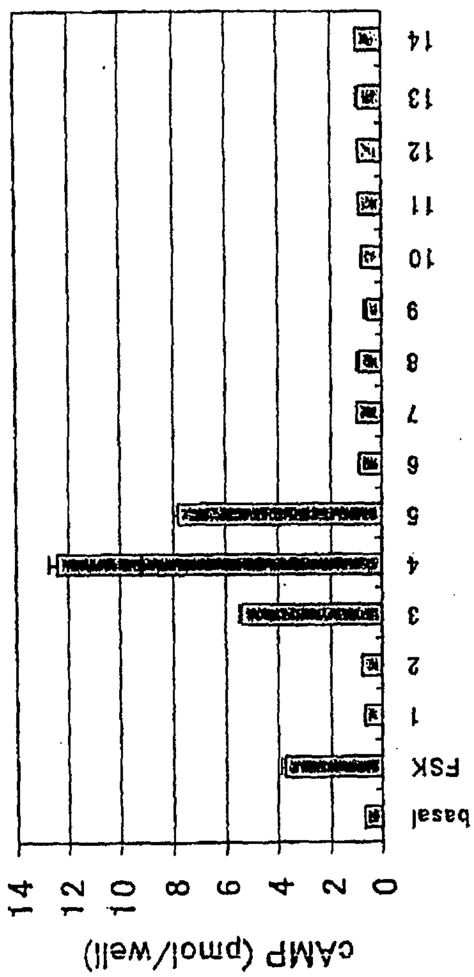
cAMP (pmol/well)
0
2
4
6
8
10
12
14
basal
FSK
1
2
3
4
5
6
7
8
9
10
11
12
13
14

Fig. 11

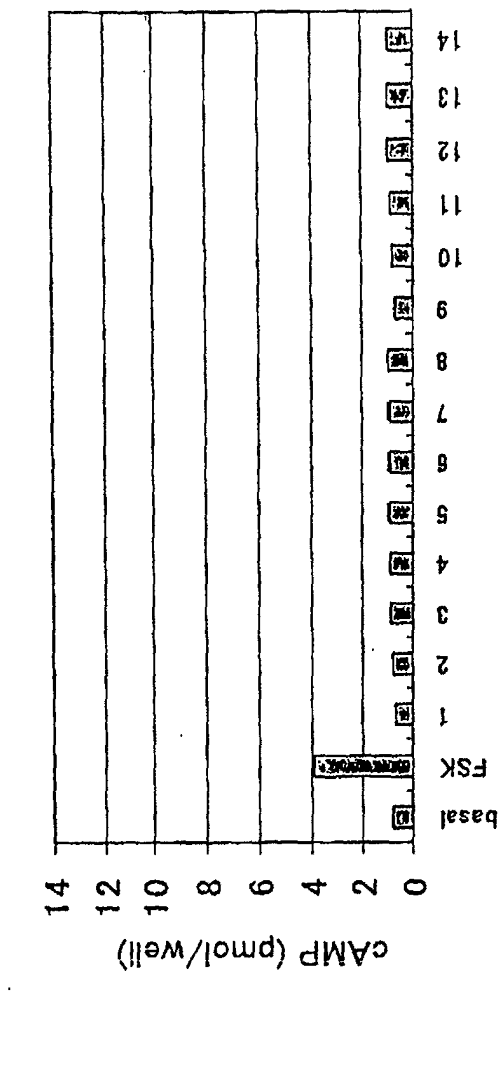
cAMP (pmol/well)
0
2
4
6
8
10
12
14
basal
FSK
1
2
3
4
5
6
7
8
9
10
11
12
13
14

Fig. 12

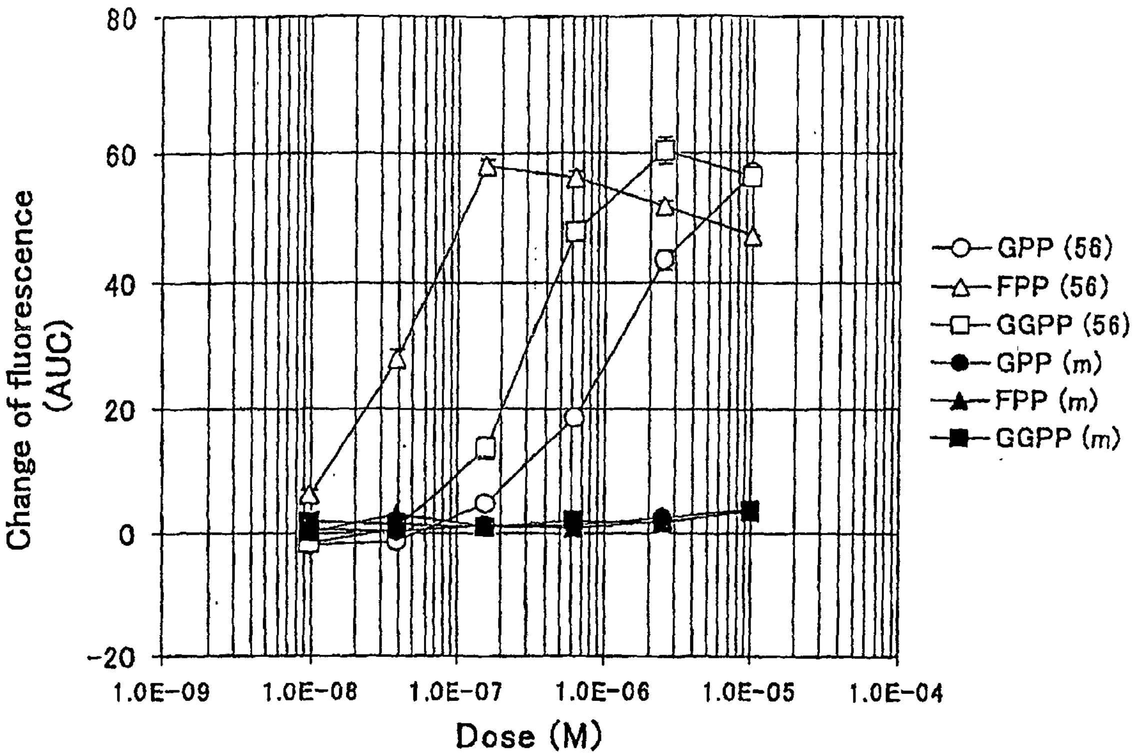
80
60
40
20
0
-20
Change of fluorescence (AUC)
1.0E-09
1.0E-08
1.0E-07
1.0E-06
1.0E-05
1.0E-04
Dose (M)
GPP (56)
FPP (56)
GGPP (56)
GPP (m)
FPP (m)
GGPP (m)

Fig. 13

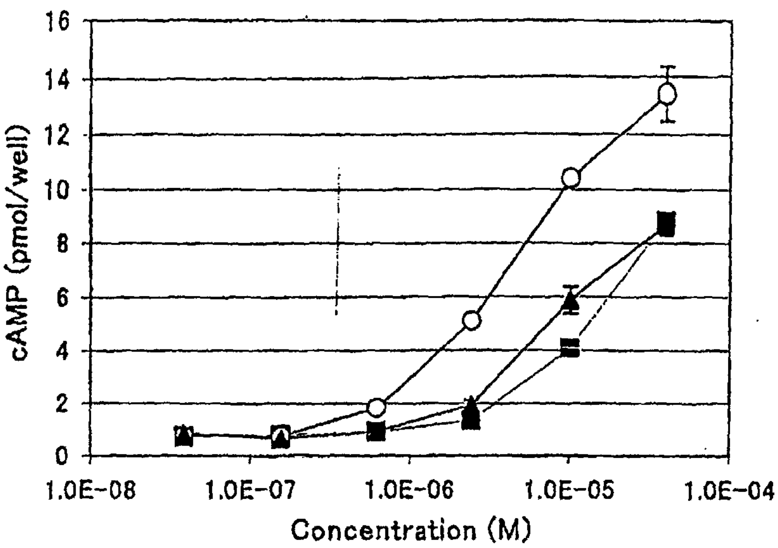
16
14
12
10
8
6
4
2
0
cAMP (pmol/well)
1.0E-08
1.0E-07
1.0E-06
1.0E-05
1.0E-04
Concentration (M)

Fig. 14

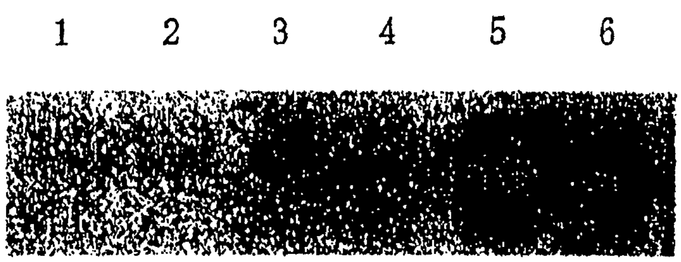

Fig. 15

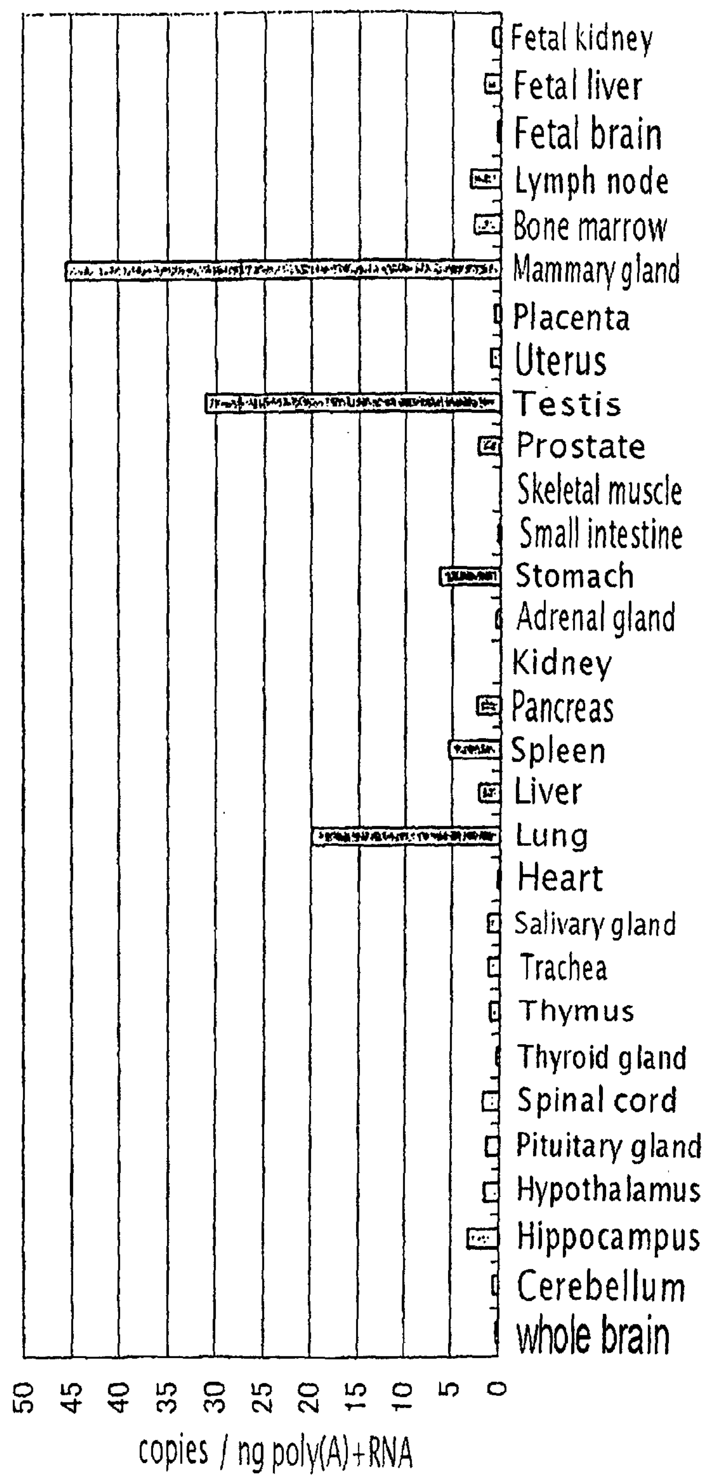
Fetal kidney
Fetal liver
Fetal brain
Lymph node
Bone marrow
Mammary gland
Placenta
Uterus
Testis
Prostate
Skeletal muscle
Small intestine
Stomach
Adrenal gland
Kidney
Pancreas
Spleen
Liver
Lung
Heart
Salivary gland
Trachea
Thymus
Thyroid gland
Spinal cord
Pituitary gland
Hypothalamus
Hippocampus
Cerebellum
whole brain
50 45 40 35 30 25 20 15 10 5 0
copies / ng poly(A)+RNA

**INTERNATIONAL SEARCH REPORT**

International application No.
PCT/JP02/08873

A. CLASSIFICATION OF SUBJECT MATTER
Int.Cl$^7$ G01N33/50, 33/15, 33/566, A61K38/17, 45/00, A61P35/00, 43/00

According to International Patent Classification (IPC) or to both national classification and IPC

B. FIELDS SEARCHED

Minimum documentation searched (classification system followed by classification symbols)
Int.Cl$^7$ G01N33/50, 33/15, 33/566, A61K38/17, 45/00, A61P35/00, 43/00

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2002 |
| Kokai Jitsuyo Shinan Koho | 1971-2002 | Jitsuyo Shinan Toroku Koho | 1996-2002 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | WO 99/32519 A (AstraZeneca Canada Inc.), 01 July, 1999 (01.07.99), & EP 1051434 A | 9<br>1-5,11-18, 20-25,27,28 |
| A | JP 2000-189174 A (Takeda Chemical Industries, Ltd.), 11 July, 2000 (11.07.00), & EP 1118621 A | 1-5,9,11-18, 20-25,27,28 |
| A | JP 2000-166576 A (Takeda Chemical Industries, Ltd.), 20 June, 2000 (20.06.00), & EP 1118620 A | 1-5,9,11-18, 20-25,27,28 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 December, 2002 (06.12.02) | 24 December, 2002 (24.12.02) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/08873

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 6-8, 10, 19, 26
because they relate to parts of the international application that do not comply with the prescribed requirements to such an extent that no meaningful international search can be carried out, specifically:
It is unclear what substances are involved in the scopes of the compound changing the binding properties of an isoprenoid-associated substance to the G protein-coupled receptor, the ligand obtained by the method as set forth in claim 9, the compound obtained by (continued to extra sheet)

3. ☐ Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1998)

**INTERNATIONAL SEARCH REPORT**

International application No.
PCT/JP02/08873

Continuation of Box No.I-2 of continuation of first sheet(1)

screening, and the compound changing the expression dose of G protein-coupled receptor protein.

Form PCT/ISA/210 (extra sheet) (July 1998)

SEQUENCE LISTING

<110> Takeda Chemical Industries, Ltd.

<120> Use of G-Protein Coupled Receptor Protein

<130> 2959WOOP

<150> JP 2001-266308
<151> 2001-09-03

<160> 10

<210> 1
<211> 322
<212> PRT
<213> Homo sapiens

<400> 1
Met Asp Pro Thr Val Pro Val Phe Gly Thr Lys Leu Thr Pro Ile Asn
1 5 10 15
Gly Arg Glu Glu Thr Pro Cys Tyr Asn Gln Thr Leu Ser Phe Thr Val
20 25 30
Leu Thr Cys Ile Ile Ser Leu Val Gly Leu Thr Gly Asn Ala Val Val
35 40 45
Leu Trp Leu Leu Gly Tyr Arg Met Arg Arg Asn Ala Val Ser Ile Tyr
50 55 60
Ile Leu Asn Leu Ala Ala Ala Asp Phe Leu Phe Leu Ser Phe Gln Ile
65 70 75 80
Ile Arg Ser Pro Leu Arg Leu Ile Asn Ile Ser His Leu Ile Arg Lys
85 90 95
Ile Leu Val Ser Val Met Thr Phe Pro Tyr Phe Thr Gly Leu Ser Met
100 105 110
Leu Ser Ala Ile Ser Thr Glu Arg Cys Leu Ser Val Leu Trp Pro Ile
115 120 125
Trp Tyr Arg Cys Arg Arg Pro Thr His Leu Ser Ala Val Val Cys Val
130 135 140
Leu Leu Trp Gly Leu Ser Leu Leu Phe Ser Met Leu Glu Trp Arg Phe
145 150 155 160
Cys Asp Phe Leu Phe Ser Gly Ala Asp Ser Ser Trp Cys Glu Thr Ser
165 170 175
Asp Phe Ile Pro Val Ala Trp Leu Ile Phe Leu Cys Val Val Leu Cys
180 185 190
Val Ser Ser Leu Val Leu Leu Val Arg Ile Leu Cys Gly Ser Arg Lys
195 200 205
Met Pro Leu Thr Arg Leu Tyr Val Thr Ile Leu Leu Thr Val Leu Val
210 215 220
Phe Leu Leu Cys Gly Leu Pro Phe Gly Ile Leu Gly Ala Leu Ile Tyr
225 230 235 240
Arg Met His Leu Asn Leu Glu Val Leu Tyr Cys His Val Tyr Leu Val
245 250 255
Cys Met Ser Leu Ser Ser Leu Asn Ser Ser Ala Asn Pro Ile Ile Tyr
260 265 270
Phe Phe Val Gly Ser Phe Arg Gln Arg Gln Asn Arg Gln Asn Leu Lys
275 280 285
Leu Val Leu Gln Arg Ala Leu Gln Asp Lys Pro Glu Val Asp Lys Gly

290 295 300
Glu Gly Gln Leu Pro Glu Glu Ser Leu Glu Leu Ser Gly Ser Arg Leu
305 310 315 320
Gly Pro

<210> 2
<211> 969
<212> DNA
<213> Homo sapiens

<400> 2
atggatccaa ccgtcccagt cttcggtaca aaactgacac caatcaacgg acgtgaggag 60
actccttgct acaatcagac cctgagcttc acggtgctga cgtgcatcat ttcccttgtc 120
ggactgacag gaaacgcggt agtgctctgg ctcctgggct accgcatgcg caggaacgct 180
gtctccatct acatcctcaa cctggccgca gcagacttcc tcttcctcag cttccagatt 240
atacgttcgc cattacgcct catcaatatc agccatctca tccgcaaaat cctcgtttct 300
gtgatgacct ttccctactt tacaggcctg agtatgctga gcgccatcag caccgagcgc 360
tgcctgtctg ttctgtggcc catctggtac cgctgccgcc gccccacaca cctgtcagcg 420
gtcgtgtgtg tcctgctctg ggcctgtcc ctgctgttta gtatgctgga gtggaggttc 480
tgtgacttcc tgtttagtgg tgctgattct agttggtgtg aaacgtcaga tttcatccca 540
gtcgcgtggc tgattttttt atgtgtggtt ctctgtgttt ccagcctggt cctgctggtc 600
aggatcctct gtggatcccg gaagatgccg ctgaccaggc tgtacgtgac catcctgctc 660
acagtgctgg tcttcctcct ctgcggcctg cccttcggca ttctgggggc cctaatttac 720
aggatgcacc tgaatttgga agtcttatat tgtcatgttt atctggtttg catgtccctg 780
tcctctctaa acagtagtgc caacccccatc atttacttct tcgtgggctc ctttaggcag 840
cgtcaaaata ggcagaacct gaagctggtt ctccagaggg ctctgcagga caagcctgag 900
gtggataaag gtgaagggca gcttcctgag gaaagcctgg agctgtcggg aagcagattg 960
gggccatga 969

<210> 3
<211> 969
<212> DNA
<213> Homo sapiens

<400> 3
atggatccaa ccgtcccagt cttcggtaca aaactgacac caatcaacgg acgtgaggag 60
actccttgct acaatcagac cctgagcttc acggtgctga cgtgcatcat ttcccttgtc 120
ggactgacag gaaacgcggt tgtgctctgg ctcctgggct accgcatgcg caggaacgct 180
gtctccatct acatcctcaa cctggccgca gcagacttcc tcttcctcag cttccagatt 240
atacgttcgc cattacgcct catcaatatc agccatctca tccgcaaaat cctcgtttct 300
gtgatgacct ttccctactt tacaggcctg agtatgctga gcgccatcag caccgagcgc 360
tgcctgtctg ttctgtggcc catctggtac cgctgccgcc gccccacaca cctgtcagcg 420
gtcgtgtgtg tcctgctctg ggcctgtcc ctgctgttta gtatgctgga gtggaggttc 480
tgtgacttcc tgtttagtgg tgctgattct agttggtgtg aaacgtcaga tttcatccca 540
gtcgcgtggc tgattttttt atgtgtggtt ctctgtgttt ccagcctggt cctgctggtc 600
aggatcctct gtggatcccg gaagatgccg ctgaccaggc tgtacgtgac catcctgctc 660
acagtgctgg tcttcctcct ctgcggcctg cccttcggca ttctgggggc cctaatttac 720
aggatgcacc tgaatttgga agtcttatat tgtcatgttt atctggtttg catgtccctg 780
tcctctctaa acagtagtgc caacccccatc atttacttct tcgtgggctc ctttaggcag 840
cgtcaaaata ggcagaacct gaagctggtt ctccagaggg ctctgcagga caagcctgag 900
gtggataaag gtgaagggca gcttcctgag gaaagcctgg agctgtcggg aagcagattg 960
gggccatga 969

<210> 4
<211> 29
<212> DNA

<213> Artificial Sequence

<220>
<223> Primer

<400> 4
gtcgacatgg atccaaccgt cccagtctt 29

<210> 5
<211> 27
<212> DNA
<213> Artificial Sequence

<220>
<223> Primer

<400> 5
actagttcat ggccccaatc tgcttcc 27

<210> 6
<211> 322
<212> PRT
<213> Homo sapiens

<400> 6
Met Asp Pro Thr Val Pro Val Leu Gly Thr Lys Leu Thr Pro Ile Asn
1               5                   10                  15
Gly Arg Glu Glu Thr Pro Cys Tyr Lys Gln Thr Leu Ser Phe Thr Val
            20                  25                  30
Leu Thr Cys Ile Ile Ser Leu Val Gly Leu Thr Gly Asn Ala Val Val
        35                  40                  45
Leu Trp Leu Leu Gly Cys Arg Met Arg Arg Asn Ala Val Ser Ile Tyr
    50                  55                  60
Ile Leu Asn Leu Ala Ala Ala Asp Phe Leu Phe Leu Ser Phe Gln Ile
65                  70                  75                  80
Ile Arg Ser Pro Leu Arg Leu Ile Asn Ile Ser His Leu Ile Arg Lys
                85                  90                  95
Ile Leu Val Ser Val Met Thr Phe Pro Tyr Phe Thr Gly Leu Ser Met
            100                 105                 110
Leu Ser Ala Ile Ser Thr Glu Arg Cys Leu Ser Val Leu Trp Pro Ile
        115                 120                 125
Trp Tyr Arg Cys Arg Arg Pro Thr His Leu Ser Ala Val Val Cys Val
    130                 135                 140
Leu Leu Trp Gly Leu Ser Leu Leu Phe Ser Met Leu Glu Trp Arg Phe
145                 150                 155                 160
Cys Asp Phe Leu Phe Ser Gly Ala Asp Ser Ser Trp Cys Glu Thr Ser
                165                 170                 175
Asp Phe Ile Pro Val Ala Trp Leu Ile Phe Leu Cys Val Val Leu Cys
            180                 185                 190
Val Ser Ser Leu Val Leu Leu Val Arg Ile Leu Cys Gly Ser Arg Lys
        195                 200                 205
Met Pro Leu Thr Arg Leu Tyr Val Thr Ile Leu Leu Thr Val Leu Val
    210                 215                 220
Phe Leu Leu Cys Gly Leu Pro Phe Gly Ile Leu Gly Ala Leu Ile Tyr
225                 230                 235                 240
Arg Met His Leu Asn Leu Glu Val Leu Tyr Cys His Val Tyr Leu Val

```
                245                 250                 255
Cys Met Ser Leu Ser Ser Leu Asn Ser Ser Ala Asn Pro Ile Ile Tyr
            260                 265                 270
Phe Phe Val Gly Ser Phe Arg Gln Arg Gln Asn Arg Gln Asn Leu Lys
        275                 280                 285
Leu Val Leu Gln Arg Ala Leu Gln Asp Lys Pro Glu Val Asp Lys Gly
    290                 295                 300
Glu Gly Gln Leu Pro Glu Glu Ser Leu Glu Leu Ser Gly Ser Arg Leu
305                 310                 315                 320
Gly Pro
```

<210> 7
<211> 969
<212> DNA
<213> Homo sapiens

<400> 7

```
atggatccaa ccgtcccagt cttgggtaca aaactgacac caatcaacgg acgtgaggag    60
actccttgct acaagcagac cctgagcttc acggtgctga cgtgcatcat ttcccttgtc   120
ggactgacag gaaacgcggt agtgctctgg ctcctgggct gccgcatgcg caggaacgct   180
gtctccatct acatcctcaa cctggccgca gcagacttcc tcttcctcag cttccagatt   240
atacgttcgc cattacgcct catcaatatc agccatctca tccgcaaaat cctcgtttct   300
gtgatgacct ttccctactt tacaggcctg agtatgctga gcgccatcag caccgagcgc   360
tgcctgtctg ttctgtggcc catctggtac cgctgccgcc gccccacaca cctgtcagcg   420
gtcgtgtgtg tcctgctctg ggcctgtcc ctgctgttta gtatgctgga gtggaggttc    480
tgtgacttcc tgtttagtgg tgctgattct agttggtgtg aaacgtcaga tttcatccca   540
gtcgcgtggc tgattttttt atgtgtggtt ctctgtgttt ccagcctggt cctgctggtc   600
aggatcctct gtggatcccg gaagatgccg ctgaccaggc tgtacgtgac catcctgctc   660
acagtgctgg tcttcctcct ctgcggcctg cccttcggca ttctgggggc cctaatttac   720
aggatgcacc tgaatttgga agtcttatat tgtcatgttt atctggtttg catgtccctg   780
tcctctctaa acagtagtgc caaccccatc atttacttct tcgtgggctc ctttaggcag   840
cgtcaaaata ggcagaacct gaagctggtt ctccagaggg ctctgcagga caagcctgag   900
gtggataaag gtgaagggca gcttcctgag gaaagcctgg agctgtcggg aagcagattg   960
gggccatga                                                           969
```

<210> 8
<211> 23
<212> DNA
<213> Artificial Sequence

<220>
<223> Primer

<400> 8

```
ggccctaatt tacaggatgc acc                23
```

<210> 9
<211> 23
<212> DNA
<213> Artificial Sequence

<220>
<223> Primer

<400> 9

```
gcccacgaag aagtaaatga tgg                23
```

<210> 10
<211> 28
<212> DNA
<213> Artificial Sequence

<220>
<223> Primer

<400> 10
tggtttgcat gtccctgtcc tctctaaa 28